(19) Europäisches Patentamt
European Patent Office
Office européen des brevets

(11) EP 1 330 457 B1

(12) EUROPEAN PATENT SPECIFICATION

(45) Date of publication and mention of the grant of the patent:
**20.10.2004 Bulletin 2004/43**

(21) Application number: **01987225.8**

(22) Date of filing: **01.11.2001**

(51) Int Cl.[7]: **C07D 487/00**

(86) International application number:
**PCT/US2001/045792**

(87) International publication number:
**WO 2002/042304 (30.05.2002 Gazette 2002/22)**

# (54) CYCLOPENTA[B][1,4] DIAZEPINO[6,7,1-HI]INDOLES AS 5HT2C ANTAGONISTS

CYCLOPENTA[B][1,4] DIAZEPINO[6,7,1-HI]INDOLE ALS 5HT2C ANTAGONISTEN

CYCLOPENTA [B][1,4]DIAZEPINO 6,7,1-HI]INDOLES COMME ANTAGONISTES DE 5HT2C

(84) Designated Contracting States:
**AT BE CH CY DE DK ES FI FR GB GR IE IT LI LU MC NL PT SE TR**
Designated Extension States:
**AL LT LV MK RO SI**

(30) Priority: **03.11.2000 US 245591 P**
**03.11.2000 US 245593 P**
**03.11.2000 US 245843 P**
**03.11.2000 US 245915 P**
**03.11.2000 US 245954 P**

(43) Date of publication of application:
**30.07.2003 Bulletin 2003/31**

(73) Proprietor: **Wyeth**
**Madison, New Jersey 07940-0874 (US)**

(72) Inventors:
- **SABB, Annmarie, Louise**
  **Pennington, NJ 08534 (US)**
- **VOGEL, Robert, Lewis**
  **Stratford, NJ 08084 (US)**
- **NELSON, James, Albert**
  **Washington Crossing, PA 18977 (US)**
- **ROSENZWEIG-LIPSON, Sharon, Joy**
  **East Brunswick, NJ 08816 (US)**
- **WELMAKER, Gregory, Scott**
  **Collegeville, PA 19426 (US)**
- **SABALSKI, Joan, Eileen**
  **Yardville, NJ 08620 (US)**
- **SMITH, Michael, David**
  **Martinez, CA 94553 (US)**
- **CHAN, Anita, Wai-Yin**
  **Fort Lee, NJ 07024 (US)**
- **ANTANE, Madelene, Miyoko**
  **West Windsor, NJ 08550 (US)**
- **RAVEENDRANATH, Panolil**
  **Monroe, NY 10950 (US)**
- **MEGATI, Sreenivasulu**
  **New City, NY 10956 (US)**

(74) Representative: **Wileman, David Francis Dr.**
**Wyeth Pharmaceuticals,**
**Huntercombe Lane South,**
**Taplow**
**Maidenhead, Berkshire SL6 OPH (GB)**

(56) References cited:
**WO-A-00/35922** **WO-A-02/08186**
**WO-A-96/29316** **US-A- 3 914 250**

- **HESTERS J. B., ET AL.: "Pyrrolo[3,2,,1-jk][1,4]benzodiazepines and Pyrrolo[1,2,3-ef][1,5]benzodiazepines Which Have Central Nervous System Activity" J. MED. CHEM., vol. 13, no. 5, 1970, pages 827-835, XP002200700 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 330 457 B1

## Description

The present invention relates to cyclopenta[b][1,4]diazepino[6,7,1-*hi*]indoles and derivatives thereof, processes for preparing them, pharmaceutical compositions containing them and intermediates used in their preparation. The active compounds of this invention are serotonin 5-hydroxytryptamine $2_C$ ($5HT_{2C}$) receptor agonists useful for the treatment of central nervous system disorders including, but not limited to, obsessive-compulsive disorder, depression, anxiety, generalized anxiety disorder, schizophrenia, panic disorder, migraine, sleep disorders, such as sleep apnea, eating disorders, such as hyperphagia, obesity, epilepsy, and spinal cord injury.

## BACKGROUND OF THE INVENTION

Obesity is a medical disorder characterized by an excess of body fat or adipose tissue. Comorbidities associated with obesity are Type II diabetes, cardiovascular disease, hypertension, hyperlipidemia, stroke, osteoarthritis, sleep apnea, gall bladder disease, gout, some cancers, some infertility, and early mortality. As the percentage of obese individuals continues to rise both in the U.S. and abroad, obesity is expected to be a major health risk in the $21^{st}$ Century. The serotonin 5-hydroxytryptamine (5-HT) receptor is a G-protein coupled receptor which is expressed in neurons in many regions of the human central nervous system. [Wilkinson, L. O. and Dourish, C. T. in *Serotonin Receptor Subtypes: Basic and Clinical Aspects* (ed. Peroutka, S. J. ) 147-210 (Wiley-Liss, New York, 1991).] The $5HT_{2c}$ receptor (formerly called the $5HT_{1C}$ receptor) is a prominent subtype of the serotonin receptor found in the central nervous system of both rats and humans. It is expressed widely in both cortical and subcortical regions. [Julius, D. MacDermott, A. B., Axel, R. Jessell, T. M. *Science* 241:558-564 (1988).] Studies in several animal species and in humans have shown that the non-selective $5HT_{2C}$ receptor agonist, *meta*-chlorophenylpiperazine (MCPP) decreases food intake. [Cowen, P.J., Clifford, E. M., Williams, C., Walsh, A. E. S., Fairbum, C. G. *Nature* 376: 557 (1995).] Tecott, *et al* have demonstrated that transgenic mice lacking the $5HT_{2C}$ receptor eat more and are heavier than Wild Type mice. [Tecott, L. H., Sun, L. M., Akana, S. F., Strack, A. M., Lowenstein, D. H., Dallman, M. F., Jullus, D. *Nature* 374: 542-546 (1995).] Compounds of this invention are $5HT_{2C}$ receptor subtype selective agonists which are selective over other monoamine receptors, causes a reduction in food intake and result in a reduction in weight gain. Other therapeutic indications for $5HT_{2C}$ agonists are obsessive compulsive disorder, depression, panic disorder, schizophrenia, sleep disorders, eating disorders, and epilepsy.

The non-selective $5\text{-}HT_{2c}$ agonist, *meta*-chlorophenylpiperazine (*m*-CPP), has been shown to block conditioned avoidance responding (CAR) in the rat, an activity usually associated with antipsychotic activity in man [Martin, Gregory E.; Elgin, Jr., Robert J.; Mathiasen, Joanne R.; Davis, Coralie B.; Kesslick, James M.; Baldy, William J.; Shank, Richard P.; DiStefano, Deena L.; Fedde, Cynthia L.; Scott, Malcolm K. *J. Med. Chem.* **1989**, *32*, 1052-1056]. More recently, additional data suggests that $5\text{-}HT_{2c}$ agonism may produce an antipsychotic-like effect in the CAR model [Browning, J. L.; Young, K. A.; Hicks, P. B. Presented at the $29^{th}$ Annual Meeting of the Society for Neuroscience, Miami Beach, Florida, October 1999, Abstract **830.12**].

United States Patent 3,914,250 (October 21, 1975) describes 1,4-diazepino[6,5,4-jk]carbazoles, having the structures below, as anticonvulsant agents.

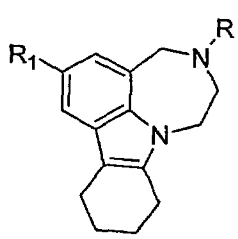

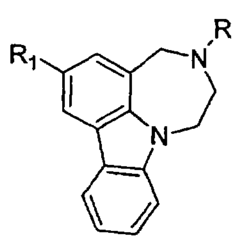

Pyrrolo[3,2,1-*jk*][1,4]benzodiazepines and 4,5-dihydropyrrolo[3,2,1-*jk*][1,4]-benzodiazepines have been described by Hester *et al.* (*J. Med. Chem.* **1970**, *13*, 827-835) to have central nervous system activity.

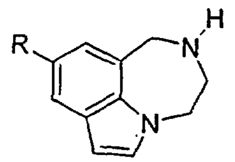
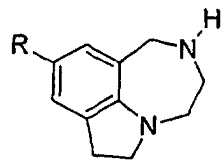

This invention provides cyclopenta[b][1,4]diazepino[6,7,1-hi]indoles and derivatives which bind to and activate $5HT_{2c}$ receptors in the CNS and are useful for the treatment of CNS disorders which can benefit from modulation of the $5HT_{2c}$ receptor.

## DESCRIPTION OF THE INVENTION

This invention provides compounds of formula **I** having the structure:

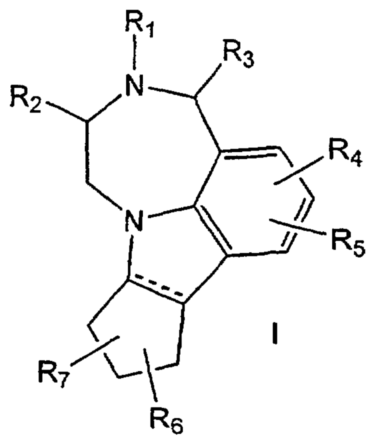

**I**

wherein:

$R_1$ is hydrogen, alkyl of 1-6 carbon atoms, aryl, acyl of 2-7 carbon atoms, or -C(O)R' wherein R' is alkyl of from 1 to 6 carbon atoms or aryl, preferably phenyl;

$R_2$ and $R_3$ are each, independently, hydrogen, alkyl of 1-6 carbon atoms, cycloalkyl of 3-7 carbon atoms, alkoxy of 1-6 carbon atoms, $-CH_2OH$, fluorinated alkyl of 1-6 carbon atoms, $-NH-SO_2$-alkyl of 1-6 carbon atoms, $-SO_2-NH$-alkyl of 1-6 carbon atoms, alkyl amide of 1-6 carbon atoms, amino, alkylamino of 1-6 carbon atoms, dialkylamino of 1-6 carbon atoms per alkyl moiety, fluorinated alkoxy of 1-6 carbon atoms, acyl of 2-7 carbon atoms, aryl, arylalkyl, heteroaryl, aroyl, or heteroaroyl;

$R_4$ and $R_5$ are each, independently, hydrogen, alkyl of 1-6 carbon atoms, alkoxy of 1-6 carbon atoms, halogen, fluorinated alkyl of 1-6 carbon atoms, -CN, $-NH-SO_2$-alkyl of 1-6 carbon atoms, $-SO_2-NH$-alkyl of 1-6 carbon atoms, alkylamide of 1-6 carbon atoms (-CO-NHalkyl), amino, alkylamino of 1-6 carbon atoms, dialkylamino of 1-6 carbon atoms per alkyl moiety, fluorinated alkoxy of 1-6 carbon atoms, acyl of 2-7 carbon atoms, aroyl or heteroaroyl;

$R_6$ and $R_7$ are each independently hydrogen, $C_1$-$C_6$ alkyl, cycloalkyl of 3 to 7 carbon atoms or $-CH_2$-(cycloalkyl of 3 to 7 carbon atoms);

the dashed line indicates an optional double bond;

or a pharmaceutically acceptable salt thereof.

As used herein alkyl as a group or part of a group, e.g. arylalhyl, alkylamino or alkoxy, etc., includes straight or branched chain alkyl groups of 1-6 carbon atoms; e.g. methyl, ethyl, propyl, isopropyl, n-butyl and t-butyl. Alkoxy groups are for example methoxy, ethoxy, propoxy, isopropoxy and butoxy. Cycloalkyl groups may be for example cyclopropyl, cyclobutyl, cyclopentyl and cyclohexyl.

In the definitions used herein, the fluorinated alkyl and fluorinated alkoxy groups indicate the specified alkyl or alkoxy groups, e.g as defined above, having any amount of fluorine substitution including, but not limited to, groups such as $-CHF_2$, $-CF_3$, $-C_2F_5$, $-OCF_3$, etc.

In the definitions used herein aryl as a group or part of a group e.g., arylalkyl has preferably 6-10 carbon atoms, e.g. phenyl or naphthyl, most preferably phenyl. Heteroaryl as a group or part of a group, e.g. heteroaroyl includes mono- or bi-cyclic rings having 5 -10 ring members and 1-3 heteroatoms, the same or different, selected from O, N and S, for example thienyl, furanyl, pyrrolyl, pyridinyl or pyrimidinyl.. In the definitions used herein such as for each of $R_2$, $R_{2'}$ and $R_3$ and $R_4$ and $R_5$, the aroyl group is preferably benzoyl. The heteroaroyl group is preferably thienoyl. The arylalkyl group is preferably benzyl.

Examples of acyl are alkanoyl groups of 2-7 carbon atoms wherein the alkyl portion is as exemplified above; e.g. acetyl, propionoyl.

In the compounds disclosed herein, including intermediates, the variables when present may have one the following values, or any combination thereof:

$R_1$ may be for example hydrogen, $C_1$-$C_6$ alkyl, benzoyl and alkanoyl of 2-7 carbon atoms.

$R_2$ may be hydrogen, alkyl or fluorinated alkyl of 1-6 carbon atoms, $-CH_2OH$ or cycloalkyl eg of 5-7 carbon atoms.

$R_3$ may be hydrogen, alkyl or fluorinated alkyl of 1-6 carbon atoms, $-CH_2OH$ or cycloalkyl eg of 5-7 carbon atoms.

$R_4$ may be hydrogen, alkyl of 1-6 carbon atoms, alkoxy of 1-6 carbon atoms, halogen, -CN, amino or fluorinated alkyl of 1 to 6 carbon atoms, such as trifluoromethyl; especially hydrogen or alkyl of 1-6 carbon atoms.

$R_5$ may be hydrogen, alkyl of 1-6 carbon atoms, alkoxy of 1-6 carbon atoms, halogen, -CN, amino or fluorinated alkyl of 1 to 6 carbon atoms, such as trifluoromethyl; especially hydrogen or alkyl of 1-6 carbon atoms.

$R_6$ may be hydrogen or alkyl of 1-6 carbon atoms.

$R_7$ may be hydrogen or alkyl of 1-6 carbon atoms.

The dotted line may be present or absent.

Two groups of compounds within this invention comprise compounds of the formulae:

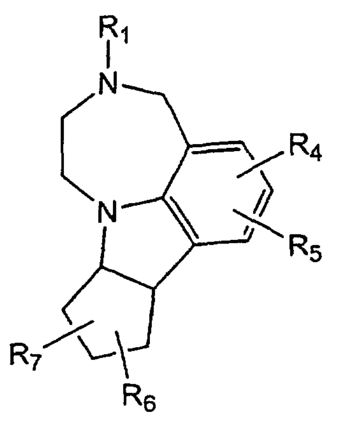

or

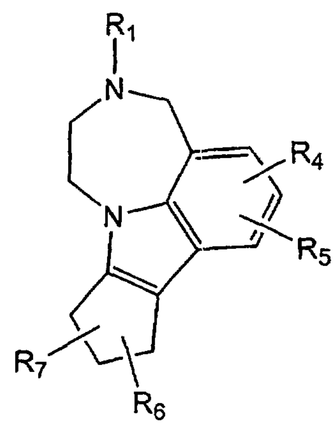

wherein $R_1$, $R_4$, $R_5$, $R_6$ and $R_7$ are as defined hereinabove, or a pharmaceutically acceptable salt thereof. A subset of this group of compounds comprise those in which $R_1$ is H or alkyl of from 1 to 6 carbon atoms, preferably H or $-CH_3$.

Another preferred group of the compounds are those of the formulae above wherein $R_1$ and $R_7$ are hydrogen and $R_4$, $R_5$, and $R_6$ are as defined above, or a pharmaceutically acceptable salt thereof. In a subset of these compounds, $R_1$, $R_4$, and $R_5$ are each hydrogen and $R_6$ and $R_7$ are as defined above, or a pharmaceutically acceptable salt thereof.

In a further preferred subset $R_1$, $R_4$, $R_5$, and $R_6$ are hydrogen and $R_7$ is as defined above.

Another set of compounds within this invention are those of the formulae:

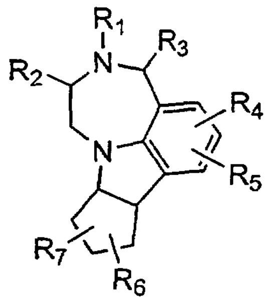

or

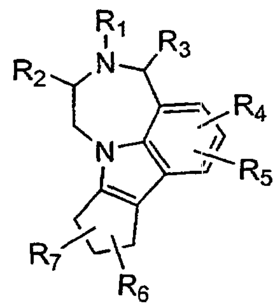

wherein:

$R_1$ is hydrogen, or alkyl of 1-6 carbon atoms;

$R_2$ and $R_3$ are each, independently, hydrogen, alkyl of 1-6 carbon atoms, cycloalkyl, alkoxy of 1-6 carbon atoms, $-CH_2OH$, or fluorinated alkyl of 1 to 6 carbon atoms, such as trifluoromethyl;

$R_4$ and $R_5$ are each, independently, hydrogen, alkyl of 1-6 carbon atoms, alkoxy of 1-6 carbon atoms, halogen, -CN, amino or fluorinated alkyl of 1 to 6 carbon atoms, such as trifluoromethyl;

$R_6$ and $R_7$ are each independently hydrogen or $C_1$-$C_6$ alkyl;

or a pharmaceutically acceptable salt thereof.

The $5HT_{2C}$ receptor agonists of this invention are useful for the treatment or prevention in mammals, preferably in humans, of disorders involving the central nervous system such as obsessive-compulsive disorder, depression, atypical depression, bipolar disorders, anxiety, generalized anxiety disorder, schizophrenia, psychoses, personality disorders, organic mental disorders, behavioral disorders associated with dementia or age-related conditions, aggressivity, drug and alcohol addiction, social phobias, sexual dysfunction, panic disorder, migraine, sleep disorders, such as sleep apnea, eating disorders, such as hyperphagia, bulimia or anorexia nervosa, obesity, epilepsy, and premenstrual tension..

This invention also includes the use of the compounds herein in the manufacture of medicaments for use as treatments or preventitive regimens for treatment of central nervous system deficiencies associated with trauma, stroke, neurodegenerative diseases or toxic or infective CNS disorders including, but not limited to, encephalitis or menengitis; or cardiovascular disorders, including thrombosis; gastrointestinal disorders such as malfunction of gastrointestinal motility; and diabetes insipidus. This includes the improvement or inhibition of further degradation of central nervous system activity during or following the malady or trauma in question. Included in these improvements are maintenance or improvement in motor and motility skills, control, coordination and strength.

This invention includes the use of a pharmaceutically or therapeutically effective amount of a compound of this invention, or a pharmaceutically acceptable salt thereof, in the manufacture of medicaments for treating each of these conditions in a mammal.

The compounds of this invention contain asymmetric carbon atoms and thus give rise to optical isomers and diastereoisomers. While shown without respect to stereochemistry in Formula **I**, the present invention includes such optical isomers and diastereoisomers; as well as the racemic and resolved, enantiomerically pure R and S stereoisomers; as well as other mixtures of the R and S stereoisomers and pharmaceutically acceptable salts thereof.

The term "alkyl" includes both straight- and branched-chain saturated aliphatic hydrocarbon groups and cycloalkyl groups. Halogen is defined as Cl, Br, F, and I.

Pharmaceutically acceptable salts can be formed from organic and inorganic acids, for example, acetic, propionic, lactic, citric, tartaric, succinic, fumaric, maleic, malonic, mandelic, malic, phthalic, hydrochloric, hydrobromic, phosphoric, nitric, sulfuric, methanesulfonic, naphthalenesulfonic, benzenesulfonic, toluenesulfonic, camphorsulfonic, and similarly known acceptable acids.

Preferred compounds of this invention are those in which $R_1$ is hydrogen. Especially preferred are compounds which are enantiomerically pure stereoisomers of compounds where $R_1$ is hydrogen and the pyrrole ring is reduced.

This invention also provides processes for preparing the compounds of formula (I), which processes comprise

one of the following:

a) reacting a compound of formula

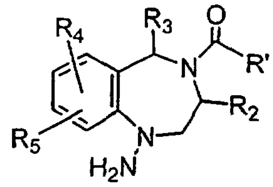

(II)

wherein R', $R_2$, $R_3$, $R_4$ and $R_5$ are as defined above,
with a compound of formula:

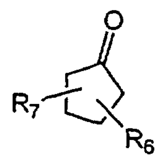

wherein $R_6$ and $R_7$ are as defined above, and cyclising the resultant hydrazone, to give a corresponding compound of formula (I) wherein $R_1$ is -C(O)R' and the optional bond is or
b) reducing a compound of formula

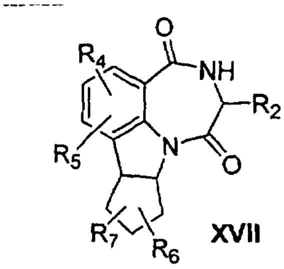

XVII

wherein $R_2$, $R_4$, $R_5$, $R_6$ and $R_7$ are as defined above,
with a reducing agent to give a corresponding compound of formula (I) wherein $R_3$ is hydrogen and the optional bond is absent;
or
c) reacting a compound of formula (XXIV):

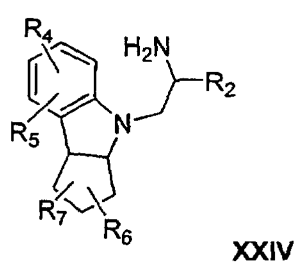

XXIV

wherein $R_2$, $R_4$, $R_5$,$R_6$ and $R_7$ are as defined above, with a compound of formula

$R_3CHO$

wherein $R_3$ is as defined above, to give a corresponding compound of formula (I) wherein the optional bond is absent;

or

d) reducing a diazabenzo[*cd*]cyclopenta[*a*]azulen-6-one compound of formula XXXV:

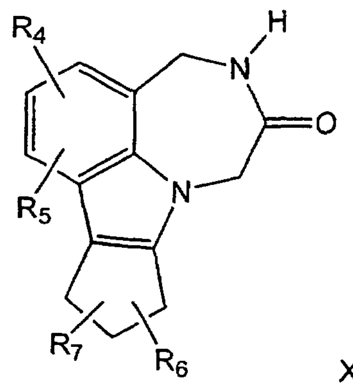

XXXV

wherein $R_4$, $R_5$,$R_6$ and $R_7$ are as defined above, with a reducing agent to a corresponding compound of formula (I) wherein $R_2$ is hydrogen;

or

e) reducing a compound of formula (I) wherein the optional bond is present to provide a compound of formula (I) wherein the optional bond is absent;

f) hydrolysing a compound of formula (I) wherein $R_1$ is acyl or -C(O)R' to give a compound of formula (I) wherein $R_1$ is hydrogen;

or

g) acylating a compound of formula (I) wherein $R_1$ is hydrogen with an acylating agent containing the group -C(O) R' to give a compound of formula (I) wherein $R_1$ is acyl or -C(O)R';

or

h) alkylating a compound of formula (I) wherein $R_1$ is hydrogen with an alkylating agent containing the group -$R_1$ wherein $R_1$ is alkyl or aryl to give a compound of formula (I) wherein $R_1$ is alkyl or aryl;

or

i) removing a protecting group from a compound of formula (I) in which at least one substituent carries a protecting group to give a compound of formula (I);

or

j) converting a basic compound of formula (I) to a salt thereof by reaction with a pharmaceutically acceptable acid or vice versa;

or

k) converting a compound of formula (I) having one or more reactive substituent groups to a different compound of formula (I);

or

1) isolating an isomer of a compound of formula (I) from a mixture thereof.

With regard to step a) the compound of formula (I) may be prepared by the following reaction sequence comprising the steps a1-a5:

a1) treating a benzodiazepine compound of the formula:

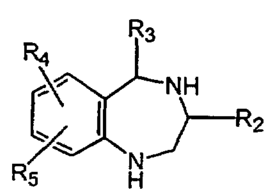

wherein $R_{2'}$, $R_2$, $R_3$, $R_4$ and $R_5$ are as defined herein; with an acylating agent containing COR' and a base in the presence of a polar solvent, to give an acylated benzodiazepine of the formula:

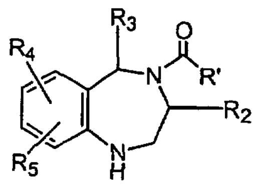

wherein R' represents alkyl of from 1 to 10 carbon atoms, preferably 1 to 6 carbon atoms, or a benzyl or naphthyl group;

a2) reacting the acylated benzodiazepine of step a1) with a nitrosating agent to provide an acylated nitroso benzodiazepine compound of the formula:

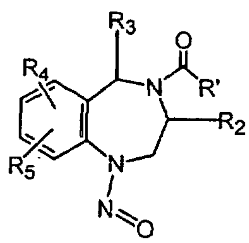

;

a3) reducing the acylated nitroso benzodiazepine compound of step a2) to yield an acylated 1-aminobenzodiazepine compound of the formula

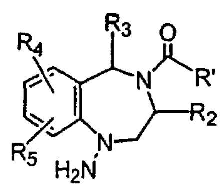

;

a4) reacting the acylated 1-aminobenzodiazepine compound of step 3) to react with a cyclopentanone compound of the formula:

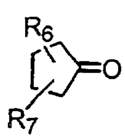

wherein $R_6$ and $R_7$ are as defined herein; to provide a cyclopentylideneamino benzodiazepine compound of the formula:

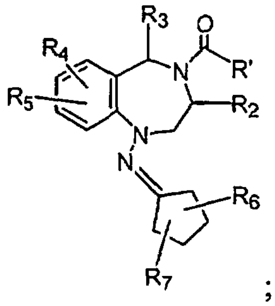

a5) cyclising the cyclopentylideneamino benzodiazepine compound of step 4) to provide an acylated compound of the formula:

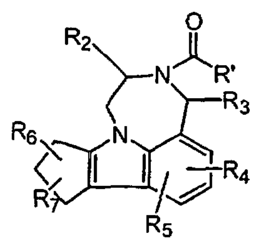

whereinR$_{2'}$, $R_2$, $R_3$, $R_4$, $R_5$, $R_6$ and $R_7$ are as defined herein.

This invention also provides processes for preparing the compounds of the invention wherein the benzodiazepine compound of step a1, above, is initially prepared by reduction of a corresponding substituted or unsubstituted benzodiazepinedione, as shown below.

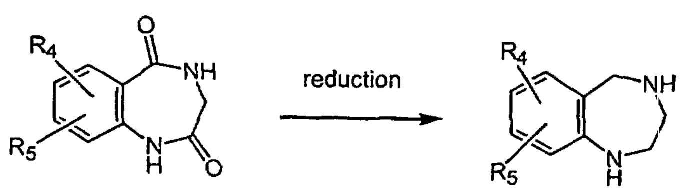


wherein $R_4$ and $R_5$ are as defined herein.
With regard to step b) the compound of formula (I) may be prepared by the following reaction sequence comprising the steps 1-5:

b1) reacting an optionally substituted cyclopentaindole-5-carboxylic acid of the formula **XIII** with an amino acid ester of formula **XIV,** wherein R' represents an alkyl group of from 1 to 10 carbon atoms, to produce an optionally substituted cyclopentaindole-5-carbonyl-amino acetic acid alkyl ester of formula **XV**;

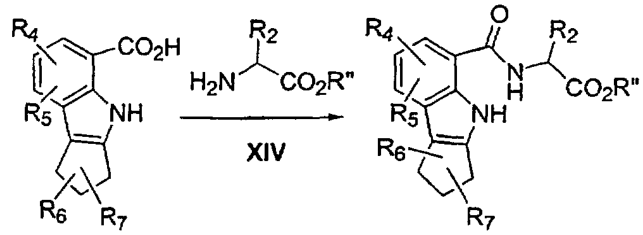

XIV

XIII XV

wherein $R_2$, $R_4$, $R_5$, $R_6$ and $R_7$ are as defined above and COOR" is an ester group ,

b2) treating the optionally substituted cyclopentaindole-5-carbonyl-amino acetic acid alkyl ester of formula **XV** from step b1) with a reducing agent to provide an optionally substituted cyclopenta[b]indole-5-carbonyl-amino-acetic acid alkyl ester of the formula **XVI:**

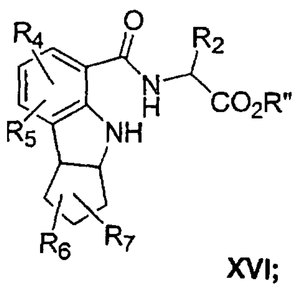

**XVI;**

wherein $R_2$, $R_4$, $R_5$,$R_6$ and $R_7$ are as defined above and COOR" is an ester group,

b3) hydrolysing the reduced ester compound of formula **XVI** in the presence of a base, followed by treatment with an acid, to form an optionally substituted diaza-benzo[*cd*]cyclopenta[a]azulene-4,7-dione compound of formula **XVII**:

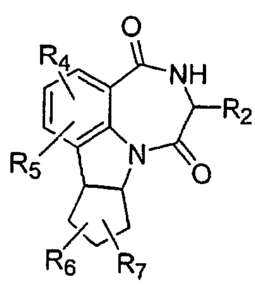

XVII

wherein $R_2$, $R_4$, $R_5$, $R_6$ and $R_7$ are as defined above,
and

b4) treating the optionally substituted diaza-benzo[*cd*]cyclopenta[a]-azulene-4,7-dione compound of formula **XVII** with a reducing agent to provide a compound of the formula **XVIII:**

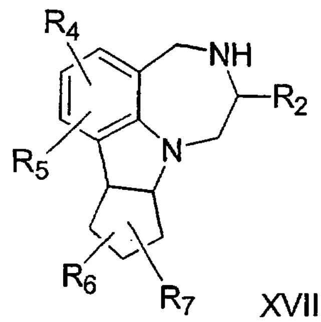

XVIII

With regard to step c) compounds of formula (I) may be prepared by the following reaction sequence comprising the steps c1)-c5):

c1) converting a cyclopenta[b]indole compound of the formula:

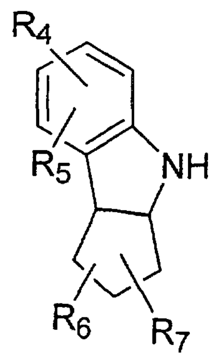

XXII

wherein $R_4$, $R_5$, $R_6$ and $R_7$ are as defined herein; to an optionally substituted cyclopenta[b]indol-4-ylacetamide compound of the formula:

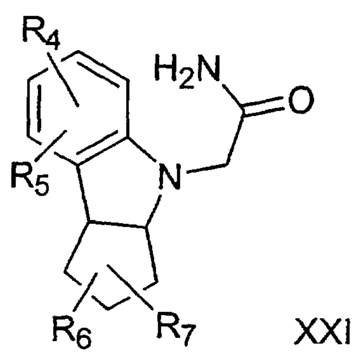

XXIII

wherein $R_4$, $R_5$, $R_6$ and $R_7$ are as defined herein;

c2) reducing the optionally substituted cyclopenta[b]indol-4-ylacetamide of step c1) to the corresponding optionally substituted cyclopenta[b]indol-4-yl-amine of the formula:

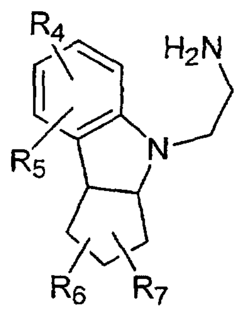

XXIV

and

c3) cyclizing the cyclopenta[b]indol-4-yl-amine of step c2) to an optionally substituted diaza-benzo[cd]cyclopenta[a]azulene compound of the formula:

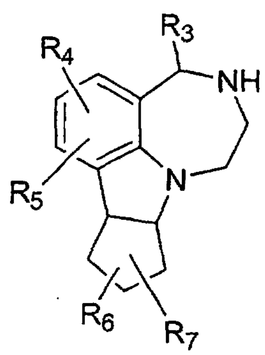

XXV

The process above further optionally comprises an initial step wherein the cyclopenta[b]indole compound of step c1) is formed from the reduction of a cyclopenta[b]indole compound of the formula:

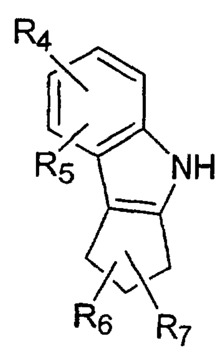

XXI

wherein $R_4$, $R_5$, $R_6$ and $R_7$ are as defined herein.
An alternate synthesis of this invention comprises the steps of:

cc1) converting an optionally substituted cyclopenta[b]indole compound of the formula:

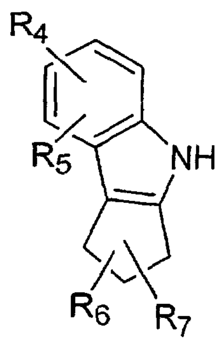

XXII

to produce an optionally substituted nitrile compound of the formula:

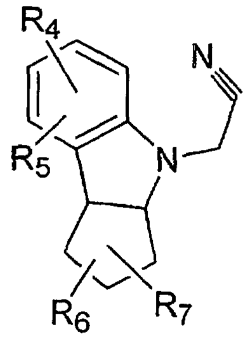

XXIIIa

cc2) reducing the optionally substituted nitrile compound of step aa1) to provide an optionally substituted amine compound of the formula:

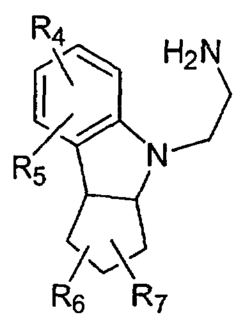

;

and

cc3) cyclizing the amine compound of step cc2) using an aldehyde of formula:

$R_3CHO$

to give an optionally substituted diaza-benzo[cd]cyclopenta[a]azulene compound of the formula:

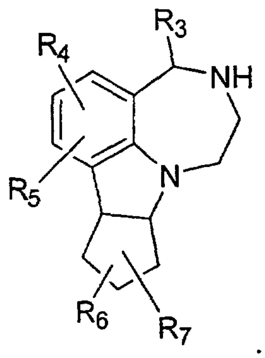

wherein $R_3$, $R_4$, $R_5$, $R_6$ and $R_7$ are as defined herein.

With regard to step d) compounds of formula **XXXV** may be prepared by a process comprising the steps of:

d1) acylating a cyclopentaindole methylamine of the formula:

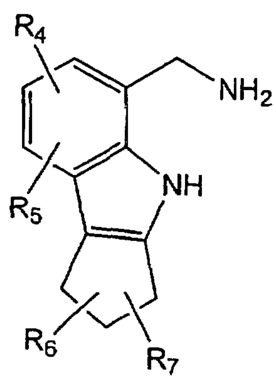

wherein $R_4$, $R_5$, $R_6$ and $R_7$ are as defined above, with an acylating agent of the formula:

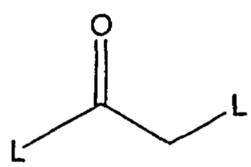

wherein L represents a leaving group as known in the art, such as a halogen, preferably Br, Cl or I, to produce an acylated compound of the formula:

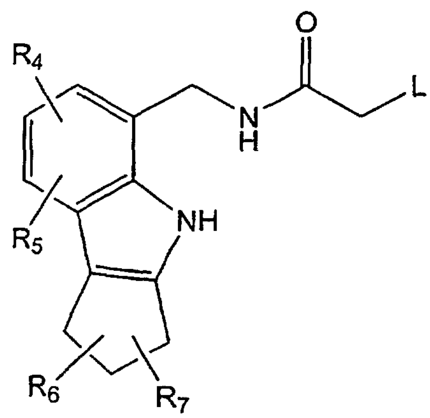

d2) cyclizing the acylated compound of step d1), e.g., by treating with a suitable base in the presence of a polar solvent, to produce an optionally substituted diazabenzo[*cd*]cyclopenta[*a*]azulen-6-one compound of the formula:

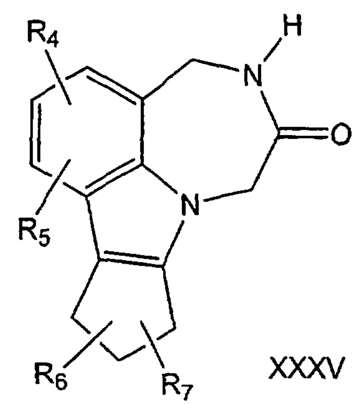

XXXV

It will be understood that the beginning optionally substituted cyclopentaindolemethylamine compounds of step d1), above, may be prepared by a number of synthetic methods known in the art. Additional steps within the scope of this invention for the preparation of this compound include:

dd1) allowing an optionally substituted 2-halophenylhydrazine compound of the formula:

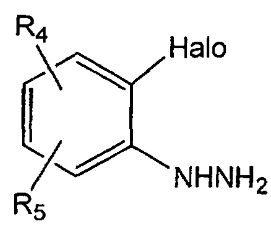

wherein Halo represents a halogen, preferably Br or I, and $R_4$ and $R_5$ are as defined above, to react with an optionally substituted cyclopentanone compound of the formula:

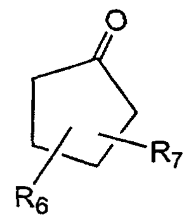

wherein $R_6$ and $R_7$ are as defined above, to produce a 5-halo-cyclopenta[*b*]indole compound of the formula:

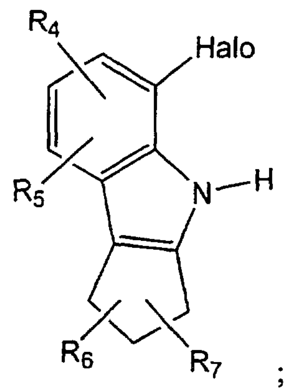

dd2) converting the 5-halo-cyclopenta[*b*]indole compound of step dd1) to an optionally substituted cyclopenta[b] indole aldehyde of the formula:

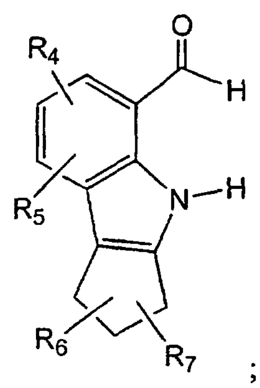

dd3) converting the optionally substituted cyclopenta[*b*]indole aldehyde of step dd2) to a corresponding optionally substituted cyclopenta[*b*]indole-5-carbaldehyde oxime of the formula: and

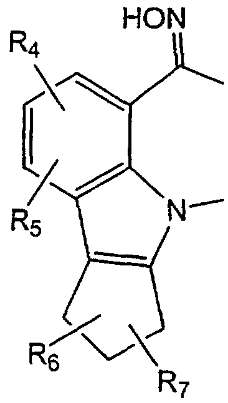

;

dd4) treating the optionally substituted cyclopenta[*b*]indole-5-carbaldehyde oxime of step dd3) with a reducing agent to provide a cyclopentaindole methylamine of the formula:

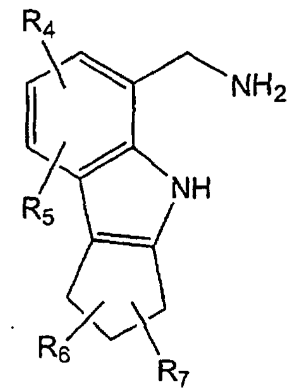

wherein $R_4$, $R_5$, $R_6$ and $R_7$ are as described above.

This invention also provides novel compounds which may be used as intermediates in the production of the pharmaceutically useful compounds described herein. These compounds include those of the formulae:

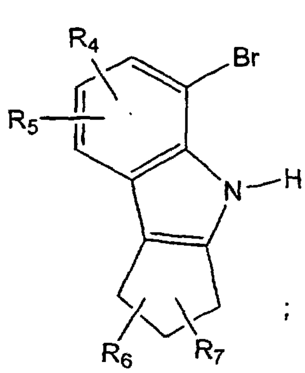

;

IV

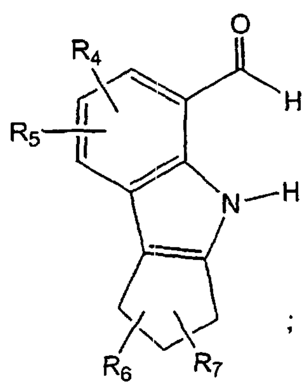

;

V

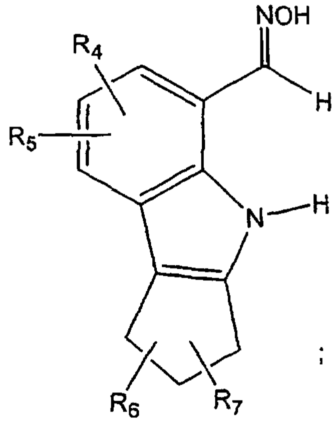
;

VI

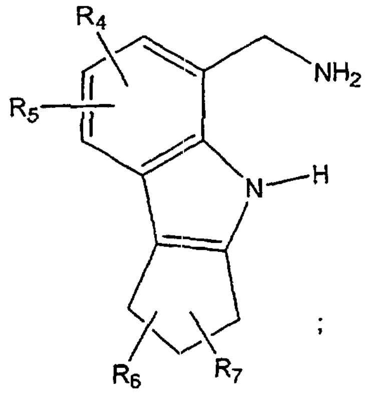
;

VII

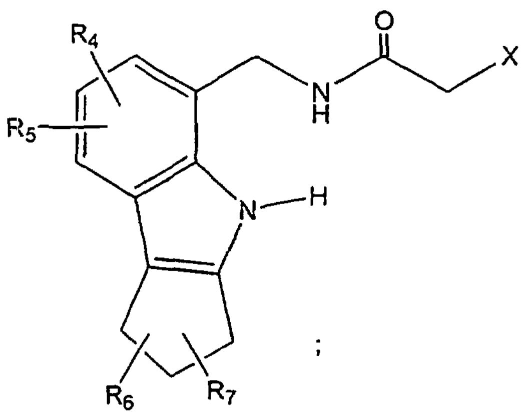
;

IX

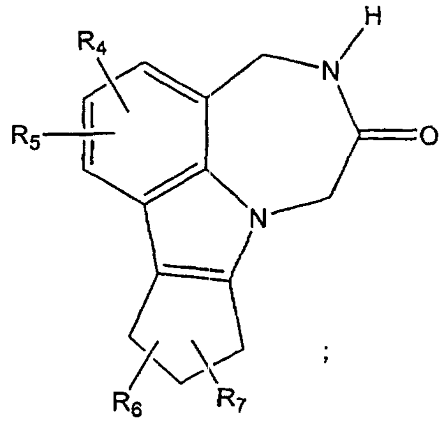
;

X

wherein in each formula $R_4$, $R_5$, $R_6$ and $R_7$ are as defined above and in Formula **IX**, the moiety X is Cl, Br or I. Subsets of the intermediate compounds of Formulas **IV**, **V**, **VI**, **VII**, **IX** and **X** which may be prepared by the processes described herein include those in which each of $R_4$, $R_3$, $R_6$ and $R_7$ are hydrogen. Another group of compounds of this invention include those in which $R_4$ is hydrogen and $R_5$, $R_6$ and $R_7$ are as defined above. In another group, $R_4$ and $R_6$ are hydrogen and $R_5$ and $R_7$ are as defined above. A further group of this invention comprises those compounds in which $R_4$, $R_5$, and $R_6$ are hydrogen and $R_7$ is as defined above.

Among the more preferred compounds of these groups are:

5-Bromo-1,2,3,4-tetrahydro-cyclopenta[*b*]indole;
5-Bromo-3-methyl-1,2,3,4-tetrahydro-cyclopenta[*b*]indole;
5-Bromo-2-methyl-1,2,3,4-tetrahydro-cyclopenta[*b*]indole;
5-Bromo-1-methyl-1,2,3,4-tetrahydro-cyclopenta[*b*]indole;
1,2,3,4-Tetrahydro-cyclopenta[*b*]indole-5-carbaldehyde;
3-Methyl-1,2,3,4-tetrahydro-cyclopenta[*b*]indole-5-carbaldehyde;
2-Methyl-1,2,3,4-tetrahydro-cyclopenta[*b*]indole-5-carbaldehyde;
1-Methyl-1,2,3,4-tetrahydro-cyclopenta[*b*]indole-5-carbaldehyde;
1,2,3,4-Tetrahydro-cyclopenta[*b*]indole-5-carbaldehyde oxime;
3-Methyl-1,2,3,4-tetrahydro-cyclopenta[*b*]indole-5-carbaldehyde oxime;
2-Methyl-1,2,3,4-tetrahydro-cyclopenta[*b*]indole-5-carbaldehyde oxime;
1-Methyl-1,2,3,4-tetrahydro-cyclopenta[*b*]indole-5-carbaldehyde oxime;

*C*-(1,2,3,4-Tetrahydro-cyclopenta[*b*]indol-5-yl)-methylamine;
*C*-(3-Methyl-1,2,3,4-tetrahydro-cyclopenta[*b*]indol-5-yl)-methylamine;
*C*-(2-Methyl-1,2,3,4-tetrahydro-cyclopenta[*b*]indol-5-yl)-methylamine;
*C*-(1-Methyl-1,2,3,4-tetrahydro-cyclopenta[*b*]indol-5-yl)-methylamine;
2-Chloro-*N*-(1,2,3,4-tetrahydro-cyclopenta[*b*]indol-5-ylmethyl)-acetamide;
2-Chloro-*N*-(3-methyl-1,2,3,4-tetrahydro-cyclopenta[*b*]indol-5-ylmethyl)-acetamide;
2-Chloro-*N*-(2-methyl-1,2,3,4-tetrahydro-cyclopenta[*b*]indol-5-ylmethyl)-acetamide;
2-Chloro-*N*-(1-methyl-1,2,3,4-tetrahydro-cyclopenta[*b*]indol-5-ylmethyl)-acetamide;
2-Bromo-*N*-(1,2,3,4-tetrahydro-cyclopenta[*b*]indol-5-ylmethyl)-acetamide;
2-Bromo-*N*-(3-methyl-1,2,3,4-tetrahydro-cyclopenta[*b*]indol-5-ylmethyl)-acetamide;
2-Bromo-*N*-(2-methyl-1,2,3,4-tetrahydro-cyclopenta[*b*]indol-5-ylmethyl)-acetamide;
2-Bromo-*N*-(1-methyl-1,2,3,4-tetrahydro-cyclopenta[*b*]indol-5-ylmethyl)-acetamide;
2-Iodo-*N*-(1,2,3,4-tetrahydro-cyclopenta[*b*]indol-5-ylmethyl)-acetamide;
2-Iodo-*N*-(3-methyl-1,2,3,4-tetrahydro-cyclopenta[*b*]indol-5-ylmethyl)-acetamide;
2-Iodo-*N*-(2-methyl-1,2,3,4-tetrahydro-cyclopenta[*b*]indol-5-ylmethyl)-acetamide;
2-Iodo-*N*-(1-methyl-1,2,3,4-tetrahydro-cyclopenta[*b*]indol-5-ylmethyl)-acetamide;
4,5,9,10-Tetrahydro-8*H*-5,7a-diaza-benzo[*cd*]cyclopenta[*a*]azulen-6-one;
8-Methyl-4,5,9,10-tetrahydro-8*H*-5,7a-diaza-benzo[*cd*]cyclopenta[*a*]azulen-6-one;
9-Methyl-4,5,9,10-tetrahydro-8*H*-5,7a-diaza-benzo[*cd*]cyclopenta[*a*]azulen-6-one; and
10-Methyl-4,5,9,10-tetrahydro-8*H*-5,7a-diaza-benzo[*cd*]cyclopenta[*a*]azulen-6-one.

More specifically, the compounds of this invention can be prepared according to the following scheme from commercially available starting materials or starting materials which can be prepared using literature procedures. Scheme 1 shows the preparation of representative compounds of this invention.

## Scheme 1

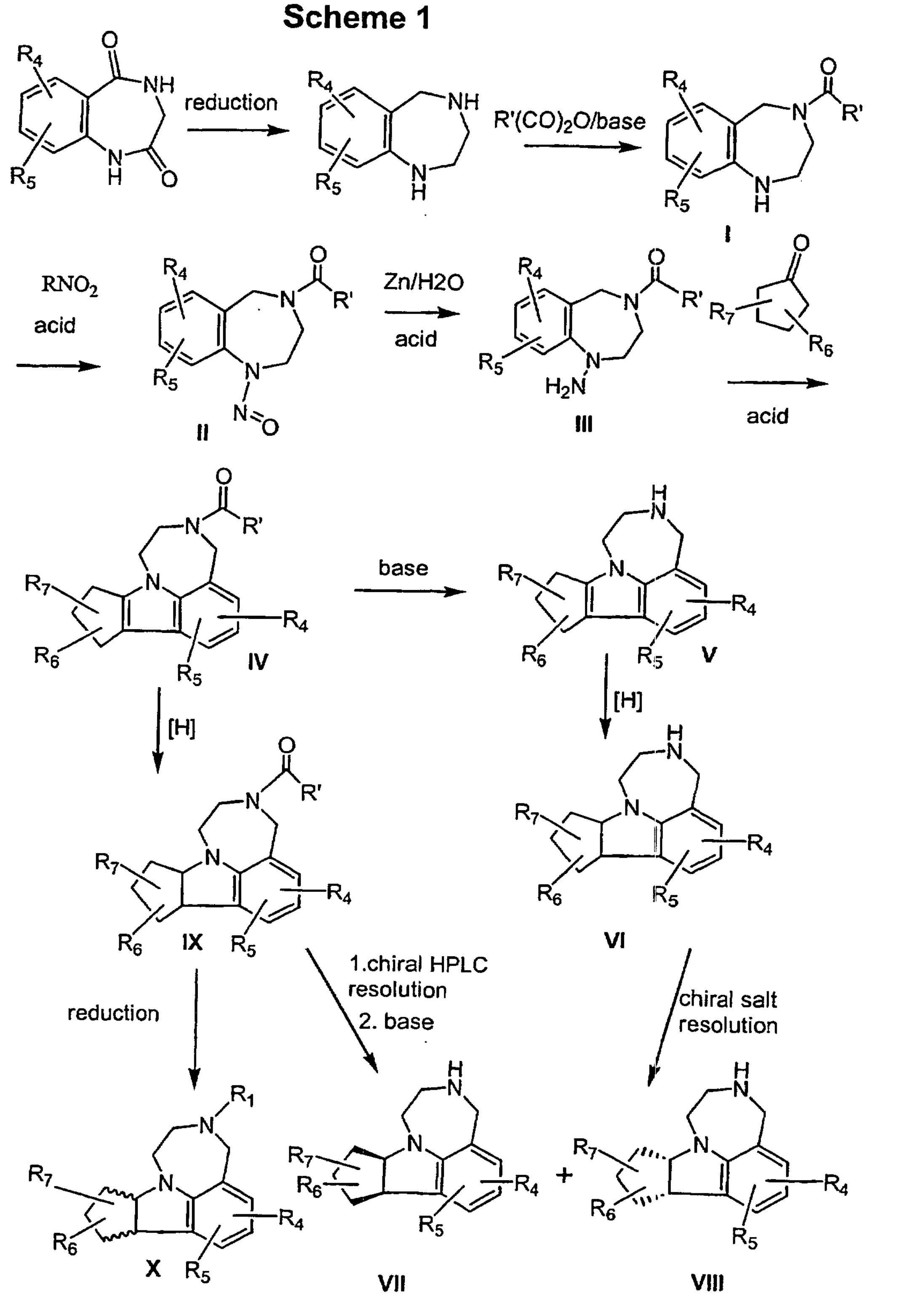


In Scheme 1, a substituted or unsubstituted benzodiazepinedione is reduced with a reducing agent, such as

lithium aluminum hydride or a borane-tetrahydrofuran complex, to give a substituted or unsubstituted benzodiazepine. The basic nitrogen of the benzodiazepine is acylated with an acylating reagent, such as an acid anhydride, in the presence of a base, such as triethylamine, in an organic solvent, such as ether, to °give intermediate **I**. Intermediate **I** is allowed to react with an organic or inorganic nitrosating agent, such as t-butyl nitrite or sodium nitrite, in the presence of an acid, such as acetic acid or hydrochloric acid, to give nitroso compounds **II.** The nitroso compounds are reduced to hydrazines **III** using a reducing agent, such as zinc powder in acetic acid and water. The hydrazines **III** are allowed to react with substituted or unsubstituted cyclopentanones in acid, such as acetic acid at 25-110 °C, to give substituted or unsubstituted hydrazones of formula

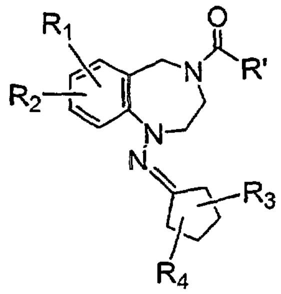

IV

The hydrazones are treated with an acid, such as sulfuric acid or p-toluenesulfonic acid, in the presence of water or an alcohol, such as 1-propanol, at elevated temperatures such as 50-110 °C to give protected fused. indoles **IV.** The fused indoles **IV** can be treated with a base, such as NaOH, in a polar solvent, such as water or an alcohol, to give the fused indoles **V,** which are products of this invention. In addition, fused indoles **V** can be reduced, such as by catalytic hydrogenation over a catalyst, such as palladium on charcoal, in an organic solvent, such as ethanol, in the presence of a trace of acid, such as trifluoroacetic acid, to give fused indolines **VI** which are products of this invention. Alternatively, fused indoles **IV** can be reduced, such as by catalytic hydrogenation over a catalyst, such as palladium on charcoal, in an organic solvent, such as ethanol, in the presence of a trace of an acid, such as trifluoroacetic acid, to give fused indolines **IX**. Fused indolines **IX** are racemic mixtures which can be resolved using chiral HPLC to give separated enantiomers which can then be treated with an inorganic base, such as NaOH in a polar solvent, such as water or methanol at elevated temperatures, such as 50-100 °C, to remove the acyl group giving enantiomers **VII** and **VIII** which are products of this invention. Finally, fused indolines **IX** can be reduced with a reducing agent, such as a borane tetrahydrofuran complex, to give fused indolines **X**, which are compounds of this invention. Enantiomers **VII** and **VIII** can also be obtained by chiral salt resolution of racemic fused indolines **VI** using a resolving agent, such as benzoyltartaric acid, in an organic solvent, such as an alcohol. As shown in the Scheme below treatment of the secondary amine of **VII** and **VIII** with an alkylating agent $R_1X$, such as an alkyl halide, gives the corresponding alkyl derivatives winch are compounds of this invention.

Scheme

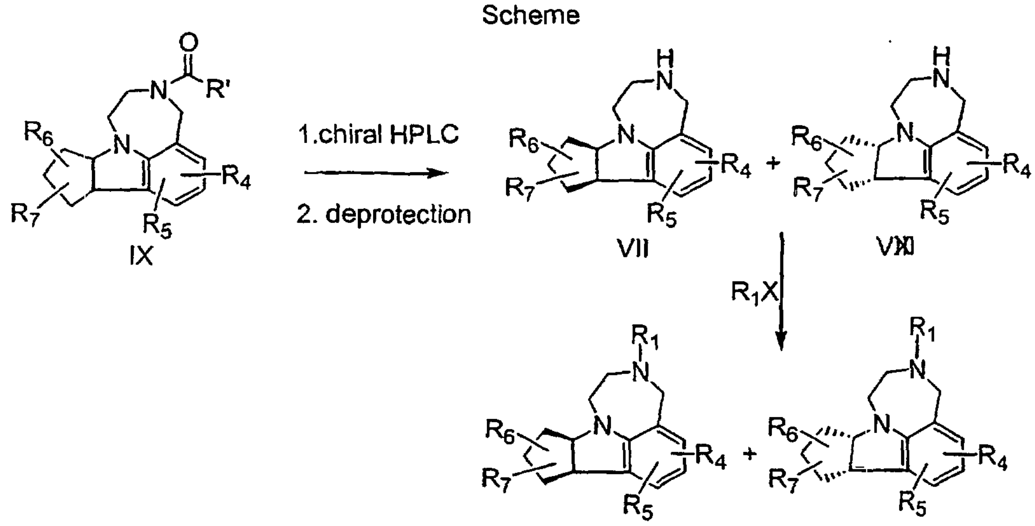


IX VII VIII

An alternate syntheic route to hydrazines **III** is described in Scheme **1A**. Substituted or unsubstituted benzodiazepines **I** are allowed to react with allyl N-[(mesitylsulfonyl)oxy]carbamate in toluene under reflux to give compounds **XII**. Compounds **XII** are allowed to react with a catalytic amount of tetrakis triphenylphosphinepalladium and a base, such as diethylamine, in an organic solvent, such as methylene chloride, to give hydrazines **III.** Hydrazines **III** are converted to fused indoles **V** as described in Scheme **1A:**

## Scheme 1A

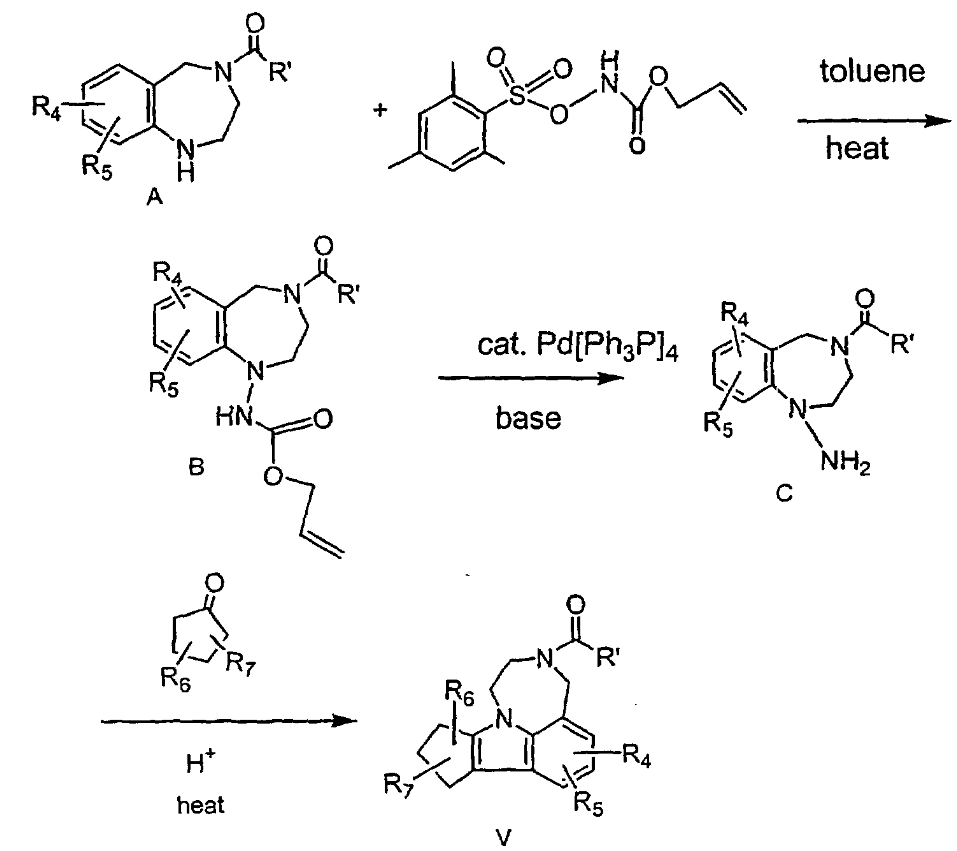


A B C V

An alternate route to hydrazones **IV** is where substituted or unsubstituted benzodiazepines **I** are allowed to react with an inorganic cyanate, such as sodium cyanate, in an organic solvent, such as acetonitrile, in the presence of an acid, such as trifluoroacetic acid, at elevated temperatures, such as 35-75°C to give a urea. Thue urea is treated with an inorganic hypochlorite, such as sodium hypochlorite, in an alcohol-water solution at 0-25 °C to give hydrazines **III.** Hydrazines **III** in solution are cooled to <25°C and treated with acetic acid and substituted or unsubstituted cyclopentanones and warmed to room temperature to give hydrazones **IV.** Hydrazones **IV** are converted to fused indoles **V** as described in Scheme 1.

It will be understood that the processes of this invention can further include analogous steps wherein the protecting group applied to the optionally substituted benzodiazepines of Formula I in Scheme 1 are other than the acetyl group used above. Other conventional protecting groups known in the art may also be used including, but not limited to alkyl and acyl chlorides, alkyl or aryl chloroformates, such as ethylchloroformate and benzyl chloroformate and dialkylcarbonates, and para-nitro benzene sulfonyl chloride.

Compounds of this invention were also prepared according to the following Scheme 2 from commercially available starting materials or starting materials which can be prepared using literature procedures.

**Scheme 2**

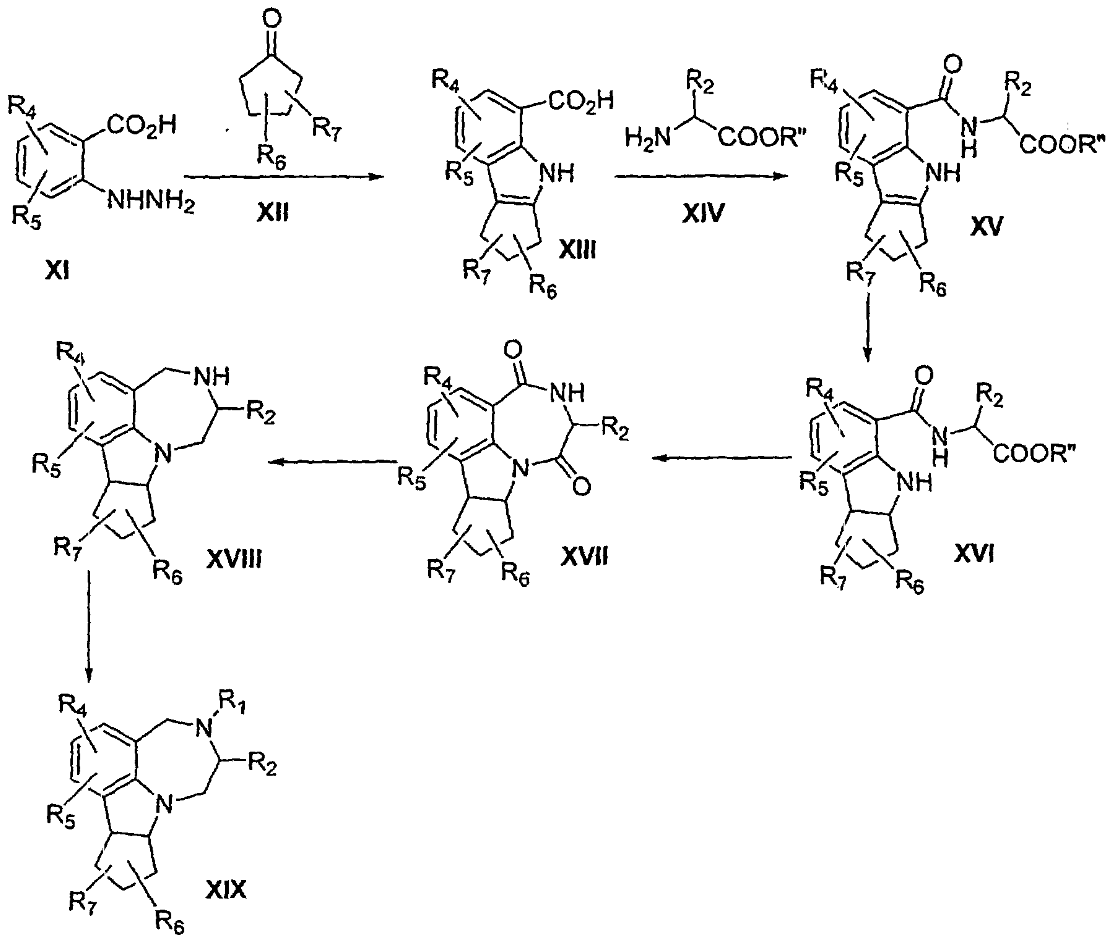

In **Scheme 2,** a 2-hydrazinobenzoic acid **XI** is allowed to react with a ketone **XII** under standard Fischer-indole conditions. The reaction is carried out in the presence of an acid, such as sulfuric acid or acetic acid, with or without a solvent, such as water or ethanol, at a temperature above ambient temperature, such as 30-150°C.

The resulting indole-carboxylic acid **XIII** is coupled with an amino acid ester **XIV,** such as L-alanine methyl ester, in the presence of peptide coupling reagents, such as 1-(3-dimethylaminopropyl)-3-ethylcarbodiimide hydrochloride (EDC) and 1-hydroxybenzotriazole hydrate (HOBt), and a base, such as diisopropylethylamine, in an inert organic solvent, such as dichloromethane.

It will be understood that the amino acid ester **XIV** may comprise any known in the art to be used in cyclization procedure as disclosed in Scheme 2. Among the most preferred are those wherein R" in formula **XIV** are represent alkyl groups of from 1 to 10 carbon atoms, either straight, branched or cyclic. Among the most preferred are the shorter chain esters, such as the methyl, ethyl, isopropyl, n-propyl, n-butyl, and t-butyl esters.

The resulting indole-amide **XV** can be reduced to indoline-amide **XVI** by catalytic hydrogenation in the presence of a metal catalyst, such as 5% Pd/C or by a hydride source, such as triethylsilane or borane, in the presence of an acid, such as trifluoroacetic acid.

The indoline-amide **XVI** can be cyclized to the bislactam **XVII** by hydrolysis of the ester with a base, such as lithium hydroxide, and subsequent treatment with an acid, such as acetic acid.

The bislactam **XVII** can be reduced to the benzodiazepine **XVIII,** which are compounds of this invention, with a reducing agent, such as borane or lithium aluminum hydride, in the presence of an inert organic solvent, such as tetrahydrofuran.

When $R_1 \neq H$, reaction of benzodiazepine **XVIII** with an alkyl halide, such as methyl iodide, or an acyl halide, such as acetyl chloride, or an aroyl chloride, such as benzoyl chloride, gives **XIX** which are also compounds of this invention.

An alternative synthetic route to compounds of this class is depicted in **Scheme 3**. An arylhydrazine **XX** is allowed to react with a ketone **XII** under standard Fischer-indole conditions. The reaction is carried out in the presence of an acid, such as sulfuric acid or acetic acid, with or without a solvent, such as water or ethanol, at a temperature above ambient temperature, such as 30-150 °C.

The resulting indole **XXI** can be reduced to indoline **XXII** by catalytic hydrogenation in the presence of a metal catalyst, such as 5% Pd/C or by a hydride source, such as triethylsilane or borane, in the presence of an acid, such as trifluoroacetic acid.

**Scheme 3**

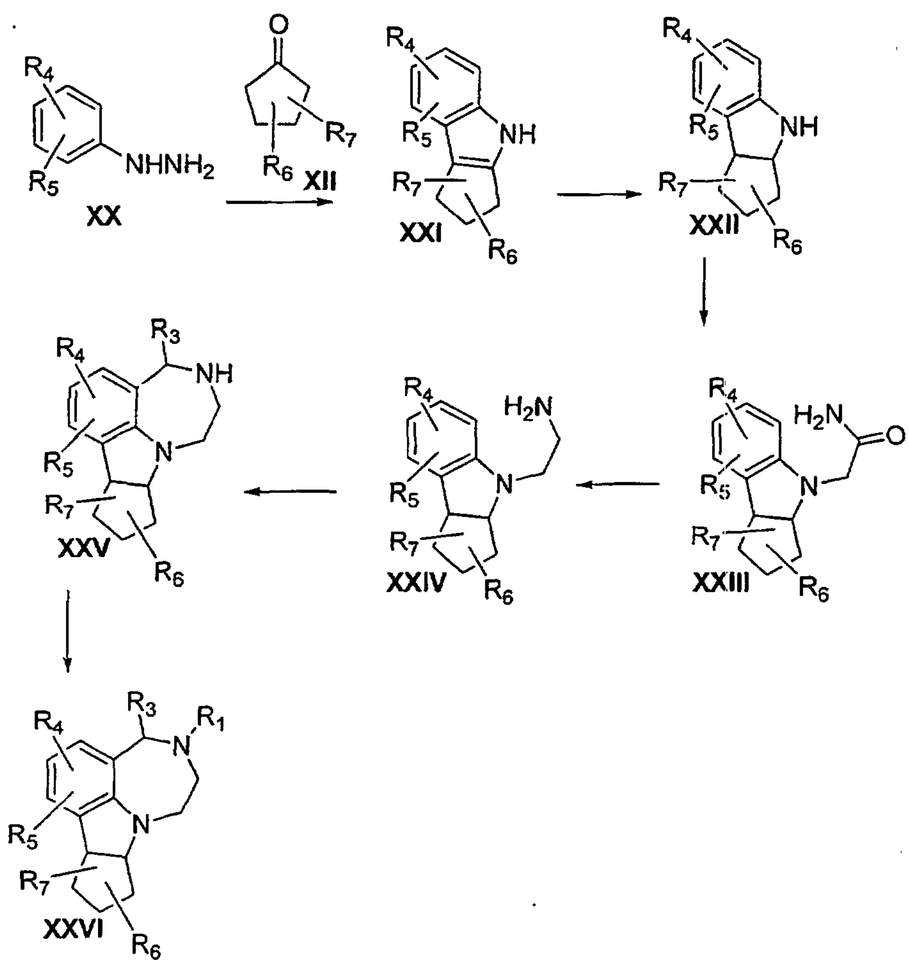
XX XII XXI XXII XXIII XXIV XXV XXVI

The indoline **XXII** can be coupled with an appropriate electrophile, such as chloroacetamide (depicted), or a corresponding synthetic equivalent such as chloroacetonitrile, etc. in the presence of a base, such as diisopropylethylamine or potassium hydroxide, in a suitable solvent, such as DMF or DMSO to give the amide **XXIII.**

The amide **XXIII** can be reduced to the amine **XXIV** with a reducing agent, such as borane or lithium aluminum hydride, in the presence of an inert organic solvent, such as tetrahydrofuran.

The amine **XXIV** can be cyclized to the benzodiazepine **XXV**, which are compounds of this invention, by treatment with an aldehyde, such as formaldehyde or acetaldehyde, in the presence of an acid, such as trifluoroacetic acid, in a suitable solvent, such as ethanol, at room temperature or elevated temperatures.

When $R_1 \neq H$, reaction of benzodiazepine **XXV** with an alkyl halide, such as methyl iodide, or an acyl halide, such as acetyl chloride, or an aroyl chloride, such as benzoyl chloride, gives **XXVI** which are also compounds of this invention.

A process of this invention is illustrated in **Scheme 3A** below:

**Scheme 3A**

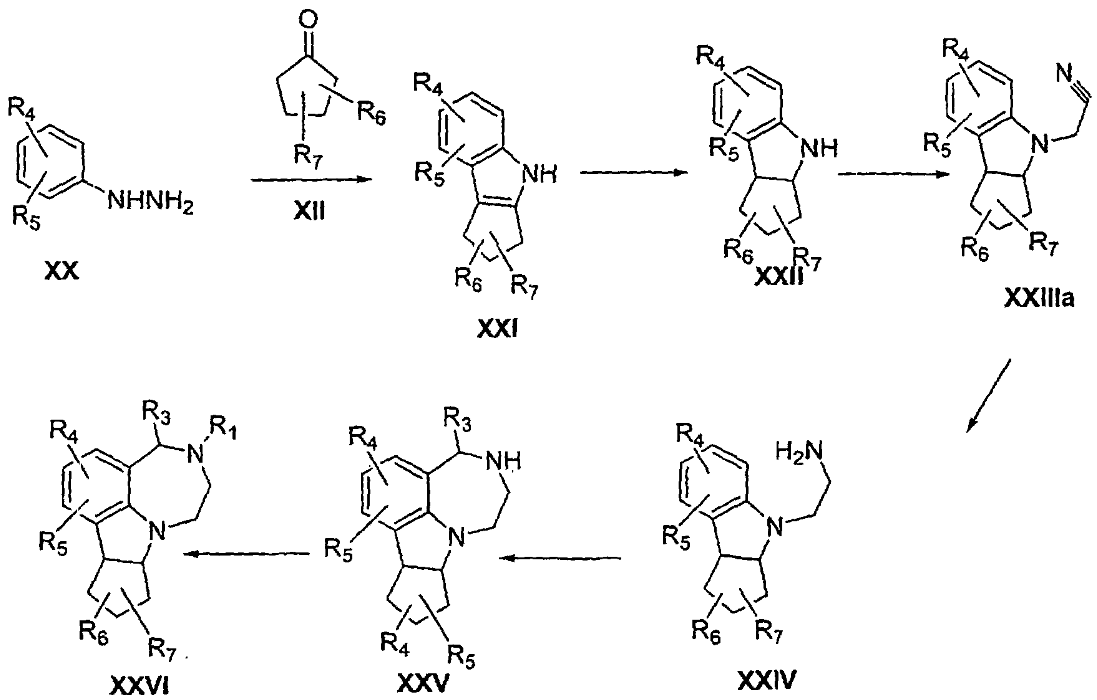

XX XXI XXII XXIIIa

XXVI XXV XXIV

In this alternative route, indoline **XXII** can be coupled with an appropriate electrophile, such as chloroacetonitrile, or a corresponding synthetic equivalent, in the presence of a base, such as diisopropylethylamine or sodium hydride, in a suitable solvent, such as DMF or DMSO to give the nitrile **XXIIIa.**

The nitrile **XXIIIa** can be reduced to the amine **XXIV** with a reducing agent, such as borane or lithium aluminum hydride, in the presence of an inert organic solvent, such as tetrahydrofuran. The nitrile **XXIIIa** can also be reduced to the amine **XXIV** by hydrogenation in the presence of a catalyst, such as palladium, in a suitable solvent, such as ethanol or ethyl acetate.

This invention also provides novel groups of compounds useful in the synthesis of the pharmaceutically useful compounds of Formula I described above. These compounds are of the general formula **XXIIa,** including **XXIII, XXIIIa** and **XXIV:**

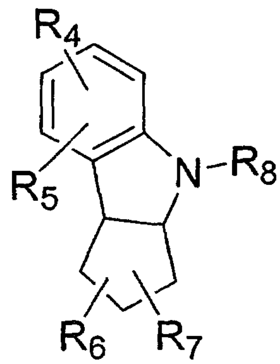

**XXIIa**

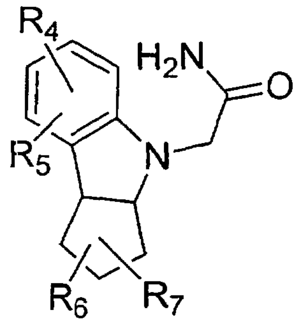

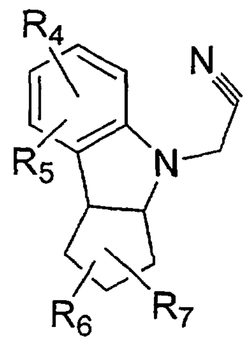

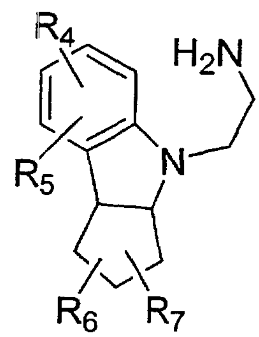

**XXIII** **XXIIIa** **XXIV**

in which formulae $R_8$ is $-CH_2-C(O)-NH_2$, $-CH_2-CN$ or $-CH_2-CH_2-NH_2$; and $R_4$, $R_5$, $R_6$ and $R_7$ are as defined herein. For example wherein $R_4$, $R_5$, $R_6$ and $R_7$ are each, independently, hydrogen, hydroxy, alkyl of 1-6 carbon atoms, cycloalkyl, alkoxy of 1-6 carbon atoms, halogen, fluorinated alkyl of from 1 to 6 carbon atoms, -CN, $-NH-SO_2$-alkyl of 1-6 carbon atoms, $-SO_2$-NH-alkyl of 1-6 carbon atoms, alkyl amide of 1-6 carbon atoms, amino, alkylamino of 1-6 carbon atoms, dialkylamino of 1-6 carbon atoms per alkyl moiety, fluorinated alkoxy of 1-6 carbon atoms, acyl of 2-7 carbon atoms, aryl or aroyl. A subset of these compounds includes those in which $R_4$ and $R_5$ are hydrogen, and $R_6$ and $R_7$ are as defined above. A further subset of these compounds are those in which $R_4$, $R_5$ and $R_6$ are hydrogen, and $R_7$ is as defined above. Preferred specific compounds are 2-(2,3,3a,8b-Tetrahydro-1*H*-cyclopenta[*b*]indol-4-yl)-acetamide, 2-(2,3,3a,8b-Tetrahydro-1*H*-cyclopenta[*b*]indol-4-yl)-acetonitrile, 2-(2,3,3a,8b-Tetrahydro-1*H*-cyclopenta[*b*]indol-4-yl)-ethylamine,

An alternate synthetic route within the scope of this invention is indicated by **Scheme 3b,** which shares the initial and final steps disclosed in **Schemes 3 and 3a.** In this method, rather than reducing the indole **XXI** to indoline **XXII,** as done in Scheme 3, the indole **XXI** is directly converted to the corresponding amide **XXIIIb** or nitrile **XXIIIc,** which can then be reduced to the corresponding amines **XXIVa** and **XXIV.**

**Scheme 3b**

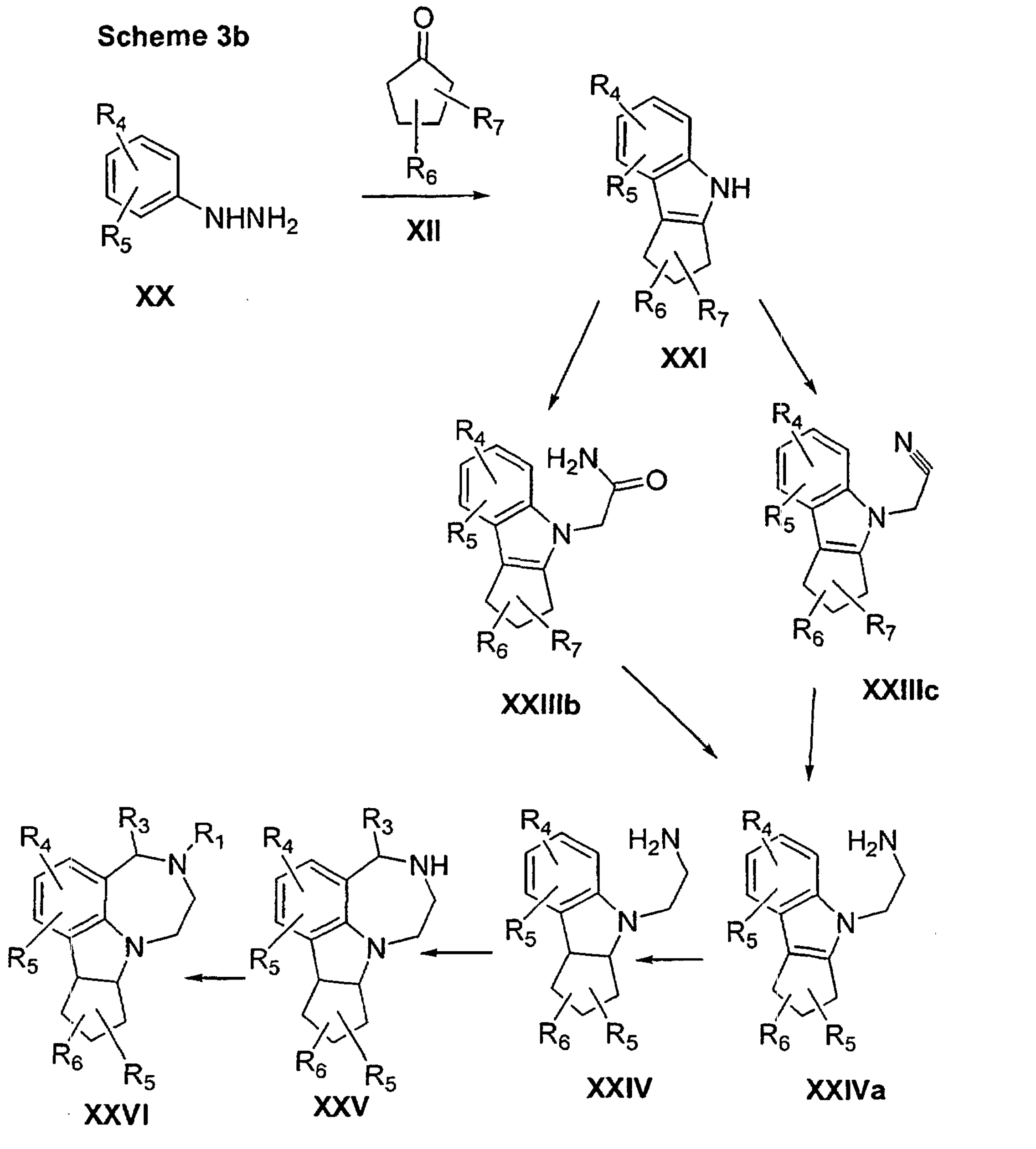

XX XII XXI XXIIIb XXIIIc XXVI XXV XXIV XXIVa

A further process for synthesizing compounds of this invention is presented in **Scheme 4,** wherein $R_1$, $R_4$, $R_5$, $R_6$ and $R_7$ are as defined above.

**Scheme 4**

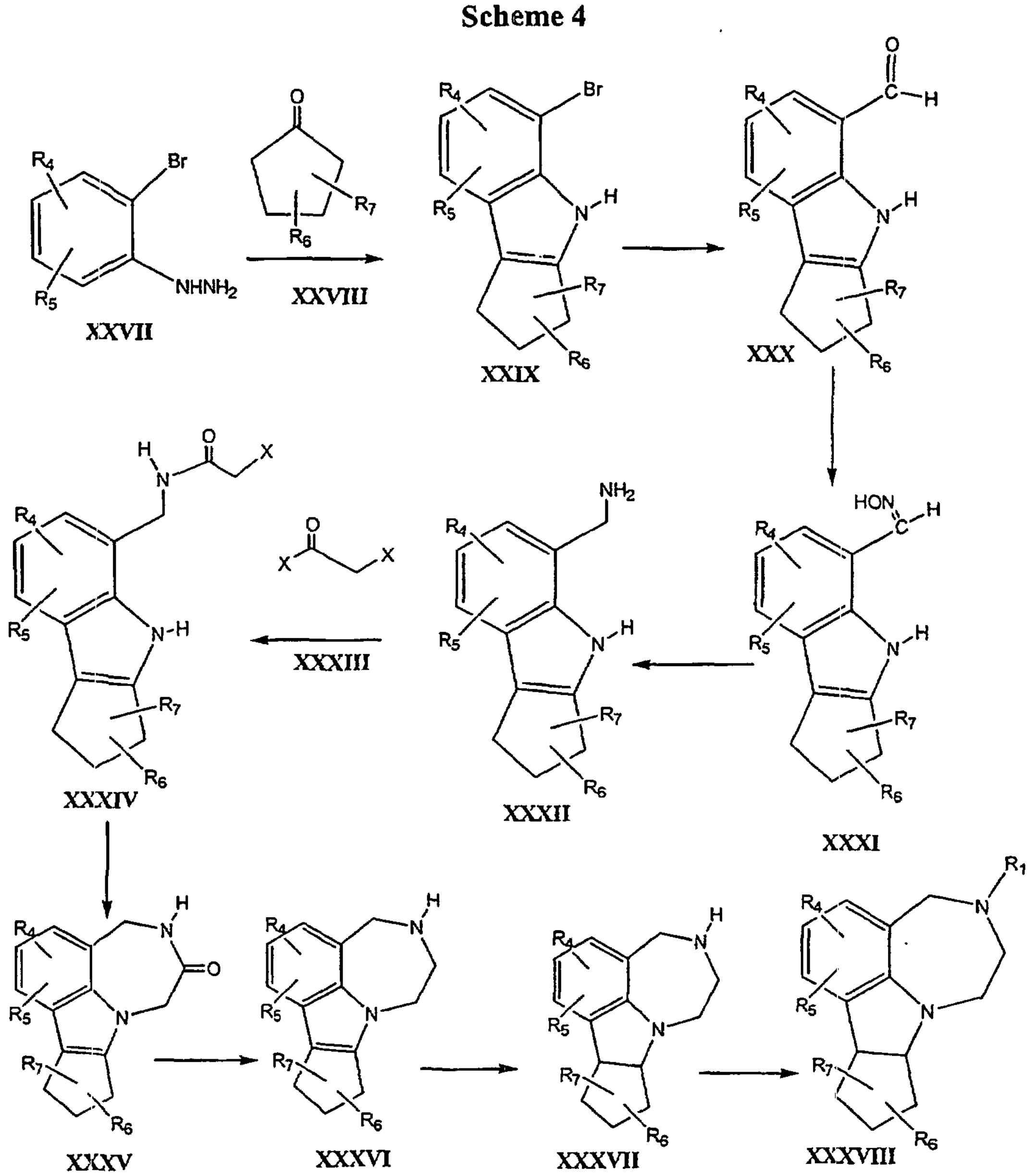


A 2-bromophenylhydrazine **XXVII** is allowed to react with a ketone **XXVIII** under standard Fisher-indole conditions. The reaction is carried out in the presence of an acid, such as sulfuric acid, acetic acid, or *p*-toluenesulfonic acid with or without a solvent, such as water, alkyl alcohol of 1-6 carbon atoms or dimethylforamide (DMF), at a temperature above ambient temperature, such as 30-150 °C.

The resulting bromoindole **XXIX** may then be lithiated with reagent such as *n*-butyl lithium and formylated with formyl transfer agents such as DMF, N-formyl morpholine or ethyl formate in an acceptable solvent such as diethyl ether ($Et_2O$) or methyl t-butyl ether (MTBE) from -78 °C to ambient temperature.

The resulting indole aldehyde **XXX** is converted to an indole oxime **XXXI** using reagent such as hydroxylamine, N- or O-benzyl protected hydroxylamine in the presence of a suitable base such as pyridine or triethylamine (TEA) in a suitable solvent such as pyridine, water or tetrahydrofuran (THF).

The resulting indole oxime **XXXI** can be reduced to indole amine **XXXII** using a hydride source such as lithium aluminum hydride (LAH) or by catalytic hydrogenation in the presence of a metal catalyst such as palladium on carbon (Pd/C) or Raney nickel.

The resulting indole amine **XXXII** can be acylated with acid halide **XXXIII,** wherein X is an acceptable leaving group, preferably a halogen, such as chloroacetyl chloride in the presence of a base such as pyridine or TEA in a

suitable solvent such as methylene chloride.

The resulting acyl indole **XXXIV** can be cyclized to indole amide **XXXV** in the presence of a suitable base such as sodium hydride (NaH), potassium hydride (KH) or lithium hydride (LiH) in the presence of a polar solvent such as THF, dimethylacetamide (DMA) or DMF.

Indole amide **XXXV** can be reduced to benzodiazepine indole **XXXVI** with reagents such as borane, LAH in a suitable solvent such as THF, $Et_2O$ or MTBE. Reduction of benzodiazepine indole **XXXVII**

Reaction of benzodiazepine **XXXVII** with an alkyl halide of 1-6 carbon atoms such as methyl iodide, or an acyl halide, such as acetyl chloride, or an aroyl chloride, such as benzoyl chloride gives benzodiazepine **XXXVIII.**

The acylation steps of this invention are understood to include reactions of the appropriate compound with any acylating agent and reaction conditions known in the art. Useful in these steps are acylating agents include acid halides and esters or anhyrides of the appropriate aliphatic carboxylic acid. Useful acid halides include acetyl chloride, propionyl chloride, isobutyryl chloride, benzoyl chloride, etc. Acid anhydrides include acetic anhydride and benzoic anhydride. Similarly, alkylation steps herein are understood to include any relevant alkylating agents and conditions known in the art. These include, but are not limited to the use of alkyl halides, such as methyl iodide, or alkyl tosylates or aldehyde alkylating agents in the presence of an applicable reducing agent.

Pharmaceutically acceptable salts can be formed from organic and inorganic acids, for example, acetic, propionic, lactic, citric, tartaric, succinic, fumaric, maleic, malonic, mandelic, malic, phthalic, hydrochloric, hydrobromic, phosphoric, nitric, sulfuric, methanesulfonic, napthalenesulfonic, benzenesulfonic, toluenesulfonic, camphorsulfonic, and similarly known pharmaceutically acceptable acids. The processes herein will be understood to include an optional additional step of forming a salt form of the products via standard addition reactions with any pharmaceutically acceptable organic or inorganic acid.

This invention also provides a method for resolving the enantiomers of the compounds described herein. A method of resolving the (R,R) enantiomer of these compounds comprises the steps of:

a) dissolving about 1 equivalent of the racemic compound mixture of a product of this invention in a solubilizing amount of an alcohol resolving agent at a temperature of from about 50°C to the reflux temperature for the alcohol, preferably between about 50°C and 70°C, under an inert atmosphere, to create a resolving solution;

b) treating the resolving solution of step a) with from about 0.1 to about 0.35 equivalents of dibenzoyl-L-tartaric acid, preferably from about 0.15 equivalents to about 0.3 equivalents, more preferably from about 0.23 to about 0.27 equivalents, most preferably about 0.25 equivalents to precipitate the desired (R,R) enantiomer from the resolving solution as the corresponding tartaric acid salt form; and

c) separating the desired enantiomer from the resolving solution through conventional means, such as filtration.

It will be understood that this process may be followed by additional steps of filtration and purification to enhance the purity and yield of the desired enantiomer product in question.

In step b) it is preferred that the temperature of the resolving solution be maintained at a temperature at or above about 50°C, preferably nearer to the reflux temperature of the alcohol in question.

The alcohol component of step a) may be comprise a single alcohol or a combination of two or more alcohols selected from those known in the art into which the compound in question can be dissolved. Among the preferred alcohols are the commercially available and relatively low boiling alcohols comprising 10 carbon atoms or less including methanol, ethanol, *n*-propanol, isopropanol, *n*-butanol, *t*-butanol, cyclohexanol, etc.

It will also be understood that the (S,S) enantiomer of the racemic mixture mentioned above could then be purified and collected from the remaining resolving solution described above after collection of the (R,R) tartaric acid salt.

This invention also provides an analogous method for resolving the (S,S) enantiomer from the racemic mixtures of compounds of this invention, the method comprising the steps a) through c) listed above, with dibenzoyl-D-tartaric acid being used in place of dibenzoyl-L-tartaric acid in step b). Comparably, the (R,R) enantiomer can be collected and purified by conventional means from the remaining solution after the tartaric acid salt form of the (S,S) enantiomer is precipitated and removed in this analogous method.

This invention also comprises pharmaceutical compositions and formulations utilizing the compounds described herein, comprising a pharmaceutically or therapeutically effective amount of one or more compounds of this invention, or a pharmaceutically acceptable salt thereof, and one or more pharmaceutically acceptable carriers or excipients.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semi-permeable membrane covering a reservoir containing the active ingredient with or without a carrier, or a matrix containing the active ingredient. Other occlusive devices are known in the literature.

A pharmaceutically or therapeutically effective amount of the compounds herein is understood to comprise an amount of the compound(s) in question which will obtain at least a minimum of desired effect in preventing, treating, inhibiting or managing the symptoms or causes of the malady in question. More preferably, the amount will be the minimum needed to alleviate or remove the undesirable physiological consequences of the malady in question and inhibit or prevent their re-occurrence.

The dosage requirements vary with the particular compositions employed, the route of administration, the severity of the symptoms presented and the particular subject being treated. Based on the results obtained in the standard pharmacological ) test procedures, projected daily dosages of active compound would be 0.02 μg/kg - 750 μg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packaged powders, vials, ampoules, pre filled syringes or sachets containing liquids. ) The unit dosage form can be, for example, a capsule or tablet itself, or it can be the appropriate number of any such compositions in package form. It will be understood that the dosage administered will be determined by a skilled medical professional taking into account the needs and physical characteristics of the recipient and the nature and extent of the malady to be treated or prevented.

The following provides the preparation of compounds representative of this invention.

**Example 1**

**1,2,3,4,9,10-Hexahydro-8H-cyclopenta[b][1,4]diazepino[6,7,1-hi]indole**

**A. 3-Acetyl-1,2,3,4,10-hexahydro-8H-cyclopenta[b][1,4]diazepino[6,7,1-hi]indole**

4-Acetyl-2,3,4,5-tetrahydro-1H-1,4-benzodiazepine (8.92 g, 46.9 mmol) was dissolved in water (110 mL) and conc. HCl (6 mL). The solution was cooled in an ice bath and a solution of $NaNO_2$ (3.20 g) in water (10 mL) was added dropwise over 10 min with stirring. After stirring an additional 20 min the solution was extracted with methylene chloride. The organic phase was dried ($K_2CO_3$), filtered, and the volatiles were removed by evaporation under reduced pressure to give a residue. The residue was dissolved in glacial acetic acid (80 mL), cooled in an ice bath. Powdered zinc (23 g) was added portionwise over 10 min while keeping the reaction temperature below 30°C. Ater stirring for an additional 1.5 h, the reaction mixture was filtered through a pad of Celite. After washing the Celite with glacial acetic acid, cyclopentanone (20 g, 952 mmol, 20 equiv.) was added to the combined filtrates and the reaction mixture was heated (oil bath, 90-105°C) for 2 h with stirring and then allowed to cool to room temperature and stir overnight.

Removal of the acetic acid by evaporation under reduced pressure, gave a residue which was partitioned between 2.5 N NaOH and ethyl acetate. The aqueous phase was extracted with additional ethyl acetate (3X) and the combined extracts were dried and evaporated under reduced pressure to give a residue. The residue was purified by column chromatography on silica gel eluting with 1-2% methanol in methylene chloride to give, 2.36 g (9.36 mmol.) of intermediate A, 3-acetyl-1,2,3,4,10-hexahydro-8H-cyclopenta[b][1,4]diazepino[6,7,1-hi]indole, as a viscous oil.

Intermediate A (2.34g, 9.20 mmol) was dissolved in MeOH . The solution was diluted with 2.5N NaOH and solid NaOH pellets were added. The reaction mixture was placed in an oil bath at 95 °C for 6 h. then cooled to room temperature and stirred overnight. The volatiles were evaporated under reduced pressure and the residue was partitioned between water and ethyl acetate. The ethyl acetate was separated and evaporated and the residue was dissolved in methylene chloride and purified by chromatography on silica gel eluting with 2-5% methanol in methylene chloride to give the product of Example 1 as a light gray solid 1.56g (80%) , mp: 66-68 °C.

| Anal. Calcd. for $C_{14}H_{16}N_2$ | | | |
|---|---|---|---|
| Theory | %C, 79.21; | %H, 7.60; | %N, 13.20. |
| Found | %C, 78.81; | %H, 7.5; | %N, 13.10 |

**Example 2**

**1,2,3,4,8,9,10,10a-Octahydro-7bH-cyclopenta[b][1,4]diazepino[6,7,1-hi]indole**

**Method A.**

The compound of Example 1 (1.56g, 7.35 mmol) was dissolved in trifluoroacetic acid ( 53 mL) and cooled in an ice/water bath. 1.5 M $BH_3$ in THF (34 mL) was added dropwise slowly and stirred for an additional 15min. after the addition was complete. Water was added slowly to quench the reaction followed by 2.5 N NaOH and 50% NaOH until the reaction mixture was basic (yellow color disappeared). After extraction with ethyl acetate (3X), the organic phase were combined and concentrated under reduced pressure to give a residue which was purified by chromatography on silica gel eluting with 3-10% MeOH in methylene chloride to give 600mg (38%) of the product as a yellow solid: mp 64-68 °C.

| Anal. Calcd for $C_{14}H_{18}N_2 \cdot 0.2\ H_2O$ | | | |
|---|---|---|---|
| Calcd | %C, 77.17; | %H, 8.51; | %N, 12.86. |
| Found | %C, 77.13; | %H, 8.18; | %N, 12.61. |

**Method B.**

**3-Acetyl-1,2,3,4,8,9,10,10a-octahydro-7bH-cyclopenta[b][1,4]diazepino[6,7,1-hi]-indole**

Intermediate A of Example 1 (2.07g, 8.12 mmol) was dissolved in EtOH and hydrogenated over 10% Pd on carbon (0.25g) in a Parr shaker at 55 lbs of hydrogen pressure. After 5 hrs, the reaction mixture was filtered through Celite to remove the catalyst and the filtrate was concentrated under reduced pressure to give a residue which was

purified by chromatography on silica gel eluting with 0.3-2% MeOH in methylene chloride to give 1.84g (87%) of 3-acetyl-1,2,3,4,8,9,10,10a-octahydro-7bH-cyclopenta[b][1,4]diazepino[6,7,1-hi]indole as a white solid, mp: 111-113ºC.

| Anal. Calcd for $C_{16}H_{20}N_2O$ + .20 $H_2O$ | | | |
|---|---|---|---|
| Theory | %C, 73.93; | %H, 7.91; | %N, 10.78. |
| Found | %C, 74.05; | %H, 7.91; | %N, 10.79. |

3-Acetyl-1,2,3,4,8,9,10,10a-octahydro-7bH-cyclopenta[b][1,4]diazepino[6,7,1-hi]indole was dissolved in conc. HCl and heated with stirring in an oil bath (110°C) for 12 hr. After cooling, the reaction mixture was made basic with 2.5N NaOH and 50% NaOH, extracted into methylene chloride, dried ($MgSO_4$), filtered and concentrated to give a residue. The residue was purified by chromatography on silica gel eluting with 1-20% MeOH in methylene chloride to give the product as a yellow solid, mp: 76-79°C.

**Example 3**

The compound of Example 2 was chromatographed on a Chiral column eluting with MeOH containing 0.1 % diethylamine.

Peak one was obtained as a yellow solid, mp: 51-54°C and identified (7bS, 10aS)-1,2,3,4,8,9,10,10a-octahydro-7bH-cyclopenta[b][1,4]diazepino[6,7,1-hi]-indole

| Anal. Calcd for $C_{14}H_{18}N_2$ +0.20 $H_2O$ | | | |
|---|---|---|---|
| Theory | %C, 78.46; | %H, 8.47; | %N, 13.07. |
| Found | %C, 77.09; | %H8.50; | %N12.72. |

Peak two was obtained as a yellow solid, mp: 43-46 °C. This peak was identified as a mixture of 92.3% (7bR, 10aR)-1,2,3,4,8,9,10,10a-octahydro-7bH-cyclopenta-[b][1,4]diazepino[6,7,1-hi]indole and 7.7% of identified (7bS, 10aS)-1,2,3,4,8,9,10,10a-octahydro-7bH-cyclopenta[b][1,4]diazepino[6,7,1-hi]indole.

**Example 4**

3-Acetyl-1,2,3,4,8,9,10,10a-octahydro-7bH-cyclopenta[b][1,4]diazepino[6,7,1-hi]indole was chromatographed on a Chiralcel AD chiral column (20 x 250 mm) eluting with 100% MeOH at room temperature, detection method: UV/VIS at 254 nm.

A. Peak one was obtained as a colorless viscous oil, identified as (7bS,10aS)-3-acetyl-1,2,3,4,8,9,10,10a-Octahydro-7bH-cyclopenta[b][1,4]diazepino[6,7,1-hi]indole.

| Anal. Calcd. for $C_{16}H_{20}N_2O$ + .60 $H_2O$ | | | |
|---|---|---|---|
| Theory | %C, 71.93; | %H, 8.00; | %N, 10.49. |
| Found | %C, 72.06; | %H, 7.79; | %N, 10.73. |

O.R.:[alpha]25/D=+118(19.2mg/mL) MeOH)

(7bS,10aS)-3-Acetyl-1,2,3,4,8,9,10,10a-octahydro-7bH-cyclopenta[b][1,4]-diazepino[6,7,1-hi]indole was treated with solid NaOH in MeOH under reflux to give (7bS,10aS)-1,2,3,4,8,9,10,10a-octahydro-7bH-cyclopenta-[b][1,4]diazepino-[6,7,1-hi]-indole mp: 54-56 °C.

O.R.: [alpha]25/D=+134.18 (10.359mg/mL, MeOH)

B. Peak two was obtained as a colorless viscous oil, identified as (7bR,10aR)-3-acetyl-1,2,3,4,8,9,10,10a-octahydro-7bH-cyclopenta[b][1,4]diazepino[6,7,1-hi]indole

| Anal. Calcd. for $C_{16}H_{20}N_2O$ + .80 $H_2O$ | | | |
|---|---|---|---|
| Theory | %C, 70.98; | %H, 8.04; | %N, 10.35 |
| Found | %C, 70.94; | %H, 7.73; | %N, 10.22 |

O.R.: [alpha]25/D=-132 (10.2mg/mL MeOH)

(7bR, 10aR)-3-Acetyl-1,2,3,4,8,9,10,10a-octahydro-7bH-cyclopenta-[b][1,4]-diazepino[6,7,1-hi]indole was treated with solid NaOH in MeOH under reflux to give 7bR,10aR)-1,2,3,4,8,9,10,10a-octahydro-7bH-cyclopenta-[b][1,4]diazepino[6,7,1-hi]-indole mp: 57-59°C

| Anal Calcd for $C_{14}H_{18}N_2$ | | | |
|---|---|---|---|
| Theory | %C,77.81; | %H,8.49; | %N, 12.96. |
| Found | %C,78.01; | %H,8.64; | %N, 12.90. |

**Example 5**

**(chiral salt resolution of 1,2,3,4,8,9,10,10a-Octahydro-7bH-cyclopenta[b][1,4]-diazepino[6,7,1-hi]indole)**

**(7bR,10aR)-1,2,3,4,8,9,10,10a-octahydro-7bH-cyclopenta-[b][1,4]diazepino[6,7,1-hi]indole**

The compound of Example 2 (10.0 g, 46.7 mmol) was dissolved in isopropanol (500 mL) at 65-70°C under nitrogen and dibenzoyl-L-tartaric acid (4.18 g, 11.7 mmol) was added all at one time. The resultant solids were slurried at 70-75°C for two h, cooled to room temperature and stored at 10°C for 12 h. The solids were filtered and washed twice with isopropanol (15 mL). The solids were reslurried in hot (80°C) isopropanol (400 mL) for 1.5 h, cooled to room temperature and. stored at 10°C for 12 h. The solids were filtered and washed twice with isopropanol (15 mL) and air dried to give 7.3g (79.9%) of the dibenzoyl-L-tartaric acid salt of the title compound as a white solid, mp: 163-5°C.

| Anal. Calcd for $C_{14}H_{18}N_2 \cdot 0.5\ C_{18}H_{14}O_8 \cdot 0.4\ C_3H_8O$ | | | |
|---|---|---|---|
| Calcd | %C, 69.60; | %H, 6.75; | %N, 6.70. |
| Found | %C, 69.80; | %H, 6.73; | %N, 6.58. |

O.R.: [alpha]25/D=-138.4 (1 mg/mL, MeOH)

The above tartrate salt (9.5 g, 12.1 mmol) was slurried in ethyl acetate (950 mL) and 1 N hydrochloric acid was added (25.0 mL, 25.0 mmol). The slurry was concentrated by atmospheric distillation (72-77°C) to a volume of 275 mL, cooled to room temperature and stored overnight at 10°C. The solids were filtered and washed twice with ethyl acetate (20 mL) and air dried to give 5.7 g (92.8 %) of the hydrochloride salt of the title compound as a white solid, mp 246-249 °C decomposed.

The HCl salt (5.6 g) was dissolved in ethanol (100 mL) at reflux. Upon cooling to room temperature, needle-like crystals formed. The mixture was stored overnight at 10°C. The solids were filtered, washed twice with ethanol (10 mL) and vacuum dried (57°C /0.1mm) to give 3.8 g (67.8 %) of white solid, mp 252-253 °C decomposed.

| Anal. Calcd for $C_{14}H_{18}N_2 \cdot HCl$ | | | |
|---|---|---|---|
| Calcd | %C, 67.05; | %H, 7.64; | %N, 11.17. |
| Found | %C, 66.74; | %H, 7.54; | %N, 11.09. |

**Example 6**

**6-Methyl-1,2,3,4,9,10-hexahydro-8H-cyclopenta[b][1,4]diazepino[6,7,1-hi]indole**

By the same procedure as described for Example 1, 4-acetyl-7-methyl-2,3,4,5-tetrahydro-1,4-benzodiazepine, (3.64 g ,17.8 mmol) was converted to 246 mg of the title compound as a white solid, mp: 168-170°C

| Anal. Calcd for $C_{15}H_{18}N_2 \cdot 0.1\ H_2O$ | | | |
|---|---|---|---|
| Calcd | %C, 78.98; | %H, 8.04; | %N, 12.28. |
| Found | %C, 78.87; | %H, 8.15; | %N, 12.04. |

## Example 7

### (2*S*)-(*rel*-7b*R*,10a*R*)-2-Methyl-1,2,3,4,8,9,10,10a-Octahydro-7b*H*-Cyclopenta[b][1,4]Diazepino[6,7,1-*hi*]Indole

### A. 1,2,3,4-Tetrahydrocyclopenta[*b*]Indole-5-Carboxylic Acid

To a stirred solution of 2-hydrazinobenzoic acid hydrochloride (53 mmol, 10.0 g) and cyclopentanone (58 mmol, 4.9 g) in 1,4-dioxane (100 mL) was added dropwise concentrated $H_2SO_4$ (~18M, 63 mmol, 3.5 mL). The resulting solution was heated to reflux for 2 hours. $^1$H NMR analysis of a crude aliquot indicated complete reaction. The reaction was allowed to cool to room temperature and then concentrated to dryness to give a red solid which was used without further purification.
$^1$H NMR (DMSO-$d_6$, 300MHz) δ 10.8(s, 1H), 7.6-7.0(m, 3H), 2.8(m, 4H), 2.4(m, 2H).

### B. Ethyl (2*S*)-2-[(1,2,3,4-Tetrahydrocyclopenta[*b*]Indol-5-ylcarbonyl)Amino]-Propanoate

To a stirred, cooled (0°C) solution of crude 1,2,3,4-tetrahydrocyclopenta[*b*]-indole-5-carboxylic acid (60 mmol, 12 g), L-alanine ethyl ester (72 mmol, 11 g), 1-[3-(dimethylamino)propyl]-3-ethylcarbodiimide hydrochloride (EDC) (72 mmol, 14 g), 1-hydroxybenzotriazole (HOBT) (72 mmol, 10 g) in $CH_2Cl_2$ (100 mL) was slowly added diisopropylethylamine (360 mmol, 46 g). The reaction mixture was stirred overnight while warming to room temperature. The reaction was concentrated *in vacuo* and the resulting oil was partitioned between water and ethyl acetate. The organic layer was washed with saturated aqueous $NH_4Cl$, $NaHCO_3$, and brine, dried over $MgSO_4$, filtered and concentrated to a brown oil. The crude material was purified by chromatography through silica gel (Biotage) eluting with 15% ethyl acetate-hexanes to afford a yellow oil (8.4 mmol, 2.5 g, 16% yield over 2 steps).
$^1$H NMR (DMSO-$d_6$, 300MHz) δ 10.9(s, 1H), 8.74(d, 1H), 7.62(d, 1H), 7.48(d, 1H), 6.98(t, 1H), 4.47(t, 1H), 4.08(m, 2H), 2.75(m, 4H), 2.40(m, 2H), 1.42(d, 3H), 1.15(t, 3H).

### C. Ethyl (2*S*)-2-[(1,2,3,3a,4,8b-Hexahydrocyclopenta[*b*]Indol-5-ylcarbonyl)-Amino]Propanoate

A solution of ethyl (2*S*)-2-[(1,2,3,4-tetrahydrocyclopenta[*b*]indol-5-ylcarbonyl)-amino]propanoate (8.4 mmol, 2.5 g) in ethanol (40 mL) was added to a mixture of 5% palladium on carbon (2 g) in ethanol (20mL). Concentrated hydrochloric acid (10 mL) was added and the resulting mixture was hydrogenated at 45 psi for 4 hours. The reaction mixture was filtered through Celite. The filter bed was washed well with ethanol and the combined filtrates were concentrated. The resulting oil was partitioned between 1 N NaOH and ethyl acetate. The organic phase was dried over $MgSO_4$, filtered and concentrated to yield an oil (1.9 g, 6.1 mmol, 73% yield).
$^1$H NMR (DMSO-$d_6$, 300MHz) δ 8.7(m, 1H), 7.63(m, 1H), 7.25(m, 1H), 6.85(m, 2H), 4.40(m, 2H), 4.10(m, 2H), 3.76 (m, 1H), 1.90(m, 2H), 1.68(m, 3H), 1.38(d, 3H), 1.31(m, 1H), 1.16(t, 3H).

### D. (2*S*)-2-[(1,2,3,3a,4,8b-Hexahydrocyclopenta[*b*]Indol-5-ylcarbonyl)Amino]-Propanoic Acid

AIM aqueous lithium hydroxide solution (13 mmol, 13 mL) was added to a solution of ethyl (2*S*)-2-[(1,2,3,3a,4,8b-hexahydrocyclopenta[*b*]indol-5-ylcarbonyl)-amino]propanoate (6.1 mmol, 1.9 g) in THF (50 mL). The reaction mixture was stirred at room temperature for 18 h. The reaction was concentrated *in vacuo* and diluted with 0.1 N HCl and ethyl acetate. The phases were separated and the organic phase was washed with water, dried over $MgSO_4$, filtered, and concentrated to give a yellow oil (1.6 g, 6.1 mmol, quantitative yield).
$^1$H NMR (DMSO-$d_6$, 300MHz) δ 12.4(s, 1H), 8.21(d, 1H), 7.43(m, 1H), 7.01(d, 1H), 6.70(br s, 1H), 6.40(t, 1H), 4.35 (m, 2H), 3.63(t, 1H), 1.88(m, 2H), 1.62(m, 4H), 1.30(d, 3H).

### E. (2*S*)-2-Methyl-2,3,8,9,10,10a-Hexahydro-7b*H*-Cyclopenta[*b*][1,4]Diazepino[6,7,1-*hi*]Indole-1,4-Dione

A solution of (2*S*)-2-[(1,2,3,3a,4,8b-hexahydrocyclopenta[*b*]indol-5-yl-carbonyl)amino]propanoic acid (6.1 mmol, 1.6 g) was dissolved in acetic acid (50 mL) and heated to reflux for 18 h. The reaction was allowed to cool to room temperature and was concentrated to dryness. The crude material was purified by flash column chromatography (silica gel; 1:1 ethyl acetate-hexanes) to provide two diastereomers: less polar product (0.88 mmol, 0.23 g, 14%) and more polar product (0.18 mmol, 45 mg, 3%). The mixed fractions were also collected to provide another 3.9 mmol (1.0 g, 64%) of material.
Less Polar Product (A)
$^1$H NMR (DMSO-$d_6$, 300MHz) δ 8.2(d, 1H), 7.61 (m, 1H), 7.46(dd, 1H), 7.16(t, 1H), 4.86(dt, 1H), 3.87(m, 2H), 1.92(m, 2H), 1.75(m, 1H), 1.58(m, 2H), 1.26(d, 3H), 1.06(m, 1H).

**(2*S*)-(*rel*-7b*R*,10a*R*)-2-Methyl-1,2,3,4,8,9,10,10a-Octahydro-7b*H*-Cyelopenta[*b*]-[1,4]Diazepino[6,7,1-*hi*]Indole**

A mixture of diastereomers of (2*S*)-2-methyl-2,3,8,9,10,10a-hexahydro-7b*H*-cyclopenta[*b*][1,4]diazepino [6,7,1-*hi*]indole-1,4-dione (3.9 mmol, 1.0 g) was suspended in 1 M $BH_3$·THF (15 mL) and heated to reflux for 18 h. After cooling to room temperature, the solution was quenched with methanol and concentrated. The resulting solid was suspended in 1 N NaOH and stirred at room temperature for 1 h. The aqueous phase was then extracted with chloroform and the combined extracts were dried over $MgSO_4$, filtered, and concentrated to give a yellow solid (3.5 mmol, 0.80 g, 90%). Flash chromatography through silica gel (gradient elution 5%-10% methanol-chloroform) afforded the two diastereomers. The less polar product was arbitrarily assigned the *R*,*R* configuration and the more polar product the *S*,*S* configuration.
Anal. Calcd. for $C_{15}H_{20}N_2$ • 1.5mol $H_2O$: C, 70.56; H, 9.08; N, 10.97.
Found: C, 70.24; H, 9.58; N, 10.81.
MS ((+))APCI, m/e (%)) 229(100, $(M+H]^+$).
IR (solid ATR, $cm^{-1}$) 2960, 2880, 2310, 1460, 1440, 1210, 1160, 1120, 1090,1070. $^1H$ NMR (DMSO-$d_6$, 400MHz) δ 7.32(t, J=7.56Hz, 1H), 7.22(d, J=7.3Hz, 1H), 7.12(d, J=7.3Hz, 1H), 4.35(m, 1H), 4.07(m, 2H), 3.82(d, J=16.84Hz, 1H), 3.62(m, 1H), 3.14(dd, J=8.54Hz, 10.74Hz, 1H), 3.0(m, 1H), 2.04-1.69(m, 4H), 1.51(m, 2H), 1.22(d, J=6.6Hz, 4H).
$[\alpha]_D$ +99 (c. 0.11, DMSO).

## Example 8

**(2*S*)-(*rel*-7b*S*,10a*S*)-2-Methyl-1,2,3,4,8,9,10,10a-Octahydro-7b*H*-Cyclopenta[*b*]-[1,4]Diazepino[6,7,1-*hi*]Indole**

Following the procedure of method 7F, the more polar material provided the product which was assigned the *S.S* configuration.
Anal. Calcd. for $C_{15}H_{20}N_2$ • 1.1mol $H_2O$: C, 72.60; H, 9.02; N, 11.29.
Found: C, 72.63; H, 8.80; N, 10.95.
MS ((+)APCI, m/e (%)) 229(100, $[M+H]^+$).
$^1H$ NMR (DMSO-$d_6$, 400MHz) δ 6.86(d, J=7.3Hz, 1H), 6.73(d, J=7.3Hz, 1H), 6.52(t, J=7.4Hz, 1H), 3.95(m, 2H), 3.82 (d, J=15.86Hz, 1H), 3.61(m, 1H), 3.41(dd, J=3.17Hz, 13.4Hz, 2H), 3.21(m, 1H), 2.83(dd, J=3.9Hz, 13.2Hz, 1H), 1.94 (m, 1H), 1.77(m, 1H), 1.55(m, 4H), 1.15(d, J=6.6Hz, 3H).
$[\alpha]_D$ +38 (c. 0.10, DMSO).

## Example 9

**(2*R*)-(*rel*-7b*R*,10a*R*)-2-Methyl-1,2,3,4,8,9,10,10a-Octahydro-7b*H*-Cyclopenta[*b*]-[1,4]Diazepino[6,7,1-*hi*]Indole**

### A. Methyl (2*R*)-2-[(1,2,3,4-Tetrahydrocyclopenta[*b*]Indol-5-ylcarbonyl)Amino]-Propanoate

Following the procedure of method 7B, employing D-alanine methyl ester (64 mmol, 8.9 g) afforded a yellow oil (4.9 mmol, 1.4 g).
$^1H$ NMR (DMSO-$d_6$, 300MHz) δ 10.8(s, 1H), 8.78(d, 1H), 7.61(d, 1H), 7.49(d, 1H), 7.0(t, 1H), 4.5(m, 1H), 3.63(s, 3H), 2.76(m, 4H), 2.42(m, 2H), 1.42(d, 3H).

### B. Methyl (2*R*)-2-[(1,2,3,3a,4,8b-Hexahydrocyclopenta[*b*]Indol-5-ylcarbonyl)-Amino]Propanoate

Following the procedure of method 7C, methyl (2*R*)-2-[(1,2,3,4-tetrahydrocyclopenta[*b*]indol-5-ylcarbonyl) amino]propanoate (4.9 mmol, 1.4 g) was hydrogenated using 5% Pd/C (1.5 g) and concentrated HCl (7 mL) in methanol (25 mL) to yield an oil (2.7 mmol, 0.77 g, 55%).
$^1H$ NMR (DMSO-$d_6$, 300MHz) δ 8.34(d, 1H), 7.44(d, 1H), 7.02(d, 1H), 6.70(s, 1H), 6.41(t, 1H), 4.39(m, 2H), 3.65(m, 1H), 3.60(s,3H), 1.89(m, 1H), 1.61(m, 4H), 1.35(d, 3H), 1.29(m, 1H).

### C. (2*R*)-2-[(1,2,3,3a,4,8b-Hexahydrocyclopenta[*b*]Indol-5-ylcarbonyl)Amino]-Propanoic Acid

Following the procedure of method 7D, methyl (2*R*)-2-[(1,2,3,3a,4,8b-hexahydrocyclopenta[*b*]indol-5-ylcarbonyl)amino]propanoate (2.7 mmol, 0.77 g) was hydrolyzed to the acid using 1 M aqueous lithium hydroxide (5.9 mL) in THF (20 mL) to yield an orange oil which was used directly in the next step.
$^1H$ NMR (DMSO-$d_6$, 300MHz) δ 12.4(br s, 1H), 8.2(d, 1H), 7.43(d, 1H), 7.01(d, 1H), 6.9(br s, 1H), 6.4(t, 1H), 4.33(m, 2H), 3.62(t, 1H), 1.89(m, 2H), 1.61 (m, 4H), 1.32(d, 3H).

### D. (2*R*)-2-Methyl-2,3,8,9,10,10a-Hexahydro-7b*H*-Cyclopenta[*b*]-[1,4]Diazepino[6,7,1-*hi*]Indole-1,4-Dione

Following the procedure of method 7E, (2*R*)-2-[(1,2,3,3a,4,8b-hexahydrocyclopenta[*b*]indol-5-ylcarbonyl) amino]propanoic acid was cyclized by refluxing in acetic acid (50 mL). Purification by flash chromatography through silica gel (elution with 5% methanol-chloroform) provided each diastereomer: less polar product (1.5 mmol, 0.39 g, 56% over 2 steps) arbitrarily assigned as the *R*,*R* configuration and more polar product (0.47 mmol, 0.11 g, 17% over 2 steps) assigned as the *S*,*S* configuration.
Less Polar Product (A)
$^1$H NMR (DMSO-$d_6$, 300MHz) δ 8.2(d, 1H), 7.62(d, 1H), 7.46(d, 1H), 7.16(t, 1H), 4.87(m, 1H), 3.88(m, 2H), 1.94(m, 3H), 1.76(m, 1H), 1.59(m, 2H), 1.28(d, 3H).

### E. (2*R*)-(*rel*-7b*R*,10a*R*)-2-Methyl-1,2,3,4,8,9,10,10a-Octahydro-7b*H*-Cyclopenta[*b*][1,4]Diazepino[6,7,1-*hi*]Indole

Following the procedure of method 7F, (2*R*)-(*rel*-7b*R*,10a*R*)-2-methyl-2,3,8,9,10,10a-hexahydro-7b*H*-cyclopenta[*b*]-[1,4]diazepino[6,7,1-*hi*]indole-1,4-dione (1.5 mmol, 0.39 g) was reduced with 1 M $BH_3$•THF (10 mL) to yield a yellow solid (0.47 mmol, 0.11 g, 31%).
Anal. Calcd. for $C_{15}H_{20}N_2$ • 0.15mol $H_2O$: C, 77.98; H, 8.86; N, 12.12.
Found: C, 77.72; H, 9.03; N, 11.89.
MS ((+)ESI, m/e(%)) 457(17, [2M+H]$^+$), 307(81, [M+H+DMSO]$^+$), 229(100, [M+H]$^+$).
IR (solid ATR, cm$^{-1}$) 3240, 2950, 2870, 1590, 1460, 1350, 1290, 1270, 740.
$^1$H NMR (DMSO-$d_6$, 400MHz) δ 6.8(d, J=7.1Hz, 1H), 6.65(d, J=7.1Hz, 1H), 6.47(t, J=7.3Hz, 1H), 3.94(m, 1H), 3.80, 3.71(ABq, $J_{AB}$=16.1Hz, 2H), 3.59(m, 1H), 3.35(dd, J=3.17Hz, 12.93Hz, 1H), 3.02(m, 1H), 2.75(dd, J=4.39Hz, 12.93Hz, 1H), 2.49(m, 1H), 1.94(m, 1H), 1.76(m, 1H), 1.56(m, 4H), 1.07(d, J=6.6Hz, 3H).
$[\alpha]_D$ -82 (c. 0.10, DMSO).

## Example 10

### (2*R*)-(*rel*-7b*S*,10a*S*)-2-Methyl-1,2,3,4,8,9,10,10a-Octahydro-7b*H*-Cyclopenta[*b*]-[1,4]Diazepino[6,7,1-*hi*]Indole

Following the procedure of method 7F, (2*R*)-(*rel*-7b*S*,10a*S*)-2-methyl-2,3,8,9,10,10a-hexahydro-7b*H*-cyclopenta[*b*]-[1,4]diazepino[6,7,1-*hi*]indole-1,4-dione (0.47 mmol, 0.11 g) was reduced with 1 M $BH_3$•THF (8 mL) to yield the product (0.27 mmol, 61 mg, 57%).
MS ((+)APCI, m/e (%)) 457(20, [2M+H]$^+$), 229(100, [M+H]$^+$).
$^1$H NMR (DMSO-$d_6$, 400MHz) δ 6.85(d, J=7.08Hz, 1H), 6.72(d, J=7.3Hz, 1H), 6.52(t, J=7.3Hz, 1H), 3.82(dd, J=5.6Hz, 9.0Hz, 1H), 3.79, 3.51(ABq, $J_{AB}$=15.1Hz, 2H), 3.70(dt, J=2.9Hz, 9.0Hz, 1H), 3.28(m, 1H), 3.06(dd, J=2.1Hz, 12.1Hz, 1H), 2.78(m, 1H), 2.43(m, 1H), 1.92-1.30(m, 6H), 1.02(d, J=6.6Hz, 3H).

## Example 11

### (2*R*,7b*S*,10a*S*)-1,2,3,4,8,9,10,10a-Octahydro-7b*H*-Cyclopenta[*b*][1,4]Diazepino[6,7,1-*hi*]Indol-2-ylmethanol

### A. Methyl (2*S*)-3-Hydroxy-2-[(1,2,3,4-Tetrahydrocyclopenta[*b*]Indol-5-yl-carbonyl)Amino]Propanoate

Following the procedure of method 7B, employing L-serine methyl ester (64 mmol, 9.9 g) afforded a yellow solid (8.9 mmol, 2.7 g, 14%).
$^1$H NMR (DMSO-$d_6$, 300MHz) δ 10.8(s, 1H), 8.51(d, 1H), 7.61(d, 1H), 7.51(d, 1H), 7.01(t, 1H), 5.08(m, 1H), 4.56(q, 1H), 3.82(d, 2H), 3.62(s, 3H), 2.76(m, 4H), 2.42(m, 2H).

### B. Methyl (2*S*)-2-[(1,2,3,3a,4,8b-Hexahydrocyclopenta[*b*]Indol-5-ylcarbonyl)-Amino]-3-Hydroxypropanoate

Following the procedure of method 7C, methyl (2*S*)-3-hydroxy-2-[(1,2,3,4-tetrahydrocyclopenta[*b*]indol-5-ylcarbonyl)amino]propanoate (8.9 mmol, 2.7 g) was hydrogenated using 5% Pd/C (2 g) and concentrated HCl (10 mL) in methanol (25 mL) to yield the crude product (8.9 mmol, 2.7 g, quantitative).
$^1$H NMR (DMSO-$d_6$, 300MHz) δ 8.3(d, 1H), 7.86(d, 1H), 7.34(d, 1H), 7.03(t, 1H), 5.80(br s, 2H), 4.45(m, 2H), 3.80(m, 2H), 3.61(s, 3H), 2.0-1.55(m, 6H).

**C. (2*S*)-2-[(1,2,3,3a,4,8b-Hexahydrocyclopenta[*b*]Indol-5-ylcarbonyl]Amino}-3-Hydroxypropanoic Acid**

Following the procedure of method 7D, methyl (2*S*)-2-[(1,2,3,3a,4,8b-hexahydrocyclopenta[*b*]indol-5-ylcarbonyl)amino]-3-hydroxypropanoate (8.9 mmol, 2.7 g) was hydrolyzed to the acid using 1 M aqueous lithium hydroxide (40 mL) in THF (40 mL) to yield a red oil (1.5 mmol, 430 mg, 17%).
$^1$H NMR (DMSO-$d_6$, 300 MHz) δ 7.92(d, 1H), 7.40(d, 1H), 7.03(d, 1H), 6.43(t, 1H), 4.39(m, 2H), 3.63(br m, 4H), 2.0-1.2 (m, 6H).

**D. (2*S*)-2-(Hydroxymethyl)-2,3,8,9,10,10a-Hexahydro-7b*H*-Cyclopenta[*b*]-[1,4]-Diazepino[6,7,1-*hi*]Indole-1,4-Dione**

Following the procedure of method 7E, (2*S*)-2-[(1,2,3,3a,4,8b-hexahydrocyclopenta[*b*]indol-5-ylcarbonyl)amino]-3-hydroxypropanoic acid (1.5 mmol, 430 mg) was cyclized by refluxing in acetic acid (40 mL). Purification by flash chromatography through silica gel (elution with 5% methanol-chloroform) provided each diastereomer: less polar product (0.55 mmol, 0.15 g, 37%) arbitrarily assigned as the *R*,*R* configuration and more polar product (0.26 mmol, 0.070 g, 17%) assigned as the *S,S* configuration.
Less Polar Product (A)
$^1$H NMR (DMSO-$d_6$, 300 MHz) δ 8.44(d, 1H), 7.63(d, 1H), 7.48(d, 1H), 7.19(m, 1H), 4.88(m, 1H), 4.39(dd, 1H), 4.22 (t, 1H), 4.07(m, 1H), 3.91(m, 1H), 2.0-1.5(m, 6H).

**E. (2*R*)-(*rel*-7b*S*,10a*S*)-1,2,3,4,8,9,10,10a-Octahydro-7b*H*-Cyclopenta[*b*][1,4]-Diazepino[6,7,1-*hi*]Indol-2-ylmethanol**

Following the procedure of method 7F, (2*S*)-(*rel*-7b*S*,10a*S*)-2-hydroxymethyl-2,3,8,9,10,10a-hexahydro-7b*H*-cyclopenta[*b*]-[1,4]diazepino[6,7,1-*hi*]indole-1,4-dione (0.26 mmol, 0.070 g) was reduced with 1 M $BH_3$•THF (1 mL) to yield a solid (0.17 mmol, 0.046 g, 65%).
MS ((+)APCI, m/e(%)) 323(35, $[M+H+DMSO]^+$, 245(100, $[M+H]^+$).
$^1$H NMR (DMSO-$d_6$, 400MHz) δ 6.85(d, J=7.1Hz, 1H), 6.73(d, J=7.3Hz, 1H), 6.52(t, J=7.3Hz, 1H), 4.70(m, 1H), 3.86, 3.50(ABq, $J_{AB}$=14.9Hz, 2H), 3.85(m, 1H), 3.70(dt, J=2.9Hz, 9.0Hz, 1H), 3.35(m, 1H), 3.22(m, 2H), 2.64(m, 1H), 2.40 (m, 1H), 1.90(m, 1H), 1.75(m, 1H), 1.60(m, 2H), 1.50(m, 1H), 1.40(m, 2H).

**Example 12**

***rel*-(4*S*,7b*S*,10a*S*)-4-Methyl-1,2,3,4,8,9,10,10a-Octahydro-7b*H*-Cyclopenta[*b*][1,4]-Diazepino[6,7,1-*hi*]Indole**

**A. 1,2,3,4-Tetrahydrocyclopenta[*b*]Indole**

Concentrated sulfuric acid (~18 M, 35 mL) was added dropwise to a mixture of phenyl hydrazine (510 mmol, 50 mL) and cyclopentanone (45 mL, 510 mmol) in water (250 mL). The resulting mixture was heated to reflux for 30 min and then allowed to cool to room temperature. The liquid was decanted from the reaction mixture leaving a red, gummy solid. Hexanes (500-600 mL) was added to the flask and the mixture was heated to reflux. The yellow hexane solution was decanted hot from the mixture and placed in the freezer (crystallization begins immediately). More hexanes is added to the flask and the procedure repeated two more times using a total volume of 1500 mL of hexanes. After 1 h in the freezer, the solid was collected from the flasks and dried providing the known indole (410 mmol, 65 g, 80%).
Anal. Calcd. for $C_{11}H_{11}N$: C, 84.04; H, 7.05; N, 8.91
Found: C, 83.92; H, 7.12; N, 8.85

**B. 1,2,3,3a,4,8b-Hexahydrocyclopenta[*b*]Indole**

A mixture of 1,2,3,4-tetrahydrocyclopenta[*b*]indole (11 mmol, 1.8 g), 5% Pd/C (0.5 g), and concentrated hydrochloric acid (1.2 mL) was hydrogenated at 45 psi on a Parr shaker. After 3 h, the mixture was removed from the shaker and filtered through Celite. The solid bed was washed with methanol. The filtrate was concentrated. The crude oil was dissolved in 1 N HCl and washed with ether. The aqueous phase was treated wi.th 2.5 N NaOH to pH >10 and then extracted with chloroform. The combined chloroform extracts were dried over $MgSO_4$, filtered and. concentrated to give the crude indoline. The material was purified by flash column chromatography through silica gel (Biotage, elution with 10% ethyl acetate-hexanes) to give the known indoline (7.6 mmol, 1.2 g, 69%) as a clear oil.
Anal. Calcd. for $C_{11}H_{13}N$: C, 82.97; H, 8.23; N, 8.80
Found: C, 82.61; H, 8.35; N, 8.72

### C. 2-(2,3,3a,8b-Tetrahydrocyclopenta[*b*]Indol-4(1*H*)-yl)Acetamide

To a stirred solution of 1,2,3,3a,4,8b-hexahydrocyclopenta[*b*]indole (130 mmol, 21 g) in DMF (50 mL) was added diisopropylethylamine (400 mmol, 70 mL) followed by 2-chloroacetamide (270 mmol, 25 g). The reaction mixture was heated to 100°C for 18 h. The reaction was concentrated and the diluted with ethyl acetate and water. The phases were separated and the organic phase was washed with brine, dried over $MgSO_4$, filtered and concentrated. The crude material was purified by flash column chromatography through silica gel (elution with 60% ethyl acetate-hexanes) to afford a yellow solid (90 mmol, 20 g, 69%).
Anal. Calcd. for $C_{13}H_{16}N_2O$: C, 72.19; H, 7.46; N, 12.95.
Found: C, 72.45; H, 7.57; N, 12.64. MS ((+)APCI, m/e(%)) 217(100, $[M+H]^+$).
IR (solid ATR, $cm^{-1}$) 3450, 2930, 2870, 1680, 1480, 1150, 740.
$^1H$ NMR (DMSO-$d_6$, 400MHz) δ 7.20(s, 1H), 7.05(s, 1H), 6.90(m, 2H), 6.48(dt, J=0.73Hz, 7.3Hz, 1H), 6.18(d, J=7.8Hz, 1H), 4.22(m, 1H), 3.70, 3.58(ABq, $J_{AB}$=17.1Hz, 2H), 3.64(m, 1H), 1.90(m, 1H), 1.78(m, 1H), 1.56(m, 3H), 1.40(m, 1H).

### D. 2-(2,3,3a,8b-Tetrahydrocyclopenta[*b*]Indol-4(1*H*)-yl)Ethylamine

2-(2,3,3a,8b-Tetrahydrocyclopenta[*b*]i ndol-4(1*H*)-yl)acetamide (90 mmol, 20 g) was dissolved in 1 M $BH_3$•THF (200 mL) and heated to reflux for 18 h. The reaction mixture was allowed to cool to room temperature and then quenched slowly with methanol. The solution was concentrated, dissolved in methanol, and again concentrated. The resulting oil was diluted with ether and extracted twice with 1 N HCl. The aqueous phase was treated with 2.5 N NaOH to pH >10 and extracted with chloroform. The combined chloroform extracts were dried over $MgSO_4$, filtered and concentrated to provide a yellow oil which was used without further purification.
Anal. Calcd. for $C_{13}H_{18}N_2$ • 0.55mol $H_2O$: C, 73.58; H, 9.07; N, 13.20. Found: C, 73.62; H, 8.80; N, 12.83.
MS (EI, m/e(%)) 202(10, $M^+$), 172(100), 130(20).
IR (film ATR, $cm^{-1}$) 2950, 2870, 1605, 1480, 1250(br), 730.
$^1H$ NMR (DMSO-$d_6$, 400MHz) δ 6.89(dd, J=0.73Hz, 7.3Hz, 2H), 6.42(dt, J=0.73Hz, 7.3Hz, 1H), 6.29(d, J=7.6Hz, 1H), 4.12(m, 1H), 3.62(dt, J=2.4Hz, 9.0Hz, 1H), 3.08(m, 2H), 2.65(m, 2H), 1.90(m, 1H), 1.75(m, 1H), 1.58(m, 3H), 1.43(m, 1H).

### E. *rel*-(4*S*,7b*S*,10a*S*)-4-Methyl-1,2,3,4,8,9,10,10a-Octahydro-7b*H*-Cyclopenta[*b*]-[1,4]Diazepino[6,7,1-*hi*]Indole

2-(2,3,3a,8b-tetrahydrocyclopenta[*b*]indol-4(1*H*)-yl)ethylamine (4.9 mmol, 1.0 g) was dissolved in ethanol (25 mL) at room temperature and trifluoroacetic acid (4.9 mmol, 380 µL) was added, followed by acetaldehyde (4.9 mmol, 280 µL). The reaction was heated to reflux overnight. The reaction vessel was cooled and then concentrated *in vacuo*. The resulting oil was partitioned between $CHCl_3$ and 1N NaOH. The aqueous phase was extracted again with $CHCl_3$. The combined organics were washed with 1N NaOH, dried over $MgSO_4$, filtered and concentrated *in vacuo* to yield a brown oil (23.8 mmol). The crude product was purified by flash chromatography ($SiO_2$) eluting with 10%MeOH/$CHCl_3$ to give the two racemic diastereomers.
Less Polar Product:
Rf 0.5 (A) 10%$Et_3N$/EtOAc
MS ((+)APCI, m/e(%)) 229(100, $[M+H]^+$).
IR (film ATR, $cm^{-1}$) 2940, 2860, 1600, 1460, 1430, 1330, 1300, 1230, 1070, 750.
$^1H$ NMR (DMSO-$d_6$, 400MHz) δ 6.88(d, J=7.3Hz, 1H), 6.81(d, J=7.8Hz, 1H), 6.59(t, J=7.4Hz, 1H), 3.87(m, 1H), 3.71 (m, 1H), 3.45(q, J=6.7Hz, 1H), 3.06(m, 2H), 2.80-2.67(m, 2H), 2.05(br m, 1H), 1.90(m, 1H), 1.75(m, 1H), 1.64(m, 1H), 1.52(m, 2H), 1.37 (m, 1H), 1.37(d, J=6.8Hz, 3H).

## Example 13

### *rel*-(4*R*,7b*S*,10a*S*)-4-Methyl-1,2,3,4,8,9,10,10a-Octahydro-7b*H*-Cyclopenta[*b*][1,4]Diazepino[6,7,1-*hi*]Indole

The title product was prepared in Example 12E isolating the more polar product.
Rf0.39(B) 10%$Et_3N$/EtOAc
MS ((+)APCI, m/e(%)) 229(100, $[M+H]^+$).
IR (film ATR, $cm^{-1}$) 2950, 2930, 2860, 1600, 1460, 1430, 1320, 1230, 1050, 750.
$^1H$ NMR (DMSO-$d_6$, 400MHz) δ6.81(d, J=7.1Hz, 1H), 6.71(d, J=7.6Hz, 1H), 6.52(t, J=7.3Hz, 1H), 4.0(q, J=6.9Hz, 1H), 3.82(dd, J=4.7Hz, 8.6Hz, 1H), 3.68(dt, J=2.7Hz, 8.9Hz, 1H), 3.05(m, 2H), 2.82-2.70(m, 2H), 2.3(br m, 1H), 1.88(m, 1H), 1.78(m, 1H), 1.64-1.48(m, 3H), 1.40(m, 1H), 1.17(d, J=7.1Hz, 3H).

**Example 14**

**Fisher Indole Synthesis of 5-Bromo-1,2,3,4-Tetrahydrocyclopenta[*b*]Indole:**

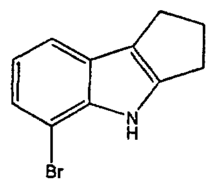

A mixture of 2-bromophenylhydrazine hydrochloride (130 g, 0.582 mol) and cyclopentanone (60 mL, 0.678 mol) in 4% sulfuric acid (1 L) was heated to 98-100°C for 6 h. This was then allowed to cool to rt overnight. After 12 h, the liquid was decanted leaving a red, gummy solid. (5:1) Hexane:EtOAc (1.5 L) was added to the flask and the mixture was hot filtered. The organic layer was concentrated *in vacuo* to give a brown oil which solidified upon standing to give 77.4 g (59%) of a brown solid. $R_f$ = 0.54 (hexane:EtOAc);
$^1$H NMR ($CDCl_3$) δ 8.12 (d, J=8.6 Hz, 1H), 7.76 (m, 2H), 7.51 (d, J=9 Hz, 1H), 7.20-7.60 (m, 4H), 4.5-4.6 (m, 1H), 4.2-4.4 (m, 3H), 4.1-4.2 (m, 1H), 2.70 (s, 3H), 2.39 (s, 3H);
$^{13}$C NMR (DMSO) δ 144.8, 139.6, 126.4, 123.2, 121.4, 121.0, 118.1, 104.9, 29.0, 26.3, 25.0;
IR (KBr): $\upsilon_{max}$ 3436, 2941, 2856, 1616, 1575, 1463, 1416, 1295, 1210, 1094, 768, 729, 629, 463 $cm^{-1}$.
Analysis for $C_{11}H_{10}BrN$: Calculated: C 55.91 H 4.24 N 5.93
Found: C 57.13 H 4.22 N 5.97

**Example 15**

Formylation to 1,2,3,4-Tetrahydrocyclopenta[*b*]Indole-5-Carbaldehyde:

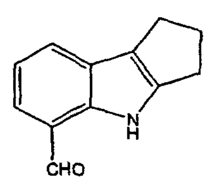

A solution of 5-bromo-1,2,3,4-tetrahydrocyclopenta[*b*]indole (15.5 g, 65.1 mmol) in MTBE (130 mL) was cooled to 0-5°C in an ice-bath before *n*-BuLi in hexanes (2.5 M, 57.5 mL, 144 mmol) was added slowly while keeping the reaction mixture below 20°C. After 30 min, DMF (50.5 mL, 0.652 mmol) was added slowly. Some solid precipitate formed upon addition. This was stirred under these conditions for an additional 1 h then the ice-bath was removed to allowed the reaction mixture to warm to rt. MTBE (130 mL) and water (100 mL) were added. The two layer were separated. The aqueous layer was extracted with MTBE (2 x 50 mL). The combined organic layers were dried over $Na_2SO_4$, filtered and concentrated *in vacuo* to afford 15.4 g (127%) of crude product as a light brown solid.
$R_f$= 0.41 (5:1 hexane:EtOAc);
$^1$H NMR ($CDCl_3$) δ 10.1 (s, 1H), 9.9 (bs, 1H), 7.71 (d, J=7.8 Hz, 1H), 7.52 (d, J=7.4 Hz, 1H), 7.19 (t, J=7.6 Hz, 1H), 2.7-3.0 (m, 4H), 2.4-2.6 (m, 2H);
$^{13}$C NMR ($CDCl_3$) δ 193.7, 146.0, 138.5, 127.1, 125.7, 120.3, 119.7, 118.9, 28.8, 25.7, 24.2; GC/MS 185, 158, 128, 115, 102, 91, 77, 70, 63, 57, 5 1, 45, 39, 39;
IR (KBr): $\upsilon_{max}$ 3394, 2909, 2862, 2799, 2727, 1668, 1565, 1472, 1352, 1185, 1124, 780, 662 $cm^{-1}$.

**Example 16**

**Preparation of 1,2,3,4-Tetrahydrocyclopenta[*b*]Indole-5-Carbaldehyde Oxime:**

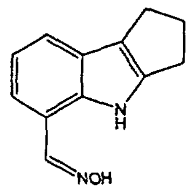

A mixture of 1,2,3,4-tetrahydrocyclopenta[*b*]indole-5-carbaldehyde (100 mg, 0.54 mmol) and hydroxylamine hydrochloride (60 mg, 0.86 mmol) in (2:1) pyridine:$H_2O$ (4.5 mL) was stirred at room temperature. After 16 h, $CHCl_3$ (25 mL) and $H_2O$ (15 mL) were added to the reaction mixture. The two layers were separated. The organic layer was extracted with 1 N HCl (2 x 10 mL), $H_2O$ (10 mL), dried over $Na_2SO_4$, filtered and concentrated *in vacuo* to afford 117 mg (108%) of crude product as a brown oil.
$R_f$= 0.50 (20:1 $CHCl_3$:$CH_3OH$);
$^1H$ NMR ($CDCl_3$) δ 9.50 (bs, 1H), 8.23 (s, 1H), 7.3-7.5 (m, 1H), 6.8-7.0 (m, 2H), 2.6-2.8 (m, 4H), 2.2-2.5 (m, 2H).

**Example 17**

**Oxime Reduction to 1,2,3,4-Tetrahydrocyclopenta[*b*]Indol-5-ylmethylamine:**

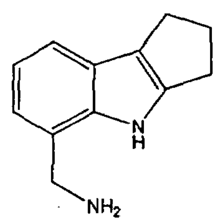

A solution of 1,2,3,4-tetrahydrocyclopenta[*b*]indole-5-carbaldehyde oxime (1.6 g, 8.1 mmol) in THF (30 mL) was cooled to 0-5 °C in an ice-bath under Ar. LAH (0.93 g, 24.5 mmol) was added in small portions. After 2 h, the ice-bath was removed to warm the reaction mixture to rt over 16 h before a sat'd $Na_2SO_4$ solution (1.5 mL) was added dropwise to quench the excess reagent. After 30 min of stirring, solid $Na_2SO_4$ and $Et_2O$ (30 mL) were added. The suspension was filtered and concentrated *in vacuo* to give 1.05 g (69%) of the title compound as a yellow solid.
$^1H$ NMY, ($CDCl_3$) δ 9.3 (bs, 1H), 7.34 (d, J=7.8 Hz, 1H), 6.99 (t, J=7.5 Hz, 1H), 6.89 (d, J=7.1 Hz, 1H), 4.22 (s, 2H), 2.7-3.0 (m, 4H), 2.4-2.6 (m, 2H), 1.6 (bs, 2H);
$^{13}C$ NMR ($CDCl_3$) δ 144.0, 140.0, 125.1, 125.2, 119.0, 118.9, 117.4, 45.3, 28.8, 25.9, 24.1; IR (KBr): $\upsilon_{max}$ 3417, 3352, 3289, 3055, 2897, 2849, 1615, 1582, 1419, 1325, 1229, 1129, 1000, 897, 741 $cm^{-1}$.
Analysis for $C_{12}H_{14}N_2$: Calculated: C 77.42 H 7.53 N 15.05
Found: C 74.60 H 7.31 N 13.52

**Example 18**

**One-Pot Reductive Amination to 1,2,3,4-Tetrahydrocyclopenta[*b*]Indol-5-yl-methylamine:**

A mixture of 1,2,3,4-tetrahydrocyclopenta[*b*]indole-5-carbaldehyde (578 mg, 3.12 mmol), hydroxylamine hydrochloride (238 mg, 3.42 mmol) and pyridine (3 mL) in THF (15 mL) was stirred at rt. After 5.5 h, the reaction mixture was cooled to 0-5°C in an ice-bath before LAH power (474 mg, 12.5 mmol) was added portionwise. The reaction mixture was then warmed to rt overnight before a sat'd $Na_2SO_4$ solution (1 mL) was added dropwise to quench the excess reagent. After 30 min of stirring, solid $Na_2SO_4$ and $Et_2O$ (30 mL) were added. The suspension was filtered and

concentrated *in vacuo* to give 338 mg (58%) of the title compound as a yellow solid.

**Example 19**

**Acvlation to 2-Chloro-N-(1,2,3,4-Tetrahydrocyclopenta[*b*]Indol-5-ylmethyl)-Acetamide:**

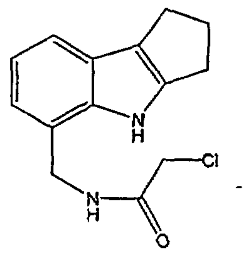

A solution of 1,2,3,4-tetrahydrocyclopenta[*b*]indol-5-ylmethylamine (100 mg, 0.538 mmol) and pyridine (0.1 mL, 1.23 mmol) in $CH_2Cl_2$ (2 mL) was cooled to 0-5°C in an ice-bath under Ar before chloroacetyl chloride (62 μL, 0.59 mmol) was added. After 1 h, the ice-bath was removed to allow the reaction to warm to rt. After an additional 12 h, $H_2O$ (3 mL) and $CHCl_3$ (3 mL) were added. The two layers were separated. The organic layer was dried over $Na_2SO_4$, filtered and concentrated *in vacuo*. Purification on $SiO_2$, eluted with (40:1) $CHCl_3$:$CH_3OH$ gave 80 mg (57%) of the title compound as a yellow solid.
$R_f$= 0.52 (40:1 $CHCl_3$:$CH_3OH$);
$^1H$ NMR ($CDCl_3$) δ 9.37 (bs, 1H), 7.41 (d, J=7.5 Hz, 1H), 7.16 (bs, 1H), 7.01 (t, J=7.3 Hz, 1H), 6.96 (d, J=7.8 Hz, 1H), 4.68 (d, J=6.8 Hz, 2H), 4.08 (s, 2H), 2.7-3.0 (m, 4H), 2.4-2.6 (m, 2H);
$^{13}C$ NMR ($CDCl_3$) δ 167.6, 145.0, 139.8, 125.4, 121.4, 121.2, 120.9, 120.2, 119.4, 43.0,41.9,29.2,26.4,24.8.

**Example 20**

**Seven-Membered Ring Closure to 3,4,9,10-Tetrahydro-8*H*-Cyclopenta[*b*][1,4]-Diazepino [6,7,1-*hi*]Indol-2(1*H*) -One:**

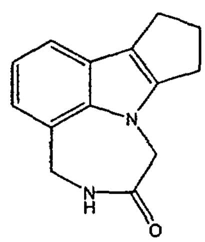

To a suspension of NaH (124 mg, 3.10 mmol) in DMF (3 mL), 2-chloro-N-(1,2,3,4-tetrahydrocyclopenta[*b*] indol-5-ylmethyl)acetamide (135 mg, 0.515 mmol) in DMF (3 mL) was added at rt under Ar. After 16 h, $H_2O$ (2 mL) was added to quench the excess reagent, $H_2$ evolved upon addition. After an additional 30 min, $CHCl_3$ (20 mL) and $H_2O$ (10 mL) were added. The two layers were separated. The aqueous layer was extracted with $CHCl_3$ (10 mL). The combined organic layers were dried over $Na_2SO_4$, filtered and concentrated *in vacuo*. Purification on $SiO_2$, eluted with (30:1) $CHCl_3$:$CH_3OH$ gave 67 mg (58%) of the title compound as a brown solid.
$R_f$ = 0.53 (20:1 $CHCl_3$:$CH_3OH$);
$^1H$ NMR ($CDCl_3$) δ 7.99 (bs, 1H), 7.33 (d, J=7.8 Hz, 1H), 6.95 (t, J=7.2 Hz, 1H), 6.75 (d, J=7.2 Hz, 1H), 4.87 (s, 2H), 4.56 (d, J=6.2 Hz, 2H), 2.7-3.0 (m, 4H), 2.4-2.6 (m, 2H);
$^{13}C$ NMR ($CDCl_3$) δ 170.0, 146.6, 139.4, 125.1, 121.4, 119.6, 119.2, 118.8, 118.5, 51.3, 44.5, 28.7, 25.1, 25.0.

**Example 21**

**Reduction to 3,4,9,10-Tetrahydro-8*H*-Cyclopenta[*b*][1,4]Diazepino [6,7,1-*hi*]-Indole:**

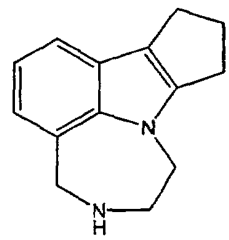

To a suspension of 3,4,9,10-tetrahydro-8*H*-cyclopenta[*b*][1,4]diazepino[6,7,1-*hi*]indol-2(1*H*)-one (67 mg, 0.30 mmol) in $Et_2O$ (7 mL), LAH power (28 mg, 0.74 mmol) was added slowly at rt under Ar. After 16 h, additional $Et_2O$ (5 mL) was added followed by dropwise addition of a sat'd $Na_2SO_4$ solution (0.1 mL). $H_2$ gas evolved upon addition. Additional $Et_2O$ (8 mL) was added, dried over $Na_2SO_4$, filtered and concentrated *in vacuo* to give 44 mg (70%) of the title compound as a yellow syrup.
$^1$H NMR ($CDCl_3$) $\delta$ 7.30 (d, J=7.7 Hz, 1H), 7.0 (t, J=7.5 Hz, 1H), 6.8 (d, J=7.1 Hz, 1H), 5.29 (s, 2H), 3.99 (t, J=5.1 Hz, 2H), 3.33 (t, J=5.0 Hz, 2H), 2.8-2.9 (m, 4H), 2.4-2.2.6 (m, 2H).

**Example 22**

**Preparation of 1,2,3,4,8,9,10,10a-Octahydro-7b*H*-Cyclopenta[*b*][1,4]-Diazepino[6,7,1-*hi*]Indole**

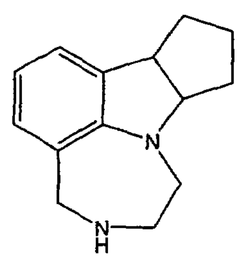

To a solution of 3,4,9,10-tetrahydro-8*H*-cyclopenta[*b*][1,4]diazepino [6,7,1-hi]indole (61 mg, 0.29 mmol) in TFA (2 mL) being cooled in an ice-bath, $BH_3$.THF (0.7 mL, 0.7 mmol, 1 M THF) was added slowly under Ar. After 4 h, the reaction mixture was concentrated *in vacuo* then $CHCl_3$ (3 mL) and 1 N HCl (3 mL) were added. The mixture was stirred for 1 h before separating the two layers. The aqueous layer was basified to pH 13-14 with 5 N NaOH then extracted with $CHCl_3$ (3 x 3 mL). The combined organic layers was dried over $Na_2SO_4$, filtered and concentrated *in vacuo* to give 36 mg (58%) of the title compound as a thin white film.
$^1$H NMR (DMSO-$d_6$) $\delta$ 6.86 (d, J=7.2 Hz, 1H), 6.74 (d, J=7.4 Hz, 1H), 6.53 (t, J=7.3 Hz, 1H), 3.88 (m, 1H), 3.81, 3.47 (ABq, $J_{AB}$=15.1 Hz, 2H), 3.73 (m, 1H), 3.08 (m, 2H), 2.55-2.80 (m, 2H), 2.15 (bs, 1H), 1.3-2.0 (m, 6H).

**Example 23**

**1,2,3,4,9,10-Hexahydro-8H-cyclopenta[b][1,4]diazepino[6,7,1-hi]indole**

To a suspension of benzodiazapine (92.0 g, 0.62 mole) and $K_2CO_3$ (90.2 g, 0.65 mole, -325 mesh) in $CH_3CN$ (1000 ml) was added acetic anhydride (63.4 g, 58.6 ml, 0.62 mole) in 0.5 h (reaction mixture was cooled in ice water to maintain the reaction mixture temperature between 15-22 °C). The suspension was stirred for 0.5 h at room temperature (check TLC after 30min, eluent ethyl acetate:MeOH 9:1). The reaction mixture was evaporated and to the residue water (400 ml) was added. The suspension was cooled in ice, filtered, washed with cold water (50 ml x 2).

Dried under vacuum to give 109.7 g of 4-acetylbenzodiazepine as a white solid, yield 93%).

**Preparation of Hydrazine (4-acetyl-2,3,4,5-tetrahydro-1H-1,4-benzodiazepin-1-ylamine**

4-Acetylbenzodiazepine (28.5 g, 50.15 m) and allyl N-[(mesitylsulfonyl)-oxy]carbamate (53.8 g, 0.18 m) were combined in toluene (270 mL) and heated under reflux for 2.5 h. The reaction was quenched with 0.5 N NaOH. The precipitate that formed was removed by filtration and the aqueous filtrate was extracted with ethyl acetate (3X). The combined organic layers were washed with saturated sodium chloride solution, dried ($MgSO_4$), and evaporated to give a residue which was dissolved in methylene chloride (490 mL) and treated with diethylamine (51 mL, 5 eq.) and tetrakis triphenylphosphinepalladium (115 mg, 0.001 eq). After stirring for 2 h at room temperature, the volatiles were removed under reduced pressure and the residue was purified by chromatography on silica gel eluting with 10-20% methanol in ethyl acetate to give 4-acetyl-2,3,4,5-tetrahydro-1H-1,4-benzodiazepin-1-ylamine (13.5 g, 44%) as a white solid.

**Preparation of Hydrazone**

**4-Acetyl-N-cyclopentylidene-2,3,4,5-tetrahydro-1H-1,4-benzodiazepin-1-amine**

4-Acetyl-2,3,4,5-tetrahydro-1H-1,4-benzodiazepine (30 g, 0.158 m) was dissolved in glacial acetic acid (287 mL). *Tert*-butylnitrite (20.6 mL) was added and the reaction mixture was stirred at room temperature. Meanwhile, a 2L round-bottomed flask was equipped with an mechanical overhead stirrer, nitrogen inlet tube, and a dropping funnel. Powdered zinc (32.8 g) was suspended in water (88 mL) with vigorous stirring. When a TLC (silica gel, 5%methanol in methylene chloride) indicated that all of the benzodiazepine had been converted to the nitroso compound, the zinc-water mixture was cooled in an ice bath and the nitroso solution was added dropwise over 1.5 h with vigorous stirring. When all of the nitroso solution had been added, the ice bath was removed and stirring was continue for another 30 minutes. Cyclopentanone (41.8 mL) was added to the reaction vessel containing the hydrazine (generated *in situ*) which was then placed in a preheated oil bath and heated at 75-85°C (internal temperature.) Heating was continued until there was no more hydrazine as indicated by TLC on silica gel eluting with 10% methanol in ethyl acetate (2 h). After cooling to room temperature, the reaction mixture was diluted with methylene chloride (600 mL) and stirred at room temperature. The insoluble material was separated by filtration and washed with methylene chloride (3x 100 mL). The layers were combined and evaporated to give crude hydrazone, 64 g.

**3-Acetyl-1,2,3,4,10-hexahydro-8H-cyclopenta[b][1,4]diazepino[6,7,1-hi]indole**

The crude hydrazone (64 g) was dissolved in 1-propanol (385 mL). *p*-Toluene-sulfonic acid hydrate (32 g) was added and the reaction vessel was placed in a preheated oil bath. The reaction mixture was heated under reflux for about 2.5 h (TLC analysis showed no hydrazone remaining). The heating bath was removed and the reaction mixture was stirred for an additional 15 minutes at room temperature. The reaction mixture was diluted with ethyl acetate (1200 mL) and cooled in an ice bath. 2.5 N NaOH was added with cooling and stirring until the aqueous phase remained basic (indicator paper, pH 12). The phases were separated and the organic phase was extracted with saturated sodium bicarbonate (2 x 300 mL). The aqueous phase was back extracted with one portion of ethyl acetate. The organic phases were combined, dried ($MgSO_4$), filtered and evaporated to give a dark brown residue. The residue was purified by column chromatography on silica gel eluting with 0.5-2% methanol in methylene chloride to give, 22.46 g (56%) of 3-acetyl-1,2,3,4,10-hexahydro-8H-cyclopenta[b][1,4]diazepino[6,7,1-hi]indole, as a viscous oil.

3-Acetyl-1,2,3,4,10-hexahydro-8H-cyclopenta[b][1,4]diazepino[6,7,1-hi]indole (2.34g , 9.20 mmol) was dissolved in MeOH . The solution was diluted with 2.5N NaOH and solid NaOH pellets were added. The reaction mixture was placed in an oil bath at 95 °C for 6 h. then cooled to room temperature and stirred overnight. The volatiles were evaporated under reduced pressure and the residue was partitioned between water and ethyl acetate. The ethyl acetate was separated and evaporated and the residue was dissolved in methylene chloride and purified by chromatography on silica gel eluting with 2-5% methanol in methylene chloride to give the product of Example 23 as a light grey solid 1.56g (80%) , mp: 66-68 °C.

| Anal. Calcd. for $C_{14}H_{16}N_2$ | | | |
|---|---|---|---|
| Theory | %C, 79.21; | %H, 7.60; | %N, 13.20. |
| Found | %C, 78.81; | %H, 7.5 ; | %N, 13.10 |

**Example 24**

**1,2,3,4,8,9,10,10a-Octahydro-7bH-cyclopenta[b][1,4]diazepino[6,7,1-hi]indole**

**Method A.**

The compound of Example 23 (1.56g, 7.35 mmol) was dissolved in trifluoroacetic acid ( 53 mL) and cooled in an ice/water bath. 1.5 M $BH_3$ in THF (34 mL) was added dropwise slowly and stirred for an additional 15min. After the addition was complete, water was added slowly to quench the reaction. This was followed by 2.5 N NaOH and 50% NaOH until the reaction mixture was basic (yellow color disappeared). After extraction with ethyl acetate (3X), the organic phases were combined and concentrated under reduced pressure to give a residue which was purified by chromatography on silica gel eluting with 3-10% MeOH in methylene chloride to give 600mg (38%) of the product as a yellow solid: mp 64-68°C.

| Anal. Calcd for $C_{14}H_{18}N_2 \cdot 0.2\ H_2O$ | | | |
|---|---|---|---|
| Calcd | %C, 77.17; | %H, 8.51; | %N, 12.86. |
| Found | %C, 77.13; | %H, 8.18; | %N, 12.61. |

**Method B.**

**4-Acetyl-2,3,4,5-tetrahydro-1H-1,4-benzodiazepine-1-carboxamide**

To a solution of 4-acetylbenzodiazepine (20.0g, 0.105 mole) in acetonitrile (100 ml) at room temperature was added sodium cyanate (27.4g, 0.42 mole). The resulting suspension was heated to 50°C and then trifluoroacetic acid (24.0 g, 0.21 mole) was added slowly dropwise over 30 min. Heating was continued at 50°C for 1h. The reaction mixture was cooled to room temperature and the solvent was evaporated to give a white residue. Water (160 ml) was added to the residue and the mixture was cooled in ice for 1h. The solids were collected by filtration and washed with cold water. After drying under house vacuum 23.6 g (96%) of product was obtained as a white solid.
NMR (300 MHz, DMSO): δ 7.2-7.5 (4H, m), 5.76 (2H, br.s), 4.46 (2H, br.s), 3.61 (2H, br.s), 2.0 (3H, two singlets).
GC-MS: 98%.
HPLC purity (area %): 97.4%
LC-MS: 96.7%.

**4-Acetyl-N-cyclopentylidene-2,3,4,5-tetrahydro-1H-1,4-benzodiazepin-1-amine**

To a mixture of ethanol (95 ml) and water (95 ml) was added sodium hydroxide pellets (11.5g, 0.288 mole). The mixture was cooled in an ice bath and then 4-acetyl-2,3,4,5-tetrahydro-1H-1,4-benzodiazepine-1-carboxamide (19.2g, 0.082 mole) was added. To the milky suspension at 4°C was added sodium hypochlorite (94.0 ml, 0.164 mole, 10-13%) over 10 min. The reaction mixture was warmed to ambient temperature. A clear solution was observed. After 2h, the reaction mixture was cooled in an ice bath and acetic acid (82.0 ml) was added. To this solution cyclopentanone (8.3g, 0.988 mole) was added. After 1.5h at room temperature the reaction mixture was cooled in an ice bath and neutralized with 10 N sodium hydroxide. The aqueous phase was extracted with ethyl acetate (125 ml, 50 ml x 2) and the combined organic layers were washed with brine (200 ml), dried ($Na_2SO_4$) and evaporated to give a syrup, 17.8g (yield 80%) of the hydrazone product. The crude hydrazone was used without purification in the Fischer-Indole cyclization.

LC-MS:93%.

GC-MS: 94%.

**3-Acetyl-1,2,3,4,10-hexahydro-8H-cyclopenta[b][1,4]diazepino[6,7,1-hi]indole**

To a gummy amber colored hydrazone (0.78 g of 94% pure by GC) was added a solution of 4% aq.$H_2SO_4$ (5 ml $H_2O$ + 0.2 ml Conc.$H_2SO_4$). The clear reaction mixture solution was heated to reflux for 1h. After cooling the reaction mixture, it was extracted with ethyl acetate. The ethyl acetate layer was washed with 5% $NaHCO_3$ solution and saturated sodium chloride solution and dried ($MgSO_4$). Filtration and evaporation of the volatiles from the filtrate gave 0.48 g of 3-acetyl-1,2,3,4,10-hexahydro-8H-cyclopenta[b][1,4]diazepino[6,7,1-hi]indole

as an off-white solid (66%).
NMR (300 MHz, DMSO): δ 7.22 (1H, m), 6.8 (2H, m), 4.85 (2H, two singlets), 4.18 (2H, m), 3.98 (2H, m) 2.7 (4H, m), 2.46 (2H, m), 2.04 (3H, two singlets).

GC-MS: 98%.

To 5%Pd/C (3.5g) was added ethanol (360 ml), 3-acetyl-1,2,3,4,10-hexahydro-8H-cyclopenta[b][1,4]diazepino[6,7,1-hi]indole (35.8g, 0.141 mole) and conc.HCl (13.9 ml, 0.141 mole). The suspension was hydrogenated in a Parr apparatus at 35 psi. for 4h. The reaction mixture was filtered through Celite, washed with ethanol (100 ml x 2) and evaporated to give 46.4 g of crude 3-acetyl-1,2,3,4,8,9,10, 10a-octahydro-7bH-cyclopenta[b][1,4]diazepino [6,7,1-hi]indole hydrochloride salt, which was used as such in the next step.

To a flask fitted with a reflux condenser, was added KOH (79.4g), MeOH (136 ml) and water (36 ml). When KOH was dissolving the reaction mixture exothermed to reflux, then it became a clear solution (Claisen alkali). The above solution (warm or hot) was added to the fused indoline (46.4g) and the resulting suspension was refluxed for 15 h. On cooling the reaction mixture to room temperature 1,2,3,4,8,9,10,10a-octahydro-7bH-cyclopenta[b][1,4]diazepino[6,7,1-hi]indole crystallized out of the reaction mixture. The solids were collected by filtration and washed with water (50 ml x 4). The wet product (28.8 g) was slurried in water (300 ml) at room temperature for 30 min, filtered, washed with water (30 ml x 6) and dried under house vacuum at 40 °C for 6 h to give 26.72g (88.6% over two steps: reduction and deprotection) of the product of Example 24.
HPLC: (97.89% area)
NMR (300 MHz, DMSO): δ 6.86 (1H, d, J=7.2Hz), 6.74 (1H, d, J=7.3Hz), 6.53 (2H, t, J=7.3Hz), 3.9 (1H, br.t), 3.81 (1H, d, J=15Hz), 3.48 (1H, d, J=15Hz), 3.04-3.15 (2H, m), 2.2 (1H, br.s), 1.85-2.0 (1H, m), 1.73-1.84 (1H, m), 1.34-1.72 (4H, m). GC-MS: 99.9%.

## Example 25

### 3-Acetyl-1,2,3,4,8,9,10,10a-octahydro-7bH-cyclopenta[b][1,4]diazepino[6,7,1-hi]-indole

3-Acetyl-1,2,3,4,10-hexahydro-8H-cyclopenta[b][1,4]diazepino[6,7,1-hi]indole (2.07g, 8.12 mmol) was dissolved in EtOH and hydrogenated over 10% Pd on carbon (0.25g) in a Parr shaker at 55 lbs of hydrogen pressure. After 5 hrs, the reaction mixture was filtered through Celite to remove the catalyst and the filtrate was concentrated under reduced pressure to give a residue which was purified by chromatography on silica gel eluting with 0.3-2% MeOH in methylene chloride to give 1.84g (87%) of 3-acetyl-1,2,3,4,8,9,10,10a-octahydro-7bH-cyclopenta[b][1,4]-diazepino [6,7,1-hi]indole as a white solid, mp:111-113°C.

| Anal. Calcd for $C_{16}H_{20}N_2O$ + .20 $H_2O$ | | | |
|---|---|---|---|
| Theory | %C, 73.93; | %H, 7.91; | %N, 10.78. |
| Found | %C, 74.05; | %H, 7.91; | %N, 10.79. |

3-Acetyl-1,2,3,4,8,9,10,10a-octahydro-7bH-cyclopenta[*b*][1,4]diazepino[6,7,1-hi]indole was dissolved in conc. HCl and heated with stirring in an oil bath (110°C) for 12 hr. After cooling, the reaction mixture was made basic with 2.5N NaOH and 50% NaOH, extracted into methylene chloride, dried ($MgSO_4$), filtered and concentrated to give a residue. The residue was purified by chromatography on silica gel eluting with 1-20% MeOH in methylene chloride to give the product of Example 24 as a yellow solid, mp: 76-79 °C.

## Example 26

The compound of Example 24 was chromatographed on a Chiralal column eluting with MeOH containing 0.1 % diethylamine.

Peak one was obtained as a yellow solid, mp: 51-54°C and identified (7bS,10aS)-1,2,3,4,8,9,10,10a-octahydro-7bH-cyclopenta[b][1,4]diazepino[6,7,1-hi]-indole.

| Anal. Calcd for $C_{14}H_{18}N_2$ +0.20 $H_2O$ | | | |
|---|---|---|---|
| Theory | %C, 78.46; | %H, 8.47; | %N, 13.07. |
| Found | %C, 77.09; | %H, 8.50; | %N, 12.72. |

Peak two was obtained as a yellow solid, mp: 43-46°C. This peak was identified as a mixture of 92.3% (7bR, 10aR)-1,2,3,4,8,9,10,10a-octahydro-7bH-cyclopenta-[b][1,4]diazepino[6,7,1-hi]indole and 7.7% of identified (7bS, 10aS)-1,2,3,4,8,9,10,10a-octahydro-7bH-cyclopenta[b][1,4]diazepino[6,7,1-hi]indole.

**Example 27**

3-Acetyl-1,2,3,4,8,9,10,10a-octahydro-7bH-cyclopenta[b][1,4]diazepino[6,7,1-hi]indole (Example No. 25) was chromatographed on a Chiralcel AD chiral column (20 x 250 mm) eluting with 100% MeOH at room temperature, detection method: UV/VIS at 254 nm..

A. Peak one was obtained as a colorless viscous oil, identified as (7bS,10aS)-3-acetyl-1,2,3,4,8,9,10,10a-Octahydro-7bH-cyclopenta[b][1,4]diazepino[6,7,1-hi]indole.

| Anal. Calcd. for $C_{16}H_{20}N_2O$ + .60 $H_2O$ | | | |
|---|---|---|---|
| Theory | %C, 71.93; | %H, 8.00; | %N, 10.49. |
| Found | %C, 72.06; | %H, 7.79; | %N, 10.73. |

O.R.:[alpha]25/D=+118(19.2mg/mL) MeOH)

(7bS,10aS)-3-Acetyl-1,2,3,4,8,9,10,10a-octahydro-7bH-cyclopenta[b][1,4]-diazepino[6,7,1-hi]indole was treated with solid NaOH in MeOH under reflux to give (7bS,10aS)-1,2,3,4,8,9,10,10a-octahydro-7bH-cyclopenta-[b][1,4]diazepino-[6,7,1-hi]-indole mp: 54-56°C.

O.R.: [alpha]25/D=+134.18 (10.359mg/mL, MeOH)

B. Peak two was obtained as a colorless viscous oil, identified as (7bR,10aR)-3-acetyl-1,2,3,4,8,9,10,10a-octahydro-7bH-cyclopenta[b][1,4]diazepino[6,7,1-hi]indole.

| Anal. Calcd. for $C_{16}H_{20}N_2O$ + 80 $H_2O$ | | | |
|---|---|---|---|
| Theory | %C, 70.98; | %H, 8.04; | %N, 10.35 |
| Found | %C, 70.94; | %H, 7.73; | %N, 10.22 |

O.R.: [alpha]25/D=-132 (10.2mg/mLMeOH)

(7bR,10aR)-3-Acetyl-1,2,3,4,8,9,10,10a-octahydro-7bH-cyclopenta-[b][1,4]-diazepino-[6,7,1-hi]indole was treated with solid NaOH in MeOH under reflux to give (7bR, 10aR)-1,2,3,4,8,9,10,10a-octahydro-7bH-cyclopenta-[b][1,4]diazepino[6,7,1-hi]-indole mp: 57-59°C

| Anal Calcd for $C_{14}H_{18}N_2$ | | | |
|---|---|---|---|
| Theory | %C,77.81; | %H,8.49; | %N, 12.96. |
| Found | %C,78.01; | %H,8.64; | %N, 12.90. |

**Example 28 (chiral salt resolution of Example No. 24)**

**(7bR,10aR)-1,2,3,4,8,9,10,10a-octahydro-7bH-cyclopenta-[b][1,4]diazepino[6,7,1-hi]indole**

The compound of Example 24 (10.0 g, 46.7 mmol) was dissolved in isopropanol (500 mL) at 65-70 °C under nitrogen and dibenzoyl-L-itartaric acid (4.18 g, 11.7 nunol) was added all at one time. The resultant solids were slurried at 70-75°C for two h, cooled to room temperature and stored at 10°C for 12 h. The solids were filtered and washed twice with isopropanol (15 mL). The solids were reslurried in hot (80°C) isopropanol (400 mL) for 1.5 h, cooled to room temperature and stored at 10 °C for 12 h. The solids were filtered and washed twice with isopropanol (15 mL) and air dried to give 7.3g (79.9%) of the dibenzoyl-L-tartaric acid salt of the title compound as a white solid, mp: 163-5 °C.

| Anal. Calcd for $C_{14}H_{18}N_2 \cdot 0.5\ C_{18}H_{14}O_8 \cdot 0.4\ C_3H_8O$ | | | |
|---|---|---|---|
| Calcd | %C, 69.60; | %H, 6.75; | %N, 6.70. |
| Found | %C, 69.80; | %H, 6.73; | %N, 6.58. |

O.R.: [alpha]25/D=-138.4 (1 mg/mL, MeOH)

The above tartrate salt (9.5 g, 12.1 mmol) was slurried in ethyl acetate (950 mL) and 1 N hydrochloric acid was added (25.0 mL, 25.0 mmol). The slurry was concentrated by atmospheric distillation (72-77 °C) to a volume of 275 mL, cooled to room temperature and stored overnight at 10°C. The solids were filtered and washed twice with ethyl acetate (20 mL) and air dried to give 5.7 g (92.8 %) of the hydrochloride salt of the title compound as a white solid, mp 246-249°C decomposed.

The HCl salt (5.6 g) was dissolved in ethanol (100 mL) at reflux. Upon cooling to room temperature, needle-like crystals formed. The mixture was stored overnight at 10°C. The solids were filtered, washed twice with ethanol (10 mL) and vacuum dried (57°C /0.1mm) to give 3.8 g (67.8 %) of white solid, mp 252-253°C decomposed.

| Anal. Calcd for $C_{14}H_{18}N_2 \cdot HCl$ | | | |
|---|---|---|---|
| Calcd | %C, 67.05; | %H, 7.64; | %N, 11.17. |
| Found | %C, 66.74; | %H, 7.54; | %N, 11.09. |

**Example 29**

**1,2,3,4,8,9,10,10a-Octahydro-7b*H*-Cyclopenta[*b*][1,4]Diazepino[6,7,1-*hi*]Indole**

**A. 1,2,3,4-Tetrahydrocyclopenta[*b*]Indole**

Concentrated sulfuric acid (~18 M, 35 mL) was added dropwise to a mixture of phenyl hydrazine (510 mmol, 50 mL) and cyclopentanone (45 mL, 510 mmol) in water (250 mL). The resulting mixture was heated to reflux for 30 min and then allowed to cool to room temperature. The liquid was decanted from the reaction mixture leaving a red, gummy solid. Hexanes (500-600 mL) was added to the flask and the mixture was heated to reflux. The yellow hexane solution was decanted hot from the mixture and placed in the freezer (crystallization begins immediately). More hexanes is added to the flask and the procedure repeated two more times using a total volume of 1500 mL of hexanes. After 1 h in the freezer, the solid was collected from the flasks and dried providing the known indole (410 mmol, 65 g, 80%).
Anal. Calcd. for $C_{11}H_{11}N$: C, 84.04; H, 7.05; N, 8.91
Found: C, 83.92; H, 7.12; N, 8.85

**B. 1,2,3,3a,4,8b-Hexahydrocyclopenta[*b*]Indole**

A mixture of 1,2,3,4-tetrahydrocyclopenta[*b*]indole (11 mmol, 1.8 g), 5% Pd/C (0.5 g), ;and concentrated hydrochloric acid (1.2 mL) was hydrogenated at 45 psi on a Parr shaker. After 3 h, the mixture was removed from the shaker and filtered through Celite. The solid bed was washed with methanol. The filtrate was concentrated. The crude oil was dissolved in 1 N HCl and washed with ether. The aqueous phase was treated with 2.5 N NaOH to pH >10 and then extracted with chloroform. The combined chloroform extracts were dried over $MgSO_4$, filtered and concentrated to give the crude indoline. The material was purified by flash column chromatography through silica gel (Biotage, elution with 10% ethyl acetate-hexanes) to give the known indoline (7.6 mmol, 1.2 g, 69%) as a clear oil.
Anal. Calcd. for $C_{11}H_{13}N$: C, 82.97; H, 8.23; N, 8.80
Found: C, 82.61; H, 8.35; N, 8.72

**C. 2-(2,3,3a,8b-Tetrahydrocyclopenta[*b*]Indol-4(1*H*)-yl)Acetamide**

To a stirred solution of 1,2,3,3a,4,8b-hexahydrocyclopenta[*b*]indole (130 mmol, 21 g) in DMF (50 mL) was added diisopropylethylamine (400 mmol, 70 mL) followed by 2-chloroacetamide (270 mmol, 25 g). The reaction mixture was heated to 100°C for 18 h. The reaction was concentrated and the diluted with ethyl acetate and water. The phases were separated and the organic phase was washed with brine, dried over $MgSO_4$, filtered and concentrated. The crude material was purified by flash column chromatography through silica gel (elution with 60% ethyl acetate-hexanes) to afford a yellow solid (90 mmol, 20 g, 69%).
Anal. Calcd. for $C_{13}H_{16}N_2O$: C, 72.19; H, 7.46; N, 12.95.
Found: C, 72.45; H, 7.57; N, 12.64. MS ((+)APCI, m/e(%)) 217(100, $[M+H]^+$).
IR (solid ATR, $cm^{-1}$) 3450, 2930, 2870, 1680, 1480, 1150, 740.
$^1H$ NMR (DMSO-$d_6$, 400MHz) δ 7.20(s, 1H), 7.05(s, 1H), 6.90(m, 2H), 6.48(dt, J=0.73Hz, 7.3Hz, 1H), 6.18(d, J=7.8Hz, 1H), 4.22(m, 1H), 3.70, 3.58(ABq, $J_{AB}$=17.1Hz, 2H), 3.64(m, 1H), 1.90(m, 1H), 1.78(m, 1H), 1.56(m, 3H), 1.40(m, 1H).

D. 2-(2,3,3a,8b-Tetrahydrocyclopenta[*b*]Indol-4(1*H*)-yl)Acetonitrile

To a stirred solution of 1,2,3,3a,4,8b-hexahydrocyclopenta[*b*]indole (22 mmol, 3.5 g) in DMSO (44 mL) was added 60% sodium hydride (24 mmol, 0.97 g) portionwise, followed by 2-chloroacetonitrile (33 mmol, 2.1 mL). The reaction mixture stirred at ambient temperature for 18 h and then additional 60% sodium hydride (22 mmol, 0.88 g) and 2-chloroacetonitrile (24 mmol, 1.5 mL) was added. The reaction was heated to 60°C for 18 h. The reaction was concentrated and the diluted with ethyl acetate and water. The phases were separated and the organic phase was washed with brine, dried over $MgSO_4$, filtered and concentrated. The crude material was purified by flash column chromatography through silica gel (elution with 60% ethyl acetate-hexanes) to afford the desired material.
$^1$H NMR (DMSO-$d_6$, 400MHz) δ 7.25(s, 1H), 7.05(m, 1H), 6.78(m, 1H), 6.46(m, 1H), 4.30-3.70(m, 4H), 2.10-1.50 (m, 6H).
MS ((-)APCI, mle(%)) 198(40, [M]).

E. 2-(2,3,3a,8b-Tetrahydrocyclopenta[*b*]Indol-4(1*H*)-yl)Ethylamine

2-(2,3,3a,8b-Tetrahydrocyclopenta[*b*]indol-4(1*H*)-yl)acetamide (90 mmol, 20 g) was dissolved in 1 M $BH_3$•THF (200 mL) and heated to reflux for 18 h. The reaction mixture was allowed to cool to room temperature and then quenched slowly with methanol. The solution was concentrated, dissolved in methanol, and again concentrated. The resulting oil was diluted with ether and extracted twice with 1 N HCl. The aqueous phase was treated with 2.5 N NaOH to pH >10 and extracted with chloroform. The combined chloroform extracts were dried over $MgSO_4$, filtered and concentrated to provide a yellow oil.
Anal. Calcd. for $C_{13}H_{18}N_2 \cdot$ 0.55mol $H_2O$: *C*, 73.58; H, 9.07; N, 13.20.
Found: C, 73.62; H, 8.80; N, 12.83.
MS (EI, m/e(%)) 202(10, $M^+$), 172(100), 130(20).
IR (film ATR, $cm^{-1}$) 2950, 2870, 1605, 1480, 1250(br), 730.
$^1$H NMR (DMSO-$d_6$, 400MHz) δ 6.89(dd, J=0.73Hz, 7.3Hz, 2H), 6.42(dt, J=0.73Hz, 7.3Hz, 1H), 6.29(d, J=7.6Hz, 1H), 4.12(m, 1H), 3.62(dt, J=2.4Hz, 9.0Hz, 1H), 3.08(m, 2H), 2.65(m, 2H), 1.90(m, 1H), 1.75(m, 1H), 1.58(m, 3H), 1.43(m, 1H).

F. 1,2,3,4,8,9,10,10a-Octahydro-7b*H*-Cyclopenta[*b*][1,4]-Diazepino[6,7,1-*hi*]-Indole

A solution of 2-(2,3,3a,8b-tetrahydrocyclopenta[*b*]indol-4(1*H*)-yl)ethylamine (25 mmol, 5.0 g) was dissolved in ethanol (125 mL) at room temperature and trifluoroacetic acid (25 mmol, 1.9 mL) was added, followed by aqueous formaldehyde(25 mmol, 37%, 1.9 mL). The reaction was heated to reflux for one hour and an aliquot was removed, concentrated and an NMR taken, which showed the reaction complete. The reaction vessel was cooled and then concentrated *in vacuo*. The resulting oil was partitioned between $CHCl_3$ and 1N NaOH, The aqueous phase was extracted again with $CHCl_3$. The combined organics were washed with 1N NaOH, dried over $MgSO_4$, filtered and concentrated *in vacuo* to yield a brown oil (23.8 mmol). The crude product was purified by flash chromatography ($SiO_2$) eluting with 5%MeOH/$CHCl_3$ to yield a yellow waxy solid (13 mmol, 2.7 g, 52%).
Anal. Calcd. for $C_{14}H_{18}N_2$ • 0.15mol $H_2O$:C, 77.49; H, 8.50; N, 12.91.
Found: C, 77.13; H, 8.07; N, 12.77.
MS ((+)APCI, mle(%)) 215(100, $[M+H]^+$.
IR (solid ATR, $cm^{-1}$) 3210, 2910, 2860, 2810, 1590, 1460, 1430, 1340,1290, 750.
$^1$H NMR (DMSO-$d_6$, 400MHz) δ 6.85(d, J=7.3Hz, 1H), 6.72(d, J=7.1Hz, 1H), 6.52(t, J=7.3Hz, 1H), 3.89(m, 1H), 3.8, 3.48(ABq, $J_{AB}$=15.13Hz, 2H), 3.69(dt, J=2.9Hz, 1H), 3.08(m, 2H), 2.71(m, 1H), 2.62(m, 1H), 2.29(br m, 1H), 1.90(m, 1H), 1.76(m, 1H), 1.68-1.48(m, 3H), 1.40(m, 1H).

## Example 30

***rel*-(4*S*,7b*S*,10a*S*)-4-Methyl-1,2,3,4,8,9,10,10a-Octahydro-7b*H*-Cyclopenta[*b*][1,4]-Diazepino[6,7,1-*hi*]Indole**

2-(2,3,3a,8b-tetrahydrocyclopenta[*b*]indol-4(1*H*)-yl)ethylamine (4.9 mmol, 1.0 g) was dissolved in ethanol (25 mL) at room temperature and trifluoroacetic acid (4.9 mmol, 380 μL) was added, followed by acetaldehyde (4.9 mmol, 37%, 280 μL). The reaction was heated to reflux overnight. The reaction vessel was cooled and then concentrated *in vacuo*. The resulting oil was partitioned between $CHCl_3$ and 1N NaOH. The aqueous phase was extracted again with $CHCl_3$. The combined organics were washed with 1N NaOH, dried over $MgSO_4$, filtered and concentrated *in vacuo* to yield a brown oil (23.8 mmol). The crude product was purified by flash chromatography ($SiO_2$) eluting with 10%MeOH/$CHCl_3$ to give the two racemic diastereomers.

Less Polar Product:
Rf0.5 (A) 10%$Et_3N$/EtOAc
MS ((+)APCI, m/c(%)) 229(100, $[M+H]^+$).
IR (film ATR, $cm^{-1}$) 2940, 2860, 1600, 1460, 1430, 1330, 1300, 1230, 1070, 750.
$^1H$ NMR (DMSO-$d_6$, 400MHz) δ 6.88(d, J=7.3Hz, 1H), 6.81(d, J=7.8Hz, 1H), 6.59(t, J=7.4Hz, 1H), 3.87(m, 1H), 3.71 (m, 1H), 3.45(q, J=6.7Hz, 1H), 3.06(m, 2H), 2.80-2.67(m, 2H), 2.05(br m, 1H), 1.90(m, 1H), 1.75(m, 1H), 1.64(m, 1H), 1.52(m, 2H), 1.37 (m, 1H), 1.37(d, J=6.8Hz, 3H).

**Example 31**

***rel*-(4*R*,7b*S*,10a*S*)-4-Methyl-1,2,3,4,8,9,10,10a-Octahydro-7b*H*-Cyclopenta[*b*][1,4]-Diazepino[6,7,1-*hi*]Indole**

Prepared in Example 30 isolating the more polar product.
Rf 0.39 (B) 10%$Et_3N$/EtOAc
MS ((+)APCI, m/e(%)) 229(100, $[M+H]^+$).
IR (film ATR, $cm^{-1}$) 2950, 2930, 2860, 1600, 1460, 1430, 1320, 1230, 1050, 750.
$^1H$ NMR (DMSO-$d_6$,400MHz) δ6.81(d, J=7.1Hz, 1H), 6.71(d, J=7.6Hz, 1H), 6.52(t, J=7.3Hz, 1H), 4.0(q, J=6.9Hz, 1H), 3.82(dd, J=4.7Hz, 8.6Hz, 1H), 3.68(dt, J=2.7Hz, 8.9Hz, 1H), 3.05(m, 2H), 2.82-2.70(m, 2H), 2.3(br m, 1H), 1.88 (m, 1H), 1.78(m, 1H), 1.64-1.48(m, 3H), 1.40(m, 1H), 1.17(d, J=7.1Hz, 3H).

**Example 32**

**(2*S*)-(*rel*-7b*R*,10a*R*)-2-Methyl-1,2,3,4,8,9,10,10a-Octahydro-7b*H*-Cyclopenta[*b*]-[1,4]Diazepino[6,7,1-*hi*]Indole**

**A. 1,2,3,4-Tetrahydrocyclopenta[*b*]Indole-5-Carboxylic Acid**

To a stirred solution of 2-hydrazinobenzoic acid hydrochloride (53 mmol, 10.0 g) and cyclopentanone (58 mmol, 4.9 g) in 1,4-dioxane (100 mL) was added dropwise concentrated $H_2SO_4$ (~18M, 63 mmol, 3.5 mL). The resulting solution was heated to reflux for 2 hours. $^1H$ NMR analysis of a crude aliquot indicated complete reaction. The reaction was allowed to cool to room temperature and then concentrated to dryness to give a red solid which was used without further purification.
$^1H$ NMR (DMSO-$d_6$, 300MHz) δ 10.8(s, 1H), 7.6-7.0(m, 3H), 2.8(m, 4H), 2.4(m, 2H).

**B. Ethyl (2*S*)-2-[(1,2,3,4-Tetrahydrocyclopenta[*b*]Indol-5-ylcarbonyl)Amino]-Propanoate**

To a stirred, cooled (0°C) solution of crude 1,2,3,4-tetrahydrocyclopenta[*b*]-indole-5-carboxylic acid (60 mmol, 12 g), L-alanine ethyl ester (72 mmol, 11 g), 1-[3-(dimethylamino)propyl]-3-ethylcarbodiimide hydrochloride (EDC) (72 mmol, 14 g), 1-hydroxybenzotriazole (HOBT) (72 mmol, 10 g) in $CH_2Cl_2$ (100 mL) was slowly added diisopropylethylamine (360 mmol, 46 g). The reaction mixture was stirred overnight while warming to room temperature. The reaction was concentrated *in vacuo* and the resulting oil was partitioned between water and ethyl acetate. The organic layer was washed with saturated aqueous $NH_4Cl$, $NaHCO_3$, and brine, dried over $MgSO_4$, filtered and concentrated to a brown oil. The crude material was purified by chromatography through silica gel (Biotage) eluting with 15% ethyl acetate-hexanes to afford a yellow oil (8.4 mmol, 2.5 g, 14%).
$^1H$ NMR (DMSO-$d_6$, 300MHz) δ 10.9(s, 1H), 8.74(d, 1H), 7.62(d, 1H), 7.48(d, 1H), 6.98(t, 1H), 4.47(t, 1H), 4.08(m, 2H), 2.75(m, 4H), 2.40(m, 2H), 1.42(d, 3H), 1.15(t, 3H).

**C. Ethyl (2*S*)-2-[(1,2,3,3a,4,8b-Hexahydrocyclopenta[*b*]Indol-5-ylcarbonyl)-Amino]Propanoate**

A solution of ethyl (2*S*)-2-[(1,2,3,4-tetrahydrocyclopenta[*b*]indol-5-ylcarbonyl)amino]propanoate (8.4 mmol, 2.5 g) in ethanol (40 mL) was added to a mixture of 5% palladium on carbon (2 g) in ethanol (20mL). Concentrated hydrochloric acid (10 mL) was added and the resulting mixture was hydrogenated at 45 psi for 4 hours. The reaction mixture was filtered through Celite. The filter bed was washed well with ethanol and the combined filtrates were concentrated. The resulting oil was partitioned between 1 N NaOH and ethyl acetate. The orgainc phase was dried over $MgSO_4$, filtered and concentrated to yield an oil (1.9 g, 6.1 mmol, 73%).
$^1H$ NMR (DMSO-$d_6$, 300MHz) δ 8.7(m, 1H), 7.63(m, 1H), 7.25(m, 1H), 6.85(m, 2H), 4.40(m, 2H), 4.10(m, 2H), 3.76 (m, 1H), 1.90(m, 2H), 1.68(m, 3H), 1.38(d, 3H), 1.31(m, 1H), 1.16(t, 3H).

**D. (2*S*)-2-[(1,2,3,3a,4,8b-Hexahydrocyclopenta[*b*]Indol-5-ylcarbonyl)Amino]-Propanoic Acid**

A 1 M aqueous lithium hydroxide solution (13 mmol, 13 mL) was added to a solution of ethyl (2*S*)-2-[(1,2,3,3a, 4,8b-hexahydrocyclopenta[*b*]indol-5-ylcarbonyl)amino]propanoate (6.1 mmol, 1.9 g) in THF (50 mL). The reaction mixture was stirred at room temperature for 18 h. The reaction was concentrated *in vacuo* and diluted with 0.1 N HCl and ethyl acetate. The phases were separated and the organic phase was washed with water, dried over $MgSO_4$, filtered, and concentrated to give a yellow oil (1.6 g, 6.1 mmol, quantitative).
$^1$H NMR (DMSO-$d_6$, 300MHz) δ 12.4(s, 1H), 8.21(d, 1H), 7.43(m, 1H), 7.01(d, 1H), 6.70(br s, 1H), 6.40(t, 1H), 4.35 (m, 2H), 3.63(t, 1H), 1.88(m, 2H), 1.62(m, 4H), 1.30(d, 3H).

**E. (2*S*)-2-Methyl-2,3,8,9,10,10a-Hexahydro-7b*H*-Cyclopenta[*b*][1,4]Diazepino[6,7,1-*hi*]Indole-1,4-Dione**

A solution of (2*S*)-2-[(1,2,3,3a,4,8b-hexahydrocyclopenta[*b*]indol-5-yl-carbonyl)amino]propanoic acid (6.1 mmol, 1.6 g) was dissolved in acetic acid (50 mL) and heated to reflux for 18 h. The reaction was allowed to cool to room temperature and was concentrated to dryness. The crude material was purified by flash column chromatography (silica gel; 1:1 ethyl acetate-hexanes) to provide two diastereomers: less polar product (0.88 mmol, 0.23 g, 14%) and more polar product (0.18 mmol, 45 mg, 3%). The mixed fractions were also collected to provide another 3.9 mmol (1.0 g, 64%) of material.
Less Polar Product (A)
$^1$H NMR (DMSO-$d_6$, 300MHz) δ 8.2(d, 1H), 7.61(m, 1H), 7.46(dd, 1H), 7.16(t, 1H), 4.86(dt, 1H), 3.87(m, 2H), 1.92(m, 2H), 1.75(m, 1H), 1.58(m, 2H), 1.26(d, 3H), 1.06(m, 1H).

**F. (2*S*)-(*rel*-7b*R*,10a*R*)-2-Methyl-1,2,3,4,8,9,10,10a-Octahydro-7b*H*-Cyclopenta[*b*][1,4]Diazepino[6,7,1-*hi*]Indole**

A mixture of diastereomers of (2*S*)-2-methyl-2,3,8,9,10,10a-hexahydro-7b*H*-cyclopenta[*b*][1,4]diazepino [6,7,1-*hi*]indole-1,4-dione (3.9 mmol, 1.0 g) was suspended in 1 M $BH_3$•THF (15 mL) and heated to reflux for 18 h. After cooling to room temperature, the solution was quenched with methanol and concentrated. The resulting solid was suspended in 1 N NaOH and stirred at room temperature for 1 h. The aqueous phase was then extracted with chloroform and the combined extracts were dried over $MgSO_4$, filtered, and concentrated to give a yellow solid (3.5 mmol, 0.80 g, 90%). Flash chromatography through silica gel (gradient elution 5%-10% methanol-chloroform) afforded the two diastereomers. The less polar product was arbitrarily assigned the *R,R* configuration and the more polar product the *S*,*S* configuration.
Anal. Calcd. for $C_{15}H_{20}N_2$ • 1.5mol $H_2O$: C, 70.56; H, 9.08; N, 10.97.
Found: C, 70.24; H, 9.58; N, 10.81.
MS ((+))APCI, m/e (%)) 229(100, $[M+H]^+$).
IR (solid ATR, $cm^{-1}$) 2960, 2880, 2310, 1460, 1440, 1210, 1160, 1120, 1090, 1070,
$^1$H NMR (DMSO-$d_6$, 400MHz) δ 7.32(t, J=7.56Hz, 1H), 7.22(d, J=7.3Hz, 1H), 7.12(d, J=7.3Hz, 1H), 4.35(m, 1H), 4.07 (m, 2H), 3.82(d, J=16.84Hz, 1H), 3.62(m, 1H), 3.14(dd, J=8.54Hz, 10.74Hz, 1H), 3.0(m, 1H), 2.04-1.69(m, 4H), 1.51 (m, 2H), 1.22(d, J=6.6Hz, 4H).
$[\alpha]_D$ +99 (c. 0.11, DMSO).

**<u>Example 33</u>**

**<u>(2*S*)-(*rel*-7b*S*,10a*S*)-2-Methyl-1,2,3,4,8,9,10,10a-Octahydro-7b*H*-Cyclopenta[*b*]-[1,4]Diazepino[6,7,1-*hi*]Indole</u>**

Following the procedure of Example 32F, the more polar material provided the product which was assigned the *S*,*S* configuration.
Anal. Calcd. for $C_{15}H_{20}N_2$ • 1.1mol $H_2O$: C, 72.60; H, 9.02; N, 11.29.
Found: C, 72.63; H, 8.80; N, 10.95.
MS ((+))APCI, m/e (%)) 229(100, $[M+H]^+$).
$^1$H NMR (DMSO-$d_6$, 400MHz) δ 6.86(d, J=7.3Hz, 1H), 6.73(d, J=7.3Hz, 1H), 6.52(t, J=7.4Hz, 1H), 3.95(m, 2H), 3.82 (d, J=15.86Hz, 1H), 3.61(m, 1H), 3.41(dd, J=3.17Hz, 13.4Hz, 2H), 3.21(m, 1H), 2.83(dd, J=3.9Hz, 13.2Hz, 1H), 1.94 (m, 1H), 1.77(m, 1H), 1.55(m, 4H), 1.15(d, J=6.6Hz, 3H).
$[\alpha]_D$ +38 (c. 0.10, DMSO).

**Example 34**

**(2*R*)-(*rel*-7b*R*,10a*R*)-2-Methyl-1,2,3,4,8,9,10,10a-Octahydro-7b*H*-Cyclopenta[*b*]-[1,4]Diazepino[6,7,1-*hi*]Indole**

**A. Methyl (2*R*)-2-[(1,2,3,4-Tetrahydrocyclopenta[*b*]Indol-5-ylcarbonyl)Amino]-Propanoate**

Following the procedure of method 1B, employing D-alanine methyl ester (64 mmol, 8.9 g) afforded a yellow oil (4.9 mmol, 1.4 g, 8%).
$^1$H NMR (DMSO-$d_6$, 300MHz) δ 10.8(s, 1H), 8.78(d, 1H), 7.61(d, 1H), 7.49(d, 1H), 7.0(t, 1H), 4.5(m, 1H), 3.63(s, 3H), 2.76(m, 4H), 2.42(m, 2H), 1.42(d, 3H).

**B. Methyl (2*R*)-2-[(1,2,3,3a,4,8b-Hexahydrocyclopenta[*b*]Indol-5-ylcarbonyl)-Amino] Propanoate**

Following the procedure of method 1C, methyl (2*R*)-2-[(1,2,3,4-tetra-hydrocyclopenta[*b*]indol-5-ylcarbonyl) amino]propanoate (4.9 mmol, 1.4 g) was hydrogenated using 5% Pd/C (1.5 g) and concentrated HCl (7 mL) in methanol (25 mL) to yield an oil (2.7 mmol, 0.77 g, 55%).
$^1$H NMR (DMSO-$d_6$, 300MHz) δ 8.34(d, 1H), 7.44(d, 1H), 7.02(d, 1H), 6.70(s, 1H), 6.41(t, 1H), 4.39(m, 2H), 3.65(m, 1H), 3.60(s,3H), 1.89(m, 1H), 1.61(m, 4H), 1.35(d, 3H), 1.29(m, 1H).

**C. (2*R*)-2-[(1,2,3,3a,4,8b-Hexahydrocyclopenta[*b*]Indol-5-ylcarbonyl)Amino]-Propanoic Acid**

Following the procedure of method 1D, methyl (2*R*)-2-[(1,2,3,3a,4,8b-hexahydrocyclopenta[*b*]indol-5-ylcarbonyl)amino]propanoate (2.7 mmol, 0.77 g) was hydrolyzed to the acid using 1 M aqueous lithium hydroxide (5.9 mL) in THF (20 mL) to yield an orange oil which was used without further purification.
$^1$H NMR(DMSO-$d_6$, 300MHz) δ 12.4(br s, 1H), 8.2(d, 1H), 7.43(d, 1H), 7.01(d, 1H), 6.9(br s, 1H), 6.4(t, 1H), 4.33(m, 2H), 3.62(t, 1H), 1.89(m, 2H), 1.61(m, 4H), 1.32(d, 3H).

**D. (2*R*)-2-Methyl-2,3,8,9,10,10a-Hexahydro-7b*H*-Cyclopenta[*b*]-[1,4]Diazepino[6,7,1-*hi*]Indole-1,4-Dione**

Following the procedure of method 1E, (2*R*)-2-[(1,2,3,3a,4,8b-hexahydrocyclopenta[*b*]indol-5-ylcarbonyl) amino]propanoic acid was cyclized by refluxing in acetic acid (50 mL). Purification by flash chromatography through silica gel (elution with 5% methanol-chloroform) provided each diastereomer: less polar product (1.5 mmol, 0.39 g, 56% over 2 steps) arbitrarily assigned as the R,R configuration and more polar product (0.47 mmol, 0.11 g, 17% over 2 steps) assigned as the *S*,*S* configuration. Less Polar Product (A)
$^1$H NMR (DMSO-$d_6$, 300MHz) δ 8.2(d, 1H), 7.62(d, 1H), 7.46(d, 1H), 7.16(t, 1H), 4.87(m, 1H), 3.88(m, 2H), 1.94(m, 3H), 1.76(m, 1H), 1.59(m, 2H), 1.28(d, 3H).

**E. (2*R*)-(*rel*-7b*R*,10a*R*)-2-Methyl-1,2,3,4,8,9,10,10a-Octahydro-7b*H*-Cyclopenta[*b*][1,4]Diazepino[6,7,1-*hi*]Indole**

Following the procedure of method 1F, (2*R*)-(*rel*-7b*R*,10a*R*)-2-methyl-2,3,8,9,10,10a-hexahydro-7b*H*-cyclopenta[*b*]-[1,4]diazepino[6,7,1-*hi*]indole-1,4-dione (1.5 mmol, 0.39 g) was reduced with 1 M $BH_3$·THF (10 mL) to yield a yellow solid (0.47 mmol, 0.11 g, 31%).
Anal. Calcd. for $C_{15}H_{20}N_2$ • 0.15mol $H_2O$: C, 77.98; H, 8.86; N, 12.12. Found: C, 77.72; H, 9.03; N, 11.89.
MS ((+)ESI, m/e(%)) 457(17, $[2M+H]^+$), 307(81, $[M+H+DMSO]^+$), 229(100, $[M+H]^+$.
IR (solid ATR, $cm^{-1}$) 3240, 2950, 2870, 1590, 1460, 1350, 1290, 1270, 740.
$^1$H NMR (DMSO-$d_6$, 400MHz) δ 6.8(d, J=7.1Hz, 1H), 6.65(d, J=7.1Hz, 1H), 6.47(t, J=7.3Hz, 1H), 3.94(m, 1H), 3.80, 3.71(ABq, $J_{AB}$=16.1Hz, 2H), 3.59(m, 1H), 3.35(dd, J=3.17Hz, 12.93Hz, 1H), 3.02(m, 1H), 2.75(dd, J=4.39Hz, 12.93Hz, 1H), 2.49(m, 1H), 1.94(m, 1H), 1.76(m, 1H), 1.56(m, 4H), 1.07(d, J=6.6Hz, 3H).
$[\alpha]_D$ -82(c. 0.10, DMSO).

**Example 35**

**(2*R*)-(*rel*-7b*S*,10a*S*)-2-Methyl-1,2,3,4,8,9,10,10a-Octahydro-7b*H*-Cyclopenta[*b*]-[1,4]Diazepino[6,7,1-*hi*]Indole**

Following the procedure of Example 32F, (2*R*)-(*rel*-7b*S*,10a*S*)-2-methyl-2,3,8,9,10,10a-hexahydro-7b*H*-cyclopenta[*b*]-[1,4]diazepino[6,7,1-*hi*]indole-1,4-dione (0.47 mmol, 0.11 g) was reduced with 1 M $BH_3$•THF (8 mL) to yield the product (0.27 mmol, 61 mg, 57%). MS ((+)APCI, m/e (%)) 457(20, $[2M+H]^+$), 229(100, $[M+H]^+$).
$^1$H NMR (DMSO-$d_6$, 400MHz) δ 6.85(d, J=7.08Hz, 1H), 6.72(d, J=7.3Hz, 1H), 6.52(t, J=7.3.Hz, 1H), 3.82(dd, J=5.6Hz,

9.0Hz, 1H), 3.79, 3.51(ABq, $J_{AB}$=15.1Hz, 2H), 3.70(dt, J=2.9Hz, 9.0Hz, 1H), 3.28(m, 1H), 3.06(dd, J=2.1Hz, 12.1Hz, 1H), 2.78(m, 1H), 2.43(m, 1H), 1.92-1.30(m, 6H), 1.02(d, J=6.6Hz, 3H).

**Example 36**

**(2*R*,7b*S*,10a*S*)-1,2,3,4,8,9,10,10a-Octahydro-7b*H*-Cyclopenta[*b*][1,4]Diazepino[6,7,1-*hi*]Indol-2-ylmethanol**

**A. Methyl (2*S*)-3-Hydroxy-2-[(1,2,3,4-Tetrahydrocyclopenta[*b*]Indol-5-yl-carbonyl)Amino]Propanoate**

Following the procedure of Example 32B, employing L-serine methyl ester (64 mmol, 9.9 g) afforded a yellow solid (8.9 mmol, 2.7 g, 14%).
$^1$H NMR (DMSO-$d_6$, 300MHz) δ 10.8 (s, 1H), 8.51(d, 1H), 7.61(d, 1H), 7.51(d, 1H), 7.01 (t, 1H), 5.08(m, 1H), 4.56(q, 1H), 3.82(d, 2H), 3.62(s, 3H), 2.76(m, 4H), 2.42(m, 2H).

**B. Methyl (2*S*)-2-[(1,2,3,3a,4,8b-Hexahydrocyclopenta[*b*]Indol-5-ylcarbonyl)-Amino]-3-Hydroxypropanoate**

Following the procedure of Example 32C, methyl (2*S*)-3-hydroxy-2-[(1,2,3,4-tetrahydrocyclopenta[*b*]indol-5-ylcarbonyl)amino]propanoate(8.9 mmol, 2.7 g) was hydrogenated using 5% Pd/C (2 g) and concentrated HCl (10 mL) in methanol (25 mL) to yield the crude product (8.9 mmol, 2.7 g, quantitative).
$^1$H NMR (DMSO-$d_6$, 300MHz) δ 8.3(d, 1H), 7.86(d, 1H), 7.34(d, 1H), 7.03(t, 1H), 5.80(br s, 2H), 4.45(m, 2H), 3.80(m, 2H), 3.61(s, 3H), 2.0-1.55(m, 6H).

**B. (2*S*)-2-[(1,2,3,3a,4,8b-Hexahydrocyclopenta[*b*]Indol-5-ylcarbonyl]Amino}-3-Hydroxypropanoic Acid**

Following the procedure of Example 32D, methyl (2*S*)-2-[(1,2,3,3a,4,8b-hexahydrocyclopenta[*b*]indol-5-ylcarbonyl)amino]-3-hydroxypropanoate (8.9 mmol, 2.7 g) was hydrolyzed to the acid using 1 M aqueous lithium hydroxide (40 mL) in THF (40 mL) to yield a red oil (1.5 mmol, 430 mg, 17%).
$^1$H NMR (DMSO-$d_6$, 300 MHz) δ 7.92(d, 1H), 7.40(d, 1H), 7.03(d, 1H), 6.43(t, 1H), 4.39(m, 2H), 3.63(br m, 4H), 2.0-1.2 (m, 6H).

**C. (2*S*)-2-(Hydroxymethyl)-2,3,8,9,10,10a-Hexahydro-7b*H*-Cyclopenta[*b*]-[1,4]-Diazepino[6,7,1-*hi*]Indole-1,4-Dione**

Following the procedure of Example 32E, (2*S*)-2-[(1,2,3,3a,4,8b-hexahydrocyclopenta[*b*]indol-5-ylcarbonyl) amino]-3-hydroxypropanoic acid (1.5 mmol, 430 mg) was cyclized by refluxing in acetic acid (40 mL). Purification by flash chromatography through silica gel (elution with 5% methanol-chloroform) provided each diastereomer: less polar product (0.55 mmol, 0.15 g, 37%) arbitrarily assigned as the *R,R* configuration and more polar product (0.26 mmol, 0.070 g, 17%) assigned as the *S,S* configuration.
Less Polar Product (A)
$^1$H NMR (DMSO-$d_6$, 300 MHz) δ 8.44(d, 1H), 7.63(d, 1H), 7.48(d, 1H), 7.19(m, 1H), 4.88(m, 1H), 4.39(dd, 1H), 4.22 (t, 1H), 4.07(m, 1H), 3.91(m, 1H), 2.0-1.5(m, 6H).

**D. (2*R*)-(*rel*-7b*S*,10a*S*)-1,2,3,4,8,9,10,10a-Octahydro-7b*H*-Cyclopenta[*b*][1,4]-Diazepino[6,7,1-*hi*]Indol-2-ylmethanol**

Following the procedure of Example 32F, (2*S*)-(*rel*-7b*S*,10a*S*)-2-hydroxymethyl-2,3,8,9,10,10a-hexahydro-7b*H*-cyclopenta[*b*]-[1,4]diazepino[6,7,1-*hi*]indole-1,4-dione (0.26 mmol, 0.070 g) was reduced with 1 M $BH_3$•THF (1 mL) to yield a solid (0.17 mmol, 0.046 g, 65%).
MS ((+)APCI, m/e(%)) 323(35, $[M+H+DMSO]^+$, 245(100, $[M+H]^+$).
$^1$H NMR (DMSO-$d_6$, 400MHz) δ 6.85(d, J=7.1Hz, 1H), 6.73(d, J=7.3Hz, 1H), 6.52(t, J=7.3Hz, 1H), 4.70(m, 1H), 3.86, 3.50(ABq, $J_{AB}$=14.9Hz, 2H), 3.85(m, 1H), 3.70(dt, J=2.9Hz, 9.0Hz, 1H), 3.35(m, 1H), 3.22(m, 2H), 2.64(m, 1H), 2.40 (m, 1H), 1.90(m, 1H), 1.75(m, 1H), 1.60(m, 2H), 1.50(m, 1H), 1.40(m, 2H).

The ability of the compounds of this invention to act as $5HT_{2c}$ agonists was established is several standard pharmacological test procedures; the procedures used and results obtained are provided below.

**Test Procedures**

5HT2$_C$ Receptor Binding Test Procedure

To evaluate high affinity for the 5HT2$_C$ receptor, a CHO (Chinese Hamster Ovary) cell line transfected with the cDNA expressing the human 5-hydroxytryptamine2$_C$ (h5HT2$_C$) receptor was maintained in DMEM (Dulbecco's Modified Eagle Media) supplied with fetal calf serum, glutamine, and the markers: guaninephosphoribosyl transferase (GTP) and hypoxanthinethymidine (HT). The cells were allowed to grow to confluence in large culture dishes with intermediate changes of media and splitting. Upon reaching confluence, the cells were harvested by scraping. The harvested cells were suspended in half volume of fresh physiological phosphate buffered saline (PBS) solution and centrifuged at low speed (900 x g). This operation was repeated once more. The collected cells were then homogenized with a polytron at setting #7 for 15 sec in ten volumes of 50 mM Tris.HCl, pH 7.4 and 0.5 mM EDTA. The homogenate was centrifuged at 900 x g for 15 min to remove nuclear particles and other cell debris. The pellet was discarded and the supernatant fluid recentrifuged at 40,000 x g for 30 min. The resulting pellet was resuspended in a small volume of Tris.HCl buffer and the tissue protein content was determined in aliquots of 10-25 microliter (µl) volumes. Bovine Serum Albumin (BSA) was used as the standard in the protein determination by the method of Lowry et al., (J. Biol. Chem., 193:265 (1951). The volume of the suspended cell membranes was adjusted with 50 mM Tris.HCl buffer containing: 0.1 % ascorbic acid, 10 mM pargyline and 4 mM $CaCl_2$ to give a tissue protein concentration of 1-2 mg per ml of suspension. The preparation membrane suspension (many times concentrated) was aliquoted in 1 ml volumes and stored at -70 C until used in subsequent binding experiments.

Binding measurements were performed in a 96 well microtiter plate format, in a total volume of 200 µl. To each well was added: 60 µl of incubation buffer made in 50 mM Tris.HCl buffer, pH 7.4 and containing 4 mM $CaCl_2$; 20 µl of [$^{125}$I] DOI (S.A., 2200 Ci/mmol, NEN Life Science).

The dissociation constant, KD of [$^{125}$I] DOI at the human serotonin 5HT$_{2C}$ receptor was 0.4 nM by saturation binding with increasing concentrations of [$^{125}$I] DOI. The reaction was initiated by the final addition of 100.0 µl of tissue suspension containing 50 µg of receptor protein. Nonspecific binding is measured in the presence of 1 µM unlabeled DOI added in 20.0 µl volume. Test compounds were added in 20.0 ml. The mixture was incubated at room temperature for 60 min. The incubation was stopped by rapid filtration. The bound ligand-receptor complex was filtered off on a 96 well unifilter with a Packard ® Filtermate 196 Harvester. The bound complex caught on the filter disk was dried in a vacuum oven heated to 60° C and the radioactivity measured by liquid scintillation with 40 µl Microscint-20 scintillant in a Packard TopCount® equipped with six (6) photomultiplier detectors.

Specific binding is defined as the total radioactivity bound less the amount bound in the presence of 1 µM unlabeled DOI. Binding in the presence of varying concentrations of test drugs is expressed as percent of specific binding in the absence of drug. These results are then plotted as log % bound vs log concentration of test drug. Non linear regression analysis of data points yields both the IC50 and the Ki values of test compounds with 95% confidence limits. Alternatively, a linear regression line of decline of data points is plotted, from which the IC50 value can be read off the curve and the Ki value determined by solving the following equation:

$$Ki = \frac{IC50}{1+L/KD}$$

where L is the concentration of the radioactive ligand used and the KD is the dissociation constant of the ligand for the receptor, both expressed in nM.

The following Ki's are provided for various reference compounds:

Ki value and 95% confidence interval.

| | |
|---|---|
| Ritanserin | 2.0(1.3-3.1)nM |
| Ketanserin | 94.8 (70.7 - 127.0) nM |
| Mianserin | 2.7 (1.9 - 3.8) nM |
| Clozapine | 23.2 (16.0 - 34.0) nM |
| Methiothepin | 4.6 (4.0 - 6.0) nM |
| Methysergide | 6.3 (4.6 - 8.6) nM |
| Loxapine | 33.0 (24.0 - 47.0) nM |
| mCPP | 6.5 (4.8 - 9.0) nM |
| DOI | 6.2 (4.9 - 8.0) nM |

Stimulation of [$^3$H] Inositol Monophosphate production by $5HT_{2C}$ agonists.

CHO cells transfected with the cDNA expressing the human $5\text{-}HT_{2c}$ receptor were cultured in Dulbecco's modified Eagle's medium (DMEM) supplemented with 10% fetal bovine serum and non-essential amino acids. Upon reaching confluence the cells were harvested using PBS/EDTA and plated in 24 well plates at an initial density of 2.5 x $10^5$ cells per well. One (1) ml of maintenance medium containing 1μCi/ml myo-[$^3$H] inositol was added to each well. After 48 hours labeling, the cells were washed once with 0.5 ml DMEM containing 25 mM HEPES and 10 mM LiCl, then preincubated with the medium for 30 min (antagonists were included in this period if tested). At the end of the preincubation, the medium was removed, the cells were then incubated with test compounds (in presence of antagonists if needed) for 30 min. The reaction was terminated by removal of the incubation solution and addition of 0.5 ml ice-cold 5% PCA, followed by 15 to 30 min incubation on ice. 200 μl of 0.5 M Tes/1.5 M $K_2CO_3$ was added to each well to neutralize to pH 7, and plates were left on ice for another 15 to 30 min to precipitate all salts. The liquid and solid phases were separated by centrifugation.

A portion (350μl) of the upper aqueous phase was applied to Dowex AG-1X8 (formate form, 100-200 mesh) columns. The columns were then washed stepwise with 10 ml of water and 10 ml of 25 mM ammonium formate to remove free myo-[$^3$H]inositol and deacylated phosphoinositol, respectively. Finally 10 ml of 0.2 M ammonium formate solution was applied to the columns to elute [$^3$H] inositol monophosphate ([$^3$H] $IP_1$) directly into scintillation vials. Of this eluate, 1 ml was used to determine radioactivity by scintillation counting.

Agonist-stimulated levels of [$^3$H]inositol monophosphate ($IP_1$) is expressed as a percentage of the response observed with a maximally effective concentration of 5-HT (10μM). A 3-parameter logistic function is used to generate estimate of $EC_{50}/IC_{50}$. Antagonists are tested in the presence of 10 μM 5-HT.

The following data are provided for various reference compounds:

| | | |
|---|---|---|
| 5-HT | 15.1 nM | $EC_{50}$ |
| mCPP | 46.8 nM | $EC_{50}$ |
| | 60% | $E_{MAX}$ (relative to 5-HT) |
| SB200646 | 286 nM | $IC_{50}$ (10μM 5-HT as agonist) |

Effects of compounds on feeding behavior in rats

Eight (8) male Sprague-Dawley rats weighing 150-180g were separated into individual cages and acclimated to a powdered diet for 2 weeks. During this period and throughout the test procedure, the food cup and the animals were weighed daily. Following the acclimation period, animals were fasted for 24 hours and then injected with either vehicle or one of 4 doses of the test compound. Food intake was assessed at 2 and 24 hours following compound administration. Compounds to be evaluated were injected 1 -2 x per week until all animals had received all doses of the test compound. The order of doses were chosen using to a modified Latin Square design. Additional studies may be conducted in satieted rats at the start of the dark cycle. Compounds were injected i.p, s.c. or p.o. At the end of the study effects of the test compound on food intake was evaluated using a repeated measures ANOVA. Data were collected were 2 hour food intake (g). Data were subjected to one-way ANOVA with posthoc t-tests to assess group differences. Where appropriate, ED50 values were calculated. The ED50 value is the dose that produces a 50% reduction in food intake during the test period.

**Results**

| Results from in vitro Test Procedures | | | |
|---|---|---|---|
| Compound | $5HT_{2C}$ Affinity DOI/Agonist binding (Ki, nM) | $5HT_{2C}$ % Emax (5HT, 100%) | Stimulation of IP3 (EC50, nM) |
| Example 1 | 97 | | |
| Example 2 | 18 | 110 | 136 |
| Example 4A | 2021 | 40 | 1254 |
| Example 4B | 10 | 100 | 76 |
| Example 6 | 985 | | |

Results from in vivo $5HT_{2C}$ Food Intake in Rats (24 hr fast)

| Compound | Route of Admin. | ED50 (mg/kg) |
|---|---|---|
| Example 2 | ip | 8.05 |
| Example 4B | ip | 2.93 |

| Compound | $5\text{-}HT_{2c}$ Affinity DOI/Agonist Binding (Ki, μM) |
|---|---|
| Example 7 | 6.0 |
| Example 8 | 5.9 |
| Example 9 | 0.91 |
| Example 10 | 4.3 |
| Example 11 | 1.9 |
| Example 12 | >5 |
| Example 13 | 0.31 |

## Claims

1. A compound of the formula:

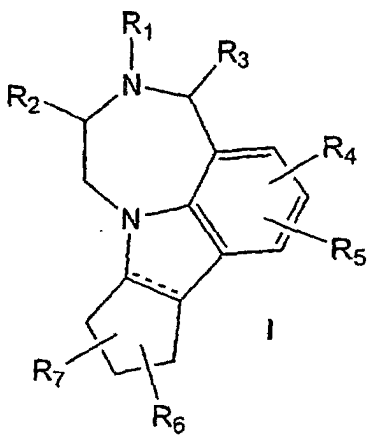

I

wherein:

$R_1$ is hydrogen, alkyl of 1-6 carbon atoms, acyl of 2-7 carbon atoms, aryl, heteroaryl, or -C(O)R' wherein R' is alkyl of from 1 to 6 carbon atoms, aryl, or heteroaryl;

$R_2$ and $R_3$ are each, independently, hydrogen, alkyl of 1-6 carbon atoms, cycloalkyl, of 3-7 carbon atoms, alkoxy of 1-6 carbon atoms, $-CH_2OH$, fluorinated alkyl of 1-6 carbon atoms, $-NH-SO_2$-alkyl of 1-6 carbon atoms, $-SO_2$-NH-alkyl of 1-6 carbon atoms, alkyl amide of 1-6 carbon atoms, amino, alkylamino of 1-6 carbon atoms, dialkylamino of 1-6 carbon atoms per alkyl moiety, fluorinated alkoxy of 1-6 carbon atoms, acyl of 2-7 carbon atoms, aryl, heteroaryl, aroyl or heteroaroyl.

$R_4$ and $R_5$ are each, independently, hydrogen, alkyl of 1-6 carbon atoms, alkoxy of 1-6 carbon atoms, halogen, fluorinated alkyl of 1-6 carbon atoms, -CN, $-NH-SO_2$-alkyl of 1-6 carbon atoms, $-SO_2$-NH-alkyl of 1-6 carbon atoms, alkyl amide of 1-6 carbon atoms, amino, alkylamino of 1-6 carbon atoms, dialkylamino of 1-6 carbon atoms per alkyl moiety, fluorinated alkoxy of 1-6 carbon atoms, acyl of 2-7 carbon atoms, aroyl or heteroaroyl;

$R_6$ and $R_7$ are each independently hydrogen, $C_1$-$C_6$ alkyl, cycloalkyl of 3 to 7 carbon atoms or -$CH_2$-(cycloalkyl of 3 to 7 carbon atoms);

the dashed line indicates an optional double bond;

or a pharmaceutically acceptable salt thereof.

**2.** A compound according to Claim wherein $R_1$ hydrogen, $C_1$-$C_6$ alkyl, benzoyl or alkanoyl of 2-7 carbon atoms.

**3.** A compound according to Claim 1 or Claim 2 wherein $R_2$ is or hydrogen, alkyl or fluorinated alkyl of 1-6 carbon atoms, -$CH_2$OH or cycloalkyl of 5-7 carbon atoms.

**4.** A compound according to any one of Claims 1 to 3 wherein $R_3$ is hydrogen, alkyl or fluorinated alkyl of 1-6 carbon atoms, -$CH_2$OH or cycloalkyl of 5-7 carbon atoms.

**5.** A compound according to any one of Claims 1 to 4 wherein $R_4$ is hydrogen, alkyl of 1-6 carbon atoms, alkoxy of 1-6 carbon atoms, halogen, -CN, amino or fluorinated alkyl of 1 to 6 carbon atoms.

**6.** A compound according to any one of Claims 1 to 5 wherein $R_5$ is hydrogen, alkyl of 1-6 carbon atoms, alkoxy of 1-6 carbon atoms, halogen, -CN, amino or fluorinated alkyl of 1 to 6 carbon atoms.

**7.** A compound according to any one of Claims 1 to 6 wherein $R_6$ is hydrogen or alkyl of 1-6 carbon atoms.

**8.** A compound according to any one of Claims 1 to 7 wherein $R_7$ is hydrogen or alkyl of 1-6 carbon atoms.

**9.** A compound of Claim 1 having one of the formulae:

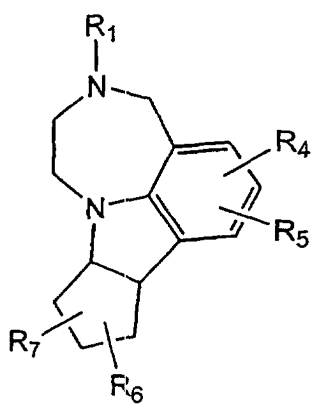

or

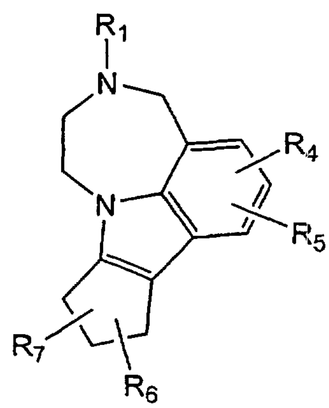

wherein $R_1$, $R_4$, $R_5$, $R_6$ and $R_7$ are as defined in Claim 1, or a pharmaceutically acceptable salt thereof.

**10.** A compound of Claim lor Claim 9 wherein $R_1$ and $R_7$ are hydrogen and $R_1$, $R_4$, $R_5$, and $R_6$ are as defined in Claim 1, or a pharmaceutically acceptable salt thereof.

**11.** A compound of Claim 1 having one of the formulae:

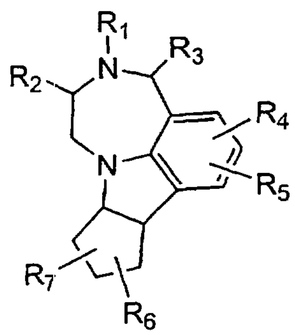

or

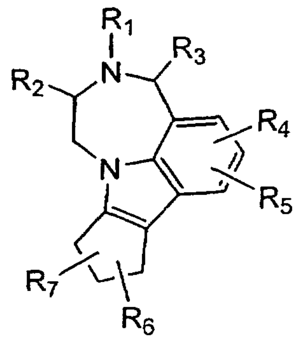

wherein:

- $R_1$ is hydrogen, or alkyl of 1-6 carbon atoms;
- $R_2$ and $R_3$ are each, independently, hydrogen, alkyl of 1-6 carbon atoms, cycloalkyl, alkoxy of 1-6 carbon atoms, $-CH_2OH$, or fluorinated alkyl of 1 to 6 carbon atoms;
- $R_4$ and $R_5$ are each, independently, hydrogen, alkyl of 1-6 carbon atoms, alkoxy of 1-6 carbon atoms, halogen, -CN, amino or fluorinated alkyl of 1 to 6 carbon atoms;
- $R_6$ and $R_7$ are each independently hydrogen or $C_1$-$C_6$ alkyl;

or a pharmaceutically acceptable salt thereof.

**12.** A compound of Claim 1 which is one of the following:

1,2,3,4,9,10-Hexahydro-8H-cyclopenta[b][1,4]diazepino[6,7,1-hi]indole;
1,2,3,4,8,9,10,10a-octahydro-7bH-cyclopenta[b][1,4]diazepino[6,7,1-hi]indole;
3-Acetyl-1,2,3,4,8,9,10,10a-octahydro-7bH-cyclopenta[b][1,4]diazepino[6,7,1-hi]-indole;
(7bR,10aR)-1,2,3,4,8,9,10,10a-octahydro-7bH-cyclopenta-[b][1,4]diazepino[6,7,1-hi]-indole;
6-Methyl-1,2,3,4,9,10-hexahydro-8H-cyclopenta[b][1,4]diazepino[6,7,1-hi]indole;
(2*S*)-(*rel*-7b*R*,10a*R*)-2-Methyl-1,2,3,4,8,9,10,10a-octahydro-7b*H*-cyclopenta[*b*][1,4]-diazepino[6,7,1-*hi*]Indole;
(2*S*)-(*rel*-7b*S*,10a*S*)-2-Methyl-1,2,3,4,8,9,10,10a-octahydro-7b*H*-cyclopenta[*b*][1,4]-diazepino[6,7,1-*hi*]indole;
(2*R*)-(*rel*-7b*R*,10a*R*)-2-Methyl-1,2,3,4,8,9,10,10a-octahydro-7b*H*-cyclopenta[*b*][1,4]-diazepino[6,7,1-*hi*]indole;
(2*R*)-(*rel*-7b*S*,10a*S*)-2-Methyl-1,2,3,4,8,9,10,10a-octahydro-7b*H*-cyclopenta[*b*][1,4]-diazepino[6,7,1-*hi*]Indole;
(2*R*,7b*S*,10a*S*)-1,2,3,4,8,9,10,10a-octahydro-7b*H*-cyclopenta[*b*][1,4]diazepino[6,7,1-*hi*]indol-2-ylmethanol;
*rel*-(4*S*,7b*S*,10a*S*)-4-Methyl-1,2,3,4,8,9,10,10a-octahydro-7b*H*-cyclopenta[*b*][1,4]-diazepino[6,7,1-*hi*]indole, or
*rel*-(4*R*,7b*S*,10a*S*)-4-Methyl-1,2,3,4,8,9,10,10a-octahydro-7b*H*-cyclopenta[*b*][1,4]-diazepino[6,7,1-*hi*]Indole

or a pharmaceutically acceptable salt thereof.

**13.** A pharmaceutical composition comprising a pharmaceutically effective amount of a compound according to any one of Claims 1 to 12 or a pharmaceutically acceptable salt thereof, and a pharmaceutically acceptable carrier or excipient.

**14.** The use of a pharmaceutically effective amount of a compound according to any one of Claims 1 to 12 or a pharmaceutically acceptable salt thereof in the manufacture of a medicament for the treatment of schizophrenia in a mammal.

**15.** The use of a pharmaceutically effective amount of a compound according to any one of Claims 1 to 12 or a pharmaceutically acceptable salt thereof in the manufacture of a medicament for the treatment of obsessive-compulsive disorder, depression, anxiety, panic disorder, anxiety, generalized anxiety disorder, obesity or epilepsy in a mammal.

**16.** A process for preparing a compound of formula (I) as claimed in claim 1 which comprises one of the following:

a) reacting a compound of formula

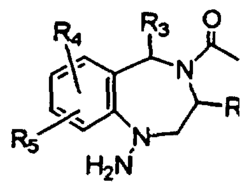

wherein R', $R_2$, $R_3$, $R_4$ and $R_5$ are as defined in Claim 1, with a compound of formula:

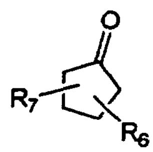

wherein $R_6$ and $R_7$ are as defined in Claim 1, and cyclising the resultant hydrazone, to give a corresponding compound of formula (I) wherein $R_1$ is -C(O)R' and the optional bond is present;
or

b) reducing a compound of formula

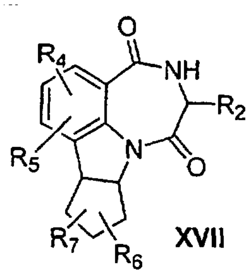

XVII

wherein $R_2$, $R_4$, $R_5$,$R_6$ and $R_7$ are as defined in Claim 1, with a reducing agent to give a corresponding compound of formula (I) wherein $R_3$ is hydrogen and the optional bond is absent;
or

c) reacting a compound of formula (XXIV):

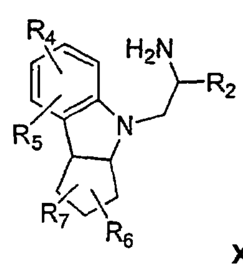

XXIV

wherein $R_2$, $R_4$, $R_5$,$R_6$ and R $_7$ are as defined in Claim 1, with a compound of formula

$R_3CHO$

wherein $R_3$ is as defined above, to give a corresponding compound of formula (I) wherein the optional bond is absent;

d) reducing a diazabenzo[*cd*]cyclopenta[*a*]azulen-6-one compound of formula XXXV.

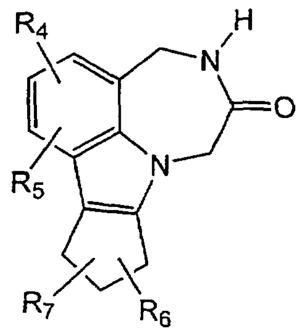

XXXV

wherein $R_4$, $R_5$, $R_6$ and $R_7$ are as defined in Claim 1, with a reducing agent to a corresponding compound of formula (I) wherein $R_2$ is hydrogen;
or

e) reducing a compound of formula (I) as defined in Claim 1 wherein the optional bond is present to provide a compound of formula (I) wherein the optional bond is absent;

f) hydrolysing a compound of formula (I) as defined in Claim 1 wherein $R_1$ is acyl or -C(O)R' to give a compound of formula (I) wherein $R_1$ is hydrogen;
or

g) acylating a compound of formula (I) as defined in Claim 1 wherein $R_1$ is hydrogen with an acylating agent containing the group -C(O)R' to give a compound of formula (I) wherein $R_1$ is acyl or -C(O)R';
or

h) alkylating a compound of formula (I) as defined in Claim 1 wherein $R_1$ is hydrogen with an alkylating agent containing the group -$R_1$ wherein $R_1$ is alkyl or aryl to give a compound of formula (I) wherein $R_1$ is alkyl or aryl;
or

i) removing a protecting group from a compound of formula (I) as defined in Claim 1 in which at least one substituent carries a protecting group to give a compound of formula (I);
or

j) converting a basic compound of formula (I) as defined in Claim 1 to a salt thereof by reaction with a pharmaceutically acceptable acid or vice versa;
or

k) converting a compound of formula (I) as defined in Claim 1 having one or more reactive substituent groups to a different compound of formula (I);
or

1) isolating an isomer of a compound of formula (I) as defined in Claim 1 from a mixture thereof.

**17.** A compound of the formula:

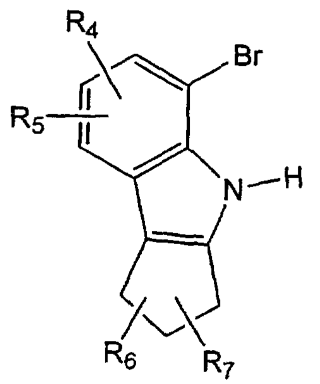

wherein $R_4$, $R_5$, $R_6$ and $R_7$ are as defined in Claim 1, or Claims 5 to 8.

**18.** A compound of Claim 17 which is one of the following:

5-Bromo-1,2,3,4-tetrahydro-cyclopenta[*b*]indole;
5-Bramo-3-methyl-1,2,3,4-tetrahydro-cyclopenta[*b*]indole;
5-Bromo-2-methyl-1,2,3,4-tetrahydro-cyclopenta[*b*]indole, or
5-Bromo-1-methyl-1,2,3,4-tetrahydro-cyclopenta[*b*]indole.

**19.** A compound of the formula:

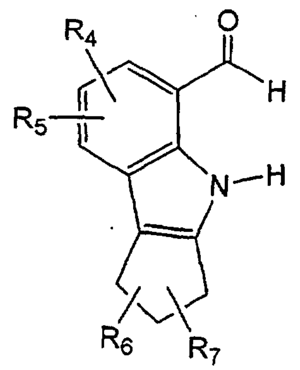

wherein $R_4$, $R_5$, $R_6$ amd $R_7$ are as defined in Claim 1 or Claims 5 to 8.

**20.** A compound of Claim 19 which is one of the following:

1,2,3,4-Tetrahydro-cyclopenta[*b*]indole-5-carbaldehyde;
3-Methyl-1,2,3,4-tetrahydro-cyclopenta[*b*]indole-5-carbaldehyde;
2-Methyl-1,2,3,4-tetrahydro-cyclopenta[*b*]indole-5-carbaldehyde, or
1-Methyl-1,2,3,4-tetrahydro-cyclopenta[*b*]indole-5-carbaldehyde.

**21.** A compound of the formula:

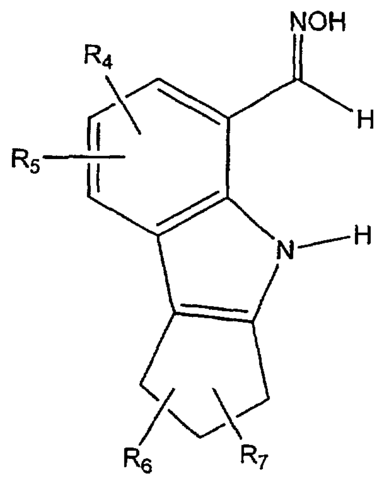

wherein $R_4$, $R_5$, $R_6$ and $R_7$ are as defined in Claim 1 or Claims 5 to 8.

**22.** A compound of Claim 21 which is one of the following:

1,2,3,4-Tetrahydro-cyclopenta[*b*]indole-5-carbaldehyde oxime;
3-Methyl-1,2,3,4-tetrahydro-cyclopenta[*b*]indole-5-carbaldehyde oxime;
2-Methyl-1,2,3,4-tetrahydro-cyclopenta[*b*]indole-5-carbaldehyde oxime, or
1-Methyl-1,2,3,4-tetrahydro-cyclopenta[*b*]indole-5-carbaldehyde oxime.

**23.** A compound of the formula:

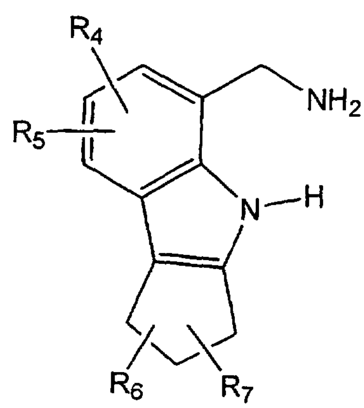

wherein $R4_1$, $R_5$, $R_6$ and $R_7$ are as defined in Claim 1 or Claims 5 to 8.

**24.** A compound of Claim 23 which is one of the following:

*C*-(1,2,3,4-Tetrahydro-cyclopenta[*b*]indol-5-yl)-methylamine;
*C*-(3-Methyl-1,2,3,4-tetrahydro-cyclopenta[*b*]indol-5-yl)-methylamine;
*C*-(2-Methyl-1,2,3,4-tetrahydro-cyclopenta[*b*]indol-5-yl)-methylamine, or
*C*-(1-Methyl-1,2,3,4-tetrahydro-cyclopenta[*b*]indol-5-yl)-methylamine.

**25.** A compound of the formula:

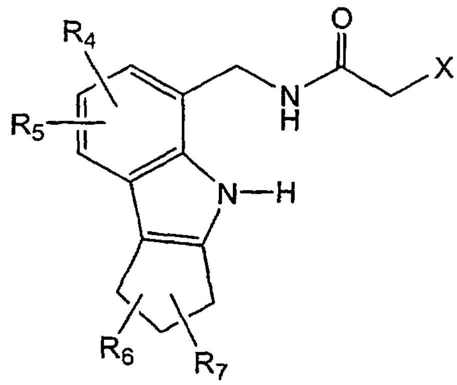

wherein $R_4$, $R_5$, $R_6$ and $R_7$ are as defined in Claim 1 or Claims 5 to 8 and X is selected from Cl, Br or I.

**26.** A compound of Claim 25 which is selected from the group of:

2-Chloro-*N*-(1,2,3,4-tetrahydro-cyclopenta[*b*]indol-5-ylmethyl)-acetamide;
2-Chloro-*N*-(3-methyl-1,2,3,4-tetrahydro-cyclopenta[*b*]indol-5-ylmethyl)-acetamide;
2-Chloro-*N*-(2-methyl-1,2,3,4-tetrahydro-cyclopenta[*b*]indol-5-ylmethyl)-acetamide;
2-Chloro-*N*-(1-methyl-1,2,3,4-tetrahydro-cyclopenta[*b*]indol-5-ylmethyl)-acetamide;
2-Bromo-*N*-(1,2,3,4-tetrahydro-cyclopenta[*b*]indol-5-ylmethyl)-acetamide;
2-Bromo-*N*-(3-methyl-1,2,3,4-tetrahydro-cyclopenta[*b*]indol-5-ylmethyl)-acetamide;
2-Bromo-*N*-(2-methyl-1,2,3,4-tetrahydro-cyclopenta[*b*]indol-5-ylmethyl)-acetamide;
2-Bromo-*N*-(1-methyl-1,2,3,4-tetrahydro-cyclopenta[*b*]indol-5-ylmethyl)-acetamide;
2-Iodo-*N*-(1,2,3,4-tetrahydro-cyclopenta[*b*]indol-5-ylmethyl)-acetamide;
2-Iodo-*N*-(3-methyl-1,2,3,4-tetrahydro-cyclopenta[*b*]indol-5-ylmethyl)-acetamide;
2-Iodo-*N*-(2-meth-1,2,3,4-tetrahydro-cyclopenta[*b*]indol-5-ylmethyl)-acetamide; or
2-Iodo-*N*-(1-methyl-1,2,3,4-tetrahydro-cyclopenta[*b*]indol-5-ylmethyl)-acetamide.

**27.** A compound of the formula:

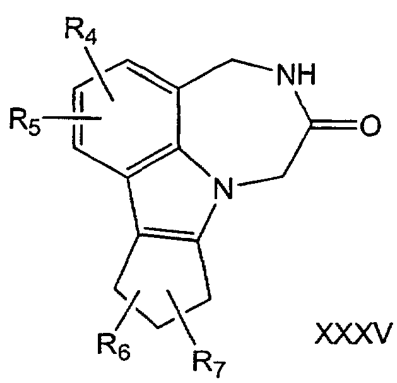

XXXV

wherein $R_4$, $R_5$, $R_3$, $R_6$ and $R_7$ are as defined in Claim 1 or claims 5-8.

**28.** A compound of Claim 27 which is one of the following:

4,5,9,10-Tetrahydro-8*H*-5,7a-diaza-benzo[*cd*]cyclopenta[*a*]azulen-6-one;
8-Methyl-4,5,9,10-tetrahydro-8*H*-5,7a-diaza-benzo[*cd*]cyclopenta[*a*]azulen-6-one;
9-Methyl-4,5,9,10-tetrahydro-8*H*-5,7a-diaza-benzo[*cd*]cyclopenta[*a*]azulen-6-one; or
10-Methyl-4,5,9,10-tetrahydro-8*H*-5,7a-diaza-benzo[*cd*]cyclopenta[*a*]azulen-6-one.

**29.** A compound having one of the formulae:

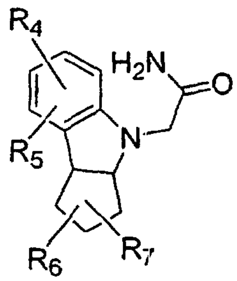

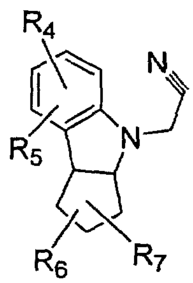

or

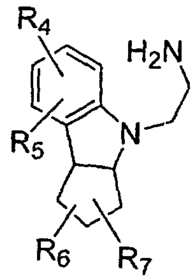

wherein $R_4$, $R_5$, $R_6$ and $R_7$ are each, independently, hydrogen, hydroxy, alkyl of 1-6 carbon atoms, cycloalkyl, of 3 to 7 carbon atoms alkoxy of 1-6 carbon atoms, halogen, fluorinated alkyl of from 1 to 6 carbon atoms, -CN, -NH-$SO_2$-alkyl of 1-6 carbon atoms, -$SO_2$-NH-alkyl of 1-6 carbon atoms, alkylamide of 1-6 carbon atoms, amino, alkylamino of 1-6 carbon atoms, dialkylamino of 1-6 carbon atoms per alkyl moiety, fluorinated alkoxy of 1-6 carbon atoms, acyl of 2-7 carbon atoms, aryl, heteroaryl, aroyl or heteroaroyl.

**30.** A compound of Claim 29 wherein $R_4$ and $R_5$ are hydrogen, and $R_6$ and $R_7$ are as defined in Claim 1.

**31.** A compound of Claim 29 wherein $R_4$, $R_5$ and $R_6$ are hydrogen, and $R_7$ is as defined in Claim 1.

**32.** A compound of Claim 29 which is 2-(2,3,3a,8b-Tetrahydro-1H-cyclo-penta[*b*]indol-4-yl)-acetamide.

**33.** A compound of Claim 29 which is one of the following:

2-(2,3,3a,8b-Tetrahydro-1*H*-cyclopenta[*b*]indol-4-yl)-acetamide;
2-(2,3,3a,8b-Tetrahydro-1*H*-cyclopenta[*b*]indol-4-yl)-acetonitrile, or
2-(2,3,3a,8b-Tetrahydro-1*H*-cyclopenta[*b*]indol-4-yl)-ethylamine.

## Patentansprüche

**1.** Verbindung der Formel:

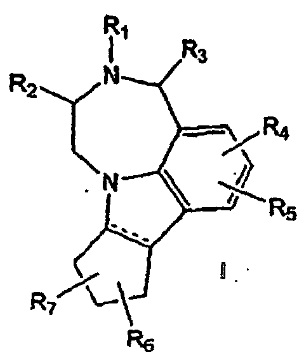

I.

worin:

$R_1$ für Wasserstoff, Alkyl mit 1-6 Kohlenstoffatomen, Acyl mit 2-7 Kohlenstoffatomen, Aryl, Heteroaryl oder -C(O)R' steht, worin R' ein Alkyl mit von 1 bis 6 Kohlenstoffatomen, Aryl oder Heteroaryl darstellt;
$R_2$ und $R_3$ jeweils unabhängig Wasserstoff, Alkyl mit 1-6 Kohlenstoffatomen, Cycloalkyl mit 3-7 Kohlenstoffatomen, Alkoxy mit 1-6 Kohlenstoffatomen, -$CH_2OH$, fluoriertes Alkyl mit 1-6 Kohlenstoffatomen, -NH-$SO_2$-Alkyl mit 1-6 Kohlenstoffatomen, -$SO_2$-NH-Alkyl mit 1-6 Kohlenstoffatomen, Alkylamid mit 1-6 Kohlenstoffatomen, Amino, Alkylamino mit 1-6 Kohlenstoffatomen, Dialkylamino mit 1-6 Kohlenstoffatomen pro Alkylkomponente, fluoriertes Alkoxy mit 1-6 Kohlenstoffatomen, Acyl mit 2-7 Kohlenstoffatomen, Aryl, Heteroaryl, Aroyl oder Heteroaroyl darstellen;

$R_4$ und $R_5$ jeweils unabhängig Wasserstoff, Alkyl mit 1-6 Kohlenstoffatomen, Alkoxy mit 1-6 Kohlenstoffatomen, Halogen, fluoriertes Alkyl mit 1-6 Kohlenstoffatomen, -CN, -NH-$SO_2$-Alkyl mit 1-6 Kohlenstoffatomen, -$SO_2$-NH-Alkyl mit 1-6 Kohlenstoffatomen, Alkylamid mit 1-6 Kohlenstoffatomen, Amino, Alkylamino mit 1-6 Kohlenstoffatomen, Dialkylamino mit 1-6 Kohlenstoffatomen pro Alkylkomponente, fluoriertes Alkoxy mit 1-6 Kohlenstoffatomen, Acyl mit 2-7 Kohlenstoffatomen, Aroyl oder Heteroaroyl darstellen;
$R_6$ und $R_7$ jeweils unabhängig Wasserstoff, $C_1$-$C_6$-Alkyl, Cycloalkyl mit 3 bis 7 Kohlenstoffatomen oder -$CH_2$-(Cycloalkyl mit 3 bis 7 Kohlenstoffatomen) darstellen;
und die gestrichelte Linie eine optionale Doppelbindung anzeigt;

oder ein pharmazeutisch annehmbares Salz davon.

**2.** Verbindung gemäß Anspruch, worin $R_1$ für Wasserstoff, $C_1$-$C_6$-Alkyl, Benzoyl oder Alkanoyl mit 2-7 Kohlenstoffatomen steht.

**3.** Verbindung gemäß Anspruch 1 oder Anspruch 2, worin $R_2$ für oder Wasserstoff, Alkyl oder fluoriertes Alkyl mit 1-6 Kohlenstoffatomen, -$CH_2OH$ oder Cycloalkyl mit 5-7 Kohlenstoffatomen steht.

**4.** Verbindung gemäß einem der Ansprüche 1 bis 3, worin $R_3$ Wasserstoff, Alkyl oder fluoriertes Alkyl mit 1-6 Kohlenstoffatomen, -$CH_2OH$ oder Cycloalkyl mit 5-7 Kohlenstoffatomen darstellt.

**5.** Verbindung gemäß einem der Ansprüche 1 bis 4, worin $R_4$ Wasserstoff, Alkyl mit 1-6 Kohlenstoffatomen, Alkoxy mit 1-6 Kohlenstoffatomen, Halogen, -CN, Amino oder fluoriertes Alkyl mit 1 bis 6 Kohlenstoffatomen darstellt.

**6.** Verbindung gemäß einem der Ansprüche 1 bis 5, worin $R_5$ Wasserstoff, Alkyl mit 1-6 Kohlenstoffatomen, Alkoxy mit 1-6 Kohlenstoffatomen, -CN, Amino oder fluoriertes Alkyl mit 1 bis 6 Kohlenstoffatomen darstellt.

**7.** Verbindung gemäß einem der Ansprüche 1 bis 6, worin $R_6$ Wasserstoff oder Alkyl mit 1-6 Kohlenstoffatomen darstellt.

**8.** Verbindung gemäß einem der Ansprüche 1 bis 7, worin $R_7$ Wasserstoff oder Alkyl mit 1-6 Kohlenstoffatomen darstellt.

**9.** Verbindung nach Anspruch 1 mit einer der Formeln:

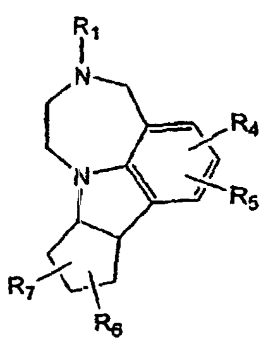

oder

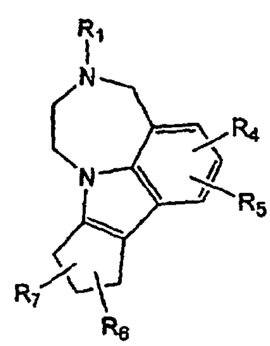

worin $R_1$, $R_4$, $R_5$, $R_6$ und $R_7$ wie in Anspruch 1 definiert sind, oder ein pharmazeutisch annehmbares Salz davon.

**10.** Verbindung nach Anspruch 1 oder Anspruch 9, worin $R_1$ und $R_7$ Wasserstoff darstellen und $R_1$, $R_4$, $R_5$ und $R_6$ wie in Anspruch 1 definiert sind, oder ein pharmazeutisch annehmbares Salz davon.

**11.** Verbindung nach Anspruch 1 mit einer der Formeln:

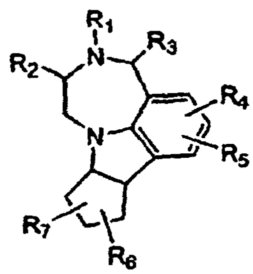

oder

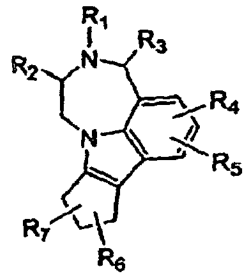

worin:

$R_1$ Wasserstoff oder Alkyl mit 1-6 Kohlenstoffatomen darstellt;
$R_2$ und $R_3$ jeweils unabhängig Wasserstoff, Alkyl mit 1-6 Kohlenstoffatomen, Cycloalkyl, Alkoxy mit 1-6 Kohlenstoffatomen, $-CH_2OH$ oder fluoriertes Alkyl mit 1 bis 6 Kohlenstoffatomen darstellen;
$R_4$ und $R_5$ jeweils unabhängig Wasserstoff, Alkyl mit 1-6 Kohlenstoffatomen, Alkoxy mit 1-6 Kohlenstoffatomen, Halogen, -CN, Amino oder fluoriertes Alkyl mit 1 bis 6 Kohlenstoffatomen darstellen;
$R_6$ und $R_7$ jeweils unabhängig Wasserstoff oder $C_1$-$C_6$-Alkyl darstellen;

oder ein pharmazeutisch annehmbares Salz davon.

**12.** Verbindung nach Anspruch 1, welche eine der Folgenden ist:

1,2,3,4,9,10-Hexahydro-8H-cyclopenta[b][1,4]diazepino[6,7,1-hi]indol;
1,2,3,4,8,9,10,10a-Octahydro-7bH-cyclopenta[b][1,4]diazepino [6,7,1-hi]indol;
3-Acetyl-1,2,3,4,8,9,10,10a-octahydro-7bH-cyclopenta[b][1,4] diazepino[6,7,1-hi]-indol;
(7bR,10aR)-1,2,3,4,8,9,10,10a-octahydro-7bH-cyclopenta-[b] [1,4]diazepino[6,7,1-hi]-indol;
6-Methyl-1,2,3,4,9,10-hexahydro-8H-cyclopenta[b][1,4]diazepino[6,7,1-hi]indol;
(2*S*)-(*rel*-7b*R*,10a*R*)-2-Methyl-1,2,3,4,8,9,10,10a-octahydro-7b H-cyclopenta[*b*][1,4]-diazepino[6,7,1-*hi*]indol;
(2*S*)-(*rel*-7b*S*,10a*S*)-2-Methyl-1,2,3,4,8,9,10,10a-octahydro-7b *H*-cyclopenta[*b*][1,4]-diazepino[6,7,1-*hi*]indol;
(2*R*)-(*rel*-7b*R*,10a*R*)-2-Methyl-1,2,3,4,8,9,10,10a-octahydro-7b *H*-cyclopenta[*b*][1,4]-diazepino[6,7,1-*hi*]indol;
(2*R*)-(*rel*-7b*S*,10a*S*)-2-Methyl-1,2,3,4,8,9,10,10a-octahydro-7b *H*-cyclopenta[*b*][1,4]-diazepino[6,7,1-*hi*]indol;
(2*R*,7b*S*,10a*S*)-1,2,3,4,8,9,10,10a-Octahydro-7b*H*-cyclopenta[*b*] [1,4]-diazepino[6,7,1-*hi*]indol-2-ylmethanol;
*rel*-(4*S*,7b*S*,10a*S*)-4-Methyl-1,2,3,4,8,9,10,10a-octahydro-7b*H*cyclopenta[*b*][1,4]-diazepino[6,7,1-*hi*]indol
oder
*rel*-(4*R*,7b*S*,10a*S*)-4-Methyl-1,2,3,4,8,9,10,10a-octahydro-7b*H*cyclopenta[*b*][1,4]-diazepino[6,7,1-*hi*]indol

oder ein pharmazeutisch annehmbares Salz davon.

**13.** Pharmazeutische Zusammensetzung, welche eine pharmazeutisch wirksame Menge einer Verbindung gemäß einem der Ansprüche 1 bis 12 oder eines pharmazeutisch annehmbaren Salzes davon und einen pharmazeutisch annehmbaren Träger oder Exzipienten umfasst.

**14.** Verwendung einer pharmazeutisch wirksamen Menge einer Verbindung gemäß einem der Ansprüche 1 bis 12 oder eines pharmazeutisch annehmbaren Salzes davon bei der Herstellung eines Medikament zur Behandlung von Schizophrenie in einem Säuger.

**15.** Verwendung einer pharmazeutisch wirksamen Menge einer Verbindung gemäß einem der Ansprüche 1 bis 12 oder eines pharmazeutisch annehmbaren Salzes davon bei der Herstellung eines Medikaments zur Behandlung von obsessiver Zwangsstörung, Depression, Angststörung, Panikstörung, Angststörung, generalisierter Angststörung, Fettsucht oder Epilepsie in einem Säuger.

**16.** Verfahren zum Herstellen einer Verbindung der Formel (I), wie in Anspruch 1 beansprucht, welches eines der Folgenden umfasst:

a) Umsetzen einer Verbindung der Formel

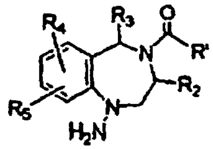

worin R' , $R_2$, $R_3$, $R_4$ und $R_5$ wie in Anspruch 1 definiert sind, mit einer Verbindung der Formel:

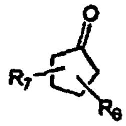

worin $R_6$ und $R_7$ wie in Anspruch 1 definiert sind, und Cyclisieren des sich ergebenden Hydrazons, um eine entsprechende Verbindung der Formel (I) zu ergeben, worin $R_1$ für -C(O)R' steht und die optionale Bindung vorhanden ist;
oder
b) Reduzieren einer Verbindung der Formel

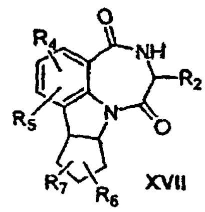
**XVII**

worin $R_2$, $R_4$, $R_5$, $R_6$ und $R_7$ wie in Anspruch 1 definiert sind, mit einem Reduktionsmittel, um eine entsprechende Verbindung der Formel (I) zu ergeben, worin $R_3$ für Wasserstoff steht und die optionale Bindung abwesend ist; oder
c) Umsetzen einer Verbindung der Formel (XXIV) :

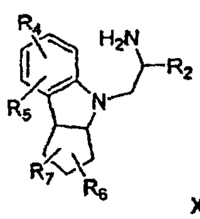
**XXIV**

worin $R_2$, $R_4$, $R_5$, $R_6$ und $R_7$ wie in Anspruch 1 definiert sind, mit einer Verbindung der Formel

$R_3CHO$,

worin $R_3$ wie oben definiert ist, um eine entsprechende Verbindung der Formel (I) zu ergeben, worin die optionale Bindung abwesend ist;
d) Reduzieren einer Diazabenzo[*cd*]cyclopenta[*a*]azulen-6-on-Verbindung der Formel **XXXV**:

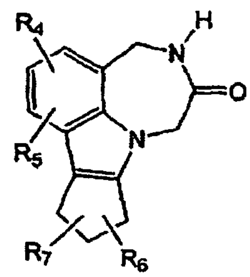

XXXV

worin $R_4$, $R_5$ und $R_7$ wie in Anspruch 1 definiert sind, mit einem Reduktionsmittel zu einer entsprechenden Verbindung der Formel (I), worin $R_2$ für Wasserstoff steht;
oder
e) Reduzieren einer Verbindung der Formel (I), wie in Anspruch 1 definiert, worin die optionale Bindung vorhanden ist, um eine Verbindung der Formel (I) vorzusehen, worin die optionale Bindung abwesend ist;
f) Hydrolysieren einer Verbindung der Formel (I), wie in Anspruch 1 definiert, worin $R_1$ für Acyl oder -C(O)R' steht, um eine Verbindung der Formel (I) zu ergeben, worin $R_1$ für Wasserstoff steht;
oder
g) Acylieren einer Verbindung der Formel (I), wie in Anspruch 1 definiert, worin $R_1$ für Wasserstoff steht, mit einem Acylierungsmittel, welches die Gruppe -C(O)R' enthält, um eine Verbindung der Formel (I) zu ergeben, worin $R_1$ für Acyl oder -C (O)R' steht;
oder
h) Alkylieren einer Verbindung der Formel (I) wie in Anspruch 1 beansprucht, worin $R_1$ für Wasserstoff steht, mit einem Alkylierungsmittel, welches die Gruppe -$R_1$ enthält, worin $R_1$ für Alkyl oder Aryl steht, um eine Verbindung der Formel (I) zu ergeben, worin $R_1$ für Alkyl oder Aryl steht;
oder
i) Entfernen einer Schutzgruppe von einer Verbindung der Formel (I) wie in Anspruch 1 definiert, worin mindestens ein Substituent eine Schutzgruppe trägt, um eine Verbindung der Formel (I) zu ergeben;
oder
j) Umwandeln einer basischen Verbindung der Formel (I), wie in Anspruch 1 definiert, in ein Salz davon durch Umsetzung mit einer pharmazeutisch annehmbaren Säure oder umgekehrt;
oder
k) Umwandeln einer Verbindung der Formel (I), wie in Anspruch 1 definiert, mit einer oder mehreren reaktiven Substituentengruppen in eine andere Verbindung der Formel (I);
oder
1) Isolieren eines Isomers einer Verbindung der Formel (I), wie in Anspruch 1 beansprucht, aus einem Gemisch daraus.

**17.** Verbindung der Formel:

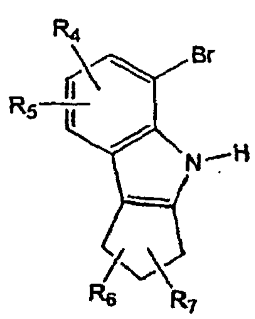

worin $R_4$, $R_5$, $R_6$ und $R_7$ wie in Anspruch 1 oder Ansprüchen 5 bis 8 definiert sind.

18. Verbindung nach Anspruch 17, welche eine der Folgenden ist:

5-Brom-1,2,3,4-tetrahydro-cyclopenta[*b*]indol;
5-Brom-3-methyl-1,2,3,4-tetrahydro-cyclopenta[*b*]indol;
5-Brom-2-methyl-1,2,3,4-tetrahydro-cyclopenta[*b*]indol oder
5-Brom-1-methyl-1,2,3,4-tetrahydro-cyclopenta[*b*]indol.

19. Verbindung der Formel:

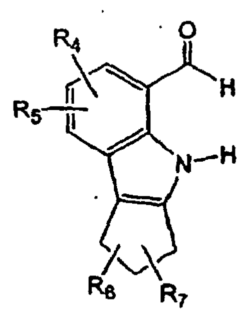

worin $R_4$, $R_5$, $R_6$ und $R_7$ wie in Anspruch 1 oder Ansprüchen 5 bis 8 definiert sind.

20. Verbindung nach Anspruch 19, welche eine der Folgenden ist:

1,2,3,4-Tetrahydro-cyclopenta[*b*]indol-5-carbaldehyd;
3-Methyl-1,2,3,4-tetrahydro-cyclopenta[*b*]indol-5-carbaldehyd;
2-Methyl-1,2,3,4-tetrahydro-cyclopenta[*b*]indol-5-carbaldehyd; oder
1-Methyl-1,2,3,4-tetrahydro-cyclopenta[*b*]indol-5-carbaldehyd.

21. Verbindung der Formel:

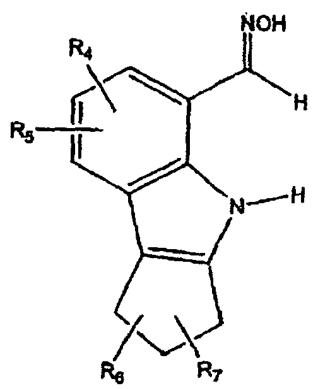

worin $R_4$, $R_5$, $R_6$ und $R_7$ wie in Anspruch 1 oder Ansprüchen 5 bis 8 definiert sind.

22. Verbindung nach Anspruch 21, welche eine der Folgenden ist:

1,2,3,4-Tetrahydro-cyclopenta[*b*]indol-5-carbaldehyd-oxim;
3-Methyl-1,2,3,4-tetrahydro-cyclopenta[*b*]indol-5-carbaldehyd-oxim;
2-Methyl-1,2,3,4-tetrahydro-cyclopenta[*b*]indol-5-carbaldehyd-oxim; oder
1-Methyl-1,2,3,4-tetrahydro-cyclopenta[*b*]indol-5-carbaldehyd-oxim.

23. Verbindung der Formel:

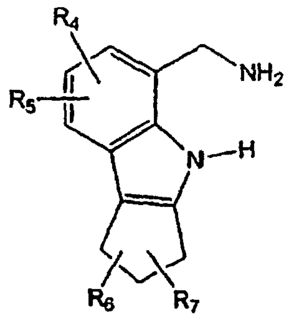

worin $R_4$, $R_5$, $R_6$ und $R_7$ wie in Anspruch 1 oder Ansprüchen 5 bis 8 definiert sind.

**24.** Verbindung nach Anspruch 23, welche eine der Folgenden ist:

*C*-(1,2,3,4-Tetrahydro-cyclopenta[*b*]indol-5-yl)-methylamin;
*C*-(3-Methyl-1,2,3,4-tetrahydro-cyclopenta[*b*]indol-5-yl)-methylamin;
*C*-(2-Methyl-1,2,3,4-tetrahydro-cyclopenta[*b*]indol-5-yl)-methylamin, oder
*C*-(1-Methyl-1,2,3,4-tetrahydro-cyclopenta[*b*]indol-5-yl)-methylamin.

**25.** Verbindung der Formel:

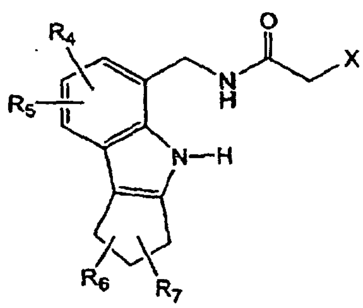

worin $R_4$, $R_5$, $R_6$ und $R_7$ wie in Anspruch 1 oder Ansprüchen 5 bis 8 definiert sind und X ausgewählt wird aus Cl, Br oder I.

**26.** Verbindung nach Anspruch 25, welche ausgewählt wird aus der Gruppe von:

2-Chlor-*N*-(1,2,3,4-tetrahydro-cyclopenta[*b*]indol-5-ylmethyl)-acetamid;
2-Chlor-*N*-(3-methyl-1,2,3,4-tetrahydro-cyclopenta[*b*]indol-5-ylmethyl)-acetamid;
2-Chlor-*N*-(2-methyl-1,2,3,4-tetrahydro-cyclopenta[*b*]indol-5-ylmethyl)-acetamid;
2-Chlor-*N*-(1-methyl-1,2,3,4-tetrahydro-cyclopenta[*b*]indol-5-ylmethyl)-acetamid;
2-Brom-*N*-(1,2,3,4-tetrahydro-cyclopenta[*b*]indol-5-ylmethyl)-acetamid;
2-Brom-*N*-(3-methyl-1,2,3,4-tetrahydro-cyclopenta[*b*]indol-5-ylmethyl)-acetamid;
2-Brom-*N*-(2-methyl-1,2,3,4-tetrahydro-cyclopenta[*b*]indol-5-ylmethyl)-acetamid;
2-Brom-*N*-(1-mathyl-1,2,3,4-tetrahydro-cyclopenta[*b*]indol-5-ylmethyl) -acetamid;
2-Iod-*N*-(1,2,3,4-tetrahydro-cyclopenta[*b*]indol-5-ylmethyl)-acetamid;
2-Iod-*N*-(3-methyl-1,2,3,4-tetrahydro-cyclopenta[*b*]indol-5-ylmethyl)-acetamid;
2-Iod-*N*-(2-methyl-1,2,3,4-tetrahydro-cyclopenta[*b*]indol-5-ylmethyl)-acetamid oder
2-Iod-*N*-(1-methyl-1,2,3,4-tetrahydro-cyclopenta[*b*]indol-5-ylmethyl) -acetamid.

**27.** Verbindung der Formel:

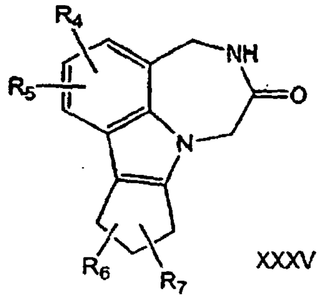

XXXV

worin $R_4$, $R_5$, $R_6$ und $R_7$ wie in Anspruch 1 oder Ansprüchen 5-8 definiert sind.

**28.** Verbindung nach Anspruch 27, welche eine der Folgenden ist:

4,5,9,10-Tetrahydro-8*H*-5,7a-diaza-benzo[*cd*]cyclopenta[a]azulen-6-on;
8-Methyl-4,5,9,10-Tetrahydro-8*H*-5,7a-diaza-benzo[*cd*]cyclopenta[a]azulen-6-on;
9-Methyl-4,5,9,10-Tetrahydro-8*H*-5,7a-diaza-benzo[*cd*]cyclopenta[*a*]azulen-6-on oder
10-Methyl-4,5,9,10-Tetrahydro-8*H*-5,7a-diaza-benzo[*cd*]cyclopenta [a] azulen-6-on.

**29.** Verbindung mit einer der Formeln:

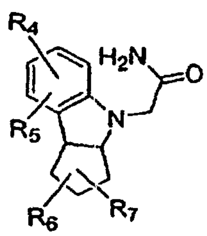

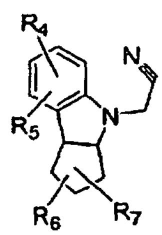

oder

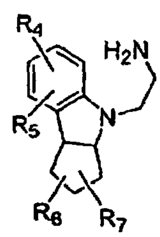

worin $R_4$, $R_5$, $R_6$ und $R_7$ jeweils unabhängig Wasserstoff, Hydroxy, Alkyl mit 1-6 Kohlenstoffatomen, Cycloalkyl mit 3 bis 7 Kohlenstoffatomen, Alkoxy mit 1-6 Kohlenstoffatomen, Halogen, fluoriertes Alkyl mit von 1 bis 6 Kohlenstoffatomen, -CN, -NH-$SO_2$-Alkyl mit 1-6 Kohlenstoffatomen, -$SO_2$-NH-Alkyl mit 1-6 Kohlenstoffatomen, Alkylamid mit 1-6 Kohlenstoffatomen, Amino, Alkylamino mit 1-6 Kohlenstoffatomen, Dialkylamino mit 1-6 Kohlenstoffatomen pro Alkylkomponente, fluoriertes Alkoxy mit 1-6 Kohlenstoffatomen, Acyl mit 2-7 Kohlenstoffatomen, Aryl, Heteroaryl, Aroyl oder Heteroaroyl darstellen.

**30.** Verbindung nach Anspruch 29, worin $R_4$ und $R_5$ Wasserstoff darstellen und $R_6$ und $R_7$ wie in Anspruch 1 definiert sind.

**31.** Verbindung nach Anspruch 29, worin $R_4$, $R_5$ und $R_6$ Wasserstoff darstellen und $R_7$ wie in Anspruch 1 definiert ist.

**32.** Verbindung nach Anspruch 29, welche 2-(2,3,3a,8b-Tetrahydro-1H-cyclopenta[*b*]indol-4-yl)-acetamid ist.

**33.** Verbindung nach Anspruch 29, welche eine der Folgenden ist:

2-(2,3,3a,8b-Tetrahydro-1*H*-cyclopenta[*b*]indol-4-yl)-acetamid;
2-(2,3,3a,8b-Tetrahydro-1*H*-cyclopenta[*b*]indol-4-yl)-acetonitril oder
2-(2,3,3a,8b-Tetrahydro-1*H*-cyclopenta[*b*]indol-4-yl)-ethylamin.

## Revendications

1. Composé de formule :

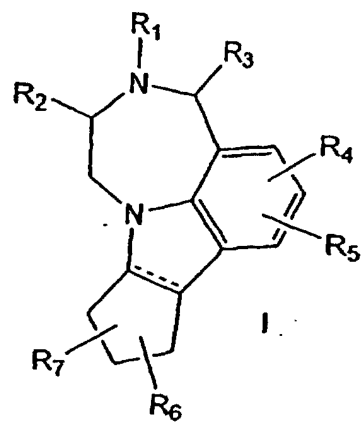

I

dans laquelle :

$R_1$ est un hydrogène, un alkyle de 1-6 atomes de carbone, un acyle de 2-7 atomes de carbone, un aryle, un hétéroaryle, ou -C(O)R' dans laquelle R' est un alkyle de 1 à 6 atomes de carbone, un aryle, ou un hétéroaryle;
$R_2$ et $R_3$ sont chacun, indépendamment, un hydrogène, un alkyle de 1-6 atomes de carbone, un cycloalkyle de 3-7 atomes de carbone, un alcoxy de 1-6 atomes de carbone, $-CH_2OH$, un alkyle fluoré de 1-6 atomes de carbone, $-NH-SO_2$-alkyle de 1-6 atomes de carbone, $-SO_2-NH$-alkyle de 1-6 atomes de carbone, un alkylamide de 1-6 atomes de carbone, un amino, un alkylamino de 1-6 atomes de carbone, un dialkylamino de 1-6 atomes de carbone par partie alkyle, un alcoxy fluoré de 1-6 atomes de carbone, un acyle de 2-7 atomes de carbone, un aryle, un hétéroaryle, un aroyle ou un hétéroaroyle.
$R_4$ et $R_5$ sont, chacun indépendamment, un hydrogène, un alkyle de 1-6 atomes de carbone, un alcoxy de 1-6 atomes de carbone, un halogène, un alkyle fluoré de 1-6 atomes de carbone, -CN, $-NH-SO_2$-alkyle de 1-6 atomes de carbone, $-SO_2-NH$-alkyle de 1-6 atomes de carbone, un alkylamide de 1-6 atomes de carbone, un amino, un alkylamino de 1-6 atomes de carbone, un dialkylamino de 1-6 atomes de carbone par partie alkyle, un alcoxy fluoré de 1-6 atomes de carbone, un acyle de 2-7 atomes de carbone, un aroyle ou un hétéroaroyle;
$R_6$ et $R_7$ sont chacun indépendamment un hydrogène, un alkyle en $C_1$-$C_6$, un cycloalkyle de 3 à 7 atomes de carbone ou $-CH_2$-(cycloalkyle de 3 à 7 atomes de carbone);
la ligne pointillée indique une double liaison facultative;

ou un sel pharmaceutiquement acceptable de celui-ci.

2. Composé suivant la revendication 1, dans lequel $R_1$ est un hydrogène, un alkyle en $C_1$-$C_6$, un benzoyle ou un alcanoyle de 2-7 atomes de carbone.

3. Composé suivant la revendication 1 ou la revendication 2, dans lequel $R_2$ est un hydrogène, un alkyle ou un alkyle fluoré de 1-6 atomes de carbone, $-CH_2OH$ ou un cycloalkyle de 5-7 atomes de carbone.

4. Composé suivant l'une quelconque des revendications 1 à 3 dans lequel $R_3$ est un hydrogène, un alkyle ou un alkyle fluoré de 1-6 atomes de carbone, $-CH_2OH$ ou un cycloalkyle de 5-7 atomes de carbone.

5. Composé suivant l'une quelconque des revendications 1 à 4, dans lequel $R_4$ est un hydrogène, un alkyle de 1-6 atomes de carbone, un alcoxy de 1-6 atomes de carbone, un halogène, -CN, un amino ou un alkyle fluoré de 1 à 6 atomes de carbone.

6. Composé suivant l'une quelconque des revendications 1 à 5, dans lequel $R_5$ est un hydrogène, un alkyle de 1-6 atomes de carbone, un alcoxy de 1-6 atomes de carbone, un halogène, -CN, un amino ou un alkyle fluoré de 1 à 6 atomes de carbone.

**7.** Composé suivant l'une quelconque des revendications 1 à 6, dans lequel $R_6$ est un hydrogène ou un alkyle de 1-6 atomes de carbone.

**8.** Composé suivant l'une quelconque des revendications 1 à 7, dans lequel $R_7$ est un hydrogène ou un alkyle de 1-6 atomes de carbone.

**9.** Composé suivant la revendication 1 présentant l'une des formules :

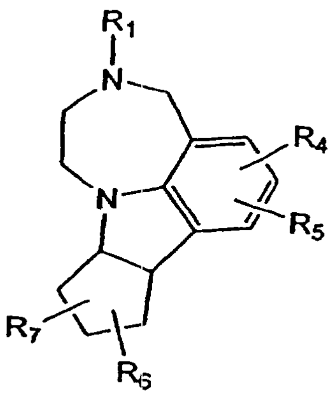

ou

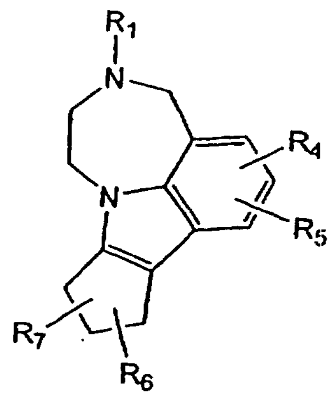

dans lesquelles $R_1$, $R_4$, $R_5$, $R_6$ et $R_7$ sont comme définis à la revendication 1, ou un sel pharmaceutiquement acceptable de celui-ci.

**10.** Composé suivant la revendication 1 ou la revendication 9 dans lequel $R_1$ et $R_7$ sont un hydrogène et $R_1$, $R_4$, $R_5$, et $R_6$ sont comme définis à la revendication 1, ou un sel pharmaceutiquement acceptable de celui-ci.

**11.** Composé suivant la revendication 1 présentant l'une des formules :

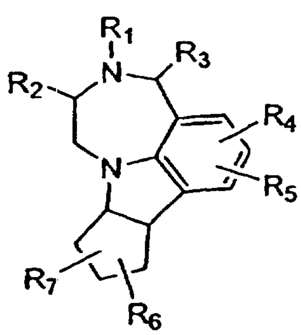

ou

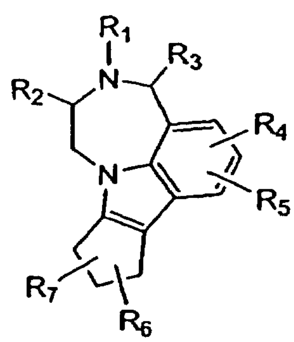

dans lesquelles :

$R_1$ est un hydrogène, ou un alkyle de 1-6 atomes de carbone;
$R_2$ et $R_3$ sont chacun, indépendamment, un hydrogène, un alkyle de 1-6 atomes de carbone, un cycloalkyle, un alcoxy de 1-6 atomes de carbone, $-CH_2OH$, ou un alkyle fluoré de 1 à 6 atomes de carbone;
$R_4$ et $R_5$ sont, chacun indépendamment, un hydrogène, un alkyle de 1-6 atomes de carbone, un alcoxy de 1-6 atomes de carbone, un halogène, -CN, un amino ou un alkyle fluoré de 1 à 6 atomes de carbone;
$R_6$ et $R_7$ sont chacun indépendamment un hydrogène ou un alkyle en $C_1$-$C_6$;

ou un sel pharmaceutiquement acceptable de celui-ci.

**12.** Composé suivant la revendication 1 qui est l'un des suivants :

le 1, 2, 3, 4, 9,10-hexahydro-8*H*-cyclopenta[*b*][1,4]diazépino[6,7,1-*hi*]indole;
le 1,2,3,4,8,9,10,10a-octahydro-7b*H*-cyclopenta [*b*][1,4]diazépino[6,7,1-*hi*]indole;
le 3-acétyl-1,2,3,4,8,9,10,10a-octahydro-7b*H*-cyclopenta[*b*][1,4]diazépino[6,7,1-*hi*]indole;
le (7b*R*,10a*R*)-1,2,3,4,8,9,10,10a-octahydro-7bH-cyclopenta[*b*][1,4]diazépino[6,7,1-*hi*]indole;
le 6-méthyl-1,2,3,4,9,10-hexahydro-8*H*-cyclopenta[*b*][1,4]diazépino[6,7,1-*hi*]indole;
le (2*S*)-(*rel*-7b*R*,10a*R*)-2-méthyl-1,2,3,4,8,9,10,10a-octahydro-7b*H*-cyclopenta[*b*][1,4]-diazépino[6,7,1-*hi*]indole;
le (2*S*)-(*rel*-7b*S*,10a*S*)-2-méthyl-1,2,3,4,8,9,10,10a-octahydro-7b*H*-cyclopenta[*b*][1,4]-diazépino[6,7,1-*hi*]indole;
le (2*R*)-(*rel*-7b*R*,10a*R*)-2-méthyl-1,2,3,4,8,9,10,10a-octahydro-7b*H*-cyclopenta[*b*][1,4]-diazépino[6,7,1-*hi*]indole;
le (2*R*)-(*rel*-7b*S*,10a*S*)-2-méthyl-1,2,3,4,8,9,10,10a-octahydro-7b*H*-cyclopenta[*b*][1,4]-diazépino[6,7,1-*hi*]indole;
le (2*R*,7b*S*,10a*S*)-1,2,3,4,8,9,10,10a-octahydro-7b*H*-cyclopenta[*b*][1,4]diazépino[6,7,1-*hi*]indol-2-ylméthanol;
le rel- (4*S*,7b*S*,10a*S*)-4-méthyl-1, 2, 3, 4, 8, 9, 10, 10a-octahydro-7b*H*-cyclopenta[*b*][1,4]-diazépino[6,7,1-*hi*] indole, ou
le *rel*- (4R, 7bS, 10a*S*)-4-méthyl-1,2,3,4, 8, 9, 10, 10a-octahydro-7b*H*-cyclopenta[*b*][1,4]-diazépino[6,7,1-*hi*] indole

ou un sel pharmaceutiquement acceptable de celui-ci.

**13.** Composition pharmaceutique comprenant une quantité efficace sur le plan pharmaceutique d'un composé suivant l'une quelconque des revendications 1 à 12 ou d'un sel pharmaceutiquement acceptable de celui-ci, et un vecteur ou excipient pharmaceutiquement acceptable.

**14.** Utilisation d'une quantité efficace sur le plan pharmaceutique d'un composé suivant l'une quelconque des revendications 1 à 12, ou d'un sel pharmaceutiquement acceptable de celui-ci dans la fabrication d'un médicament pour le traitement de la schizophrénie chez un mammifère.

**15.** Utilisation d'une quantité efficace sur le plan pharmaceutique d'un composé suivant l'une quelconque des revendications 1 à 12, ou d'un sel pharmaceutiquement acceptable de celui-ci dans la fabrication d'un médicament pour le traitement d'un trouble obsessionnel-impulsif, de la dépression, de l'anxiété, d'un trouble de panique, de l'anxiété, de l'anxiété généralisée, de l'obésité ou de l'épilepsie chez un mammifère.

**16.** Procédé de préparation d'un composé de formule (I) suivant la revendication 1, qui comprend l'une des étapes suivantes :

a) la réaction d'un composé de formule

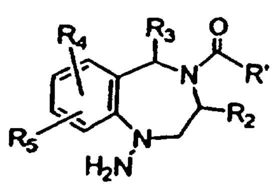

dans laquelle R' , $R_2$, $R_3$, $R_4$ et $R_5$ sont comme définis à la revendication 1, avec un composé de formule :

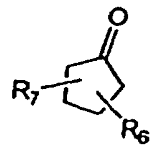

dans laquelle $R_6$ et $R_7$ sont comme définis à la revendication 1, et la cyclisation de l'hydrazone obtenue, pour donner un composé correspondant de formule (I) dans laquelle $R_1$ est -C(O)R' et la liaison facultative est présente;

ou

b) la réduction d'un composé de formule

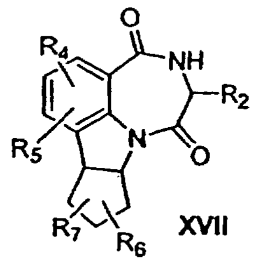

XVII

dans laquelle $R_2$, $R_4$, $R_5$, $R_6$ et $R_7$ sont comme définis à la revendication 1, avec un agent réducteur pour donner un composé correspondant de formule (I) dans laquelle $R_3$ est un hydrogène et la liaison facultative est absente;

ou

c) la réaction d'un composé de formule (XXIV) :

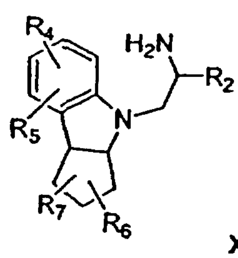

XXIV

dans laquelle $R_2$, $R_4$, $R_5$, $R_6$ et $R_7$ sont comme définis à la revendication 1, avec un composé de formule

$$R_3CHO$$

dans laquelle $R_3$ est comme défini ci-dessus, pour donner un composé correspondant de formule (I) dans laquelle la liaison facultative est absente;

d) la réduction d'un composé diazabenzo[*cd*]cyclopenta[*a*]azulén-6-one de formule XXXV :

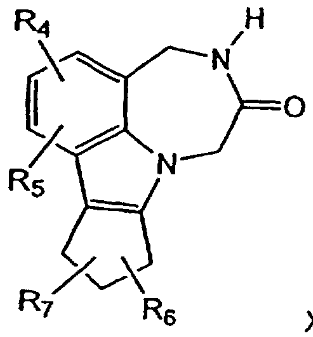

XXXV

dans laquelle $R_4$, $R_5$, $R_6$ et $R_7$ sont comme définis à la revendication 1, avec un agent réducteur pour donner un composé correspondant de formule (I) dans laquelle $R_2$ est un hydrogène;

ou

e) la réduction d'un composé de formule (I) comme défini à la revendication 1 dans laquelle la liaison facultative est présente pour donner un composé de formule (I) dans laquelle la liaison facultative est absente;

f) l'hydrolyse d'un composé de formule (I) comme défini à la revendication 1 dans laquelle $R_1$ est un acyle ou -C(O)R' pour donner un composé de formule (I) dans laquelle $R_1$ est un hydrogène;

ou

g) l'acylation d'un composé de formule (I) comme défini à la revendication 1 dans laquelle $R_1$ est un hydrogène avec un agent acylant contenant le groupement -C(O)R' pour donner un composé de formule (I) dans laquelle $R_1$ est un acyle ou -C(O)R';

ou

h) l'alkylation d'un composé de formule (I) comme défini à la revendication 1 dans laquelle $R_1$ est un hydrogène avec un agent alkylant contenant le groupement -$R_1$ dans laquelle $R_1$ est un alkyle ou un aryle pour donner un composé de formule (I) dans laquelle $R_1$ est un alkyle ou un aryle;

ou

i) l'enlèvement d'un groupement protecteur d'un composé de formule (I) comme défini à la revendication 1 dans laquelle au moins un substituant porte un groupement protecteur pour donner un composé de formule (I);

ou

j) la conversion d'un composé basique de formule (I) comme défini à la revendication 1 en un sel de celui-ci par réaction avec un acide pharmaceutiquement acceptable ou vice versa;

ou

k) la conversion d'un composé de formule (I) comme défini à la revendication 1 présentant un ou plusieurs groupements substituants réactifs en un composé différent de formule (I);

ou

1) l'isolement d'un isomère d'un composé de formule (I) comme défini à la revendication 1 d'un mélange de ceux-ci.

**17.** Composé de formule :

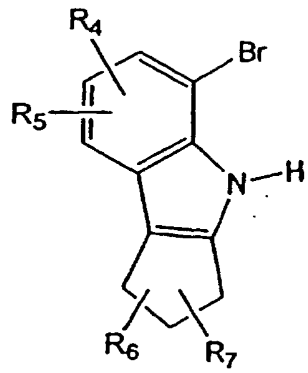

dans laquelle $R_4$, $R_5$, $R_6$ et $R_7$ sont comme définis à la revendication 1, ou aux revendications 5 à 8.

**18.** Composé suivant la revendication 17, qui est l'un des suivants :

le 5-bromo-1,2,3,4-tétrahydrocyclopenta[*b*]indole;
le 5-bromo-3-méthyl-1,2,3,4-tétrahydrocyclopenta[*b*]indole;
le 5-bromo-2-méthyl-1,2,3,4-tétrahydrocyclopenta[*b*]indole, ou
le 5-bromo-1-méthyl-1,2,3,4-tétrahydrocyclopenta[*b*]indole.

**19.** Composé de formule :

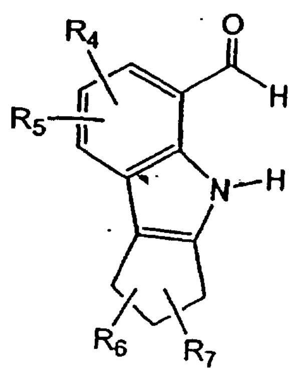

dans laquelle $R_4$, $R_5$, $R_6$ et $R_7$ sont comme définis à la revendication 1 ou aux revendications 5 à 8.

**20.** Composé suivant la revendication 19 qui est l'un des suivants :

le 1,2,3,4-tétrahydrocyclopenta[*b*]indole-5-carbaldéhyde; le 3-méthyl-1,2,3,4-tétrahydrocyclopenta[*b*]indole-5-carbaldéhyde;
le 2-méthyl-1,2,3,4-tétrahydrocyclopenta[*b*]indole-5-carbaldéhyde,
ou
le 1-méthyl-1,2,3,4-tétrahydrocyclopenta[*b*]indole-5-carbaldéhyde.

**21.** Composé de formule :

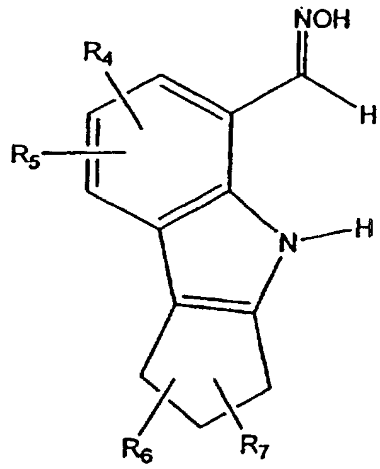

dans laquelle $R_4$, $R_5$, $R_6$ et $R_7$ sont comme définis à la revendication 1 ou aux revendications 5 à 8.

**22.** Composé suivant la revendication 21 qui est l'un des suivants :

le 1,2,3,4-tétrahydrocyclopenta[*b*]indole-5-carbaldéhyde oxime;
le 3-méthyl-1,2,3,4-tétrahydrocyclopenta[*b*]indole-5-carbaldéhyde oxime ;
le 2-méthyl-1,2,3,4-tétrahydrocyclopenta[*b*]indole-5-carbaldéhyde oxime, ou
le 1-méthyl-1,2,3,4-tétrahydrocyclopenta[*b*]indole-5-carbaldéhyde oxime.

**23.** Composé de formule :

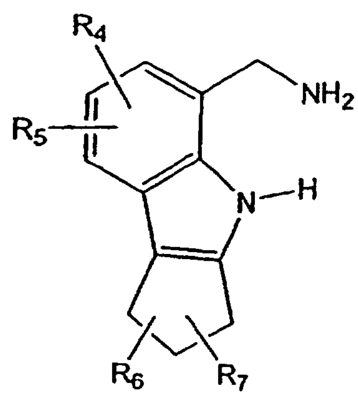

dans laquelle $R_4$, $R_5$, $R_6$ et $R_7$ sont comme définis à la revendication 1 ou aux revendications 5 à 8.

**24.** Composé suivant la revendication 23, qui est l'un des suivants:

la *C*- (1, 2,3, 4-tétrahydrocyclopenta[*b*]indol-5-yl)méthylamine;
la *C*-(3-méthyl-1,2,3,4-tétrahydrocyclopenta[*b*]indol-5-yl)méthylamine;
la *C*-(2-méthyl-1,2,3,4-tétrahydrocyclopenta[*b*]indol-5-yl)méthylamine, ou
la *C*-(1-méthyl-1,2,3,4-tétrahydrocyclopenta[*b*]indol-5-yl)méthylamine.

**25.** Composé de formule :

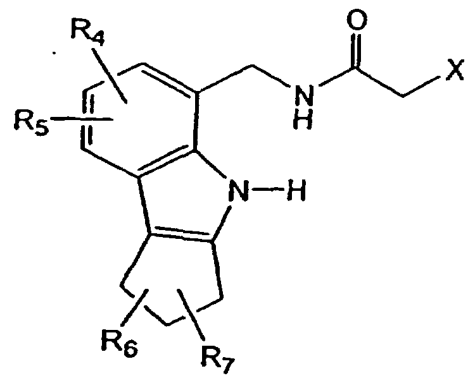

dans laquelle $R_4$, $R_5$, $R_6$ et $R_7$ sont comme définis à la revendication 1 ou aux revendications 5 à 8 et X est sélectionné parmi Cl, Br ou I.

**26.** Composé suivant la revendication 25, qui est sélectionné parmi le groupe des suivants :

le 2-chloro-*N*-(1,2,3,4-tétrahydrocyclopenta[*b*]indol-5-ylméthyl)acétamide;
le 2-chloro-*N*-(3-méthyl-1,2,3,4-tétrahydrocyclopenta[*b*]indol-5-ylméthyl) acétamide;
le 2-chloro-*N*-(2-méthyl-1,2,3,4-tétrahydrocyclopenta[*b*]indol-5-ylméthyl) acétamide;
le 2-chloro-*N*-(1-méthyl-1,2,3,4-tétrahydrocyclopenta[*b*]indol-5-ylméthyl)acétamide;
le 2-bromo-*N*-(1,2,3,4-tétrahydrocyclopenta[*b*]indol-5-ylméthyl)acétamide;
le 2-bromo-*N*-(3-méthyl-1,2, 3, 4-tétrahydrocyclopenta[*b*]indol-5-ylméthyl) acétamide;
le 2-bromo-*N*-(2-méthyl-1,2,3,4-tétrahydrocyclopenta[*b*]indol-5-ylméthyl)acétamide;
le 2-bromo-*N*-(1-méthyl-1,2,3,4-tétrahydrocyclopenta[*b*]indol-5-ylméthyl)acétamide;
le 2-iodo-*N*-(1,2,3,4-tétrahydrocyclopenta[*b*]indol-5-yl-méthyl)acétamide;
le 2-iodo-*N*-(3-méthyl-1,2,3,4-tétrahydrocyclopenta[*b*]indol-5-ylméthyl)acétamide;
le 2-iodo-*N*-(2-méthyl-1,2,3,4-tétrahydrocyclopenta[*b*]indol-5-ylméthyl)acétamide;
ou
le 2-iodo-*N*-(1-méthyl-1,2,3,4-tétrahydrocyclopenta[*b*]indol-5-ylméthyl)acétamide.

**27.** Composé de formule :

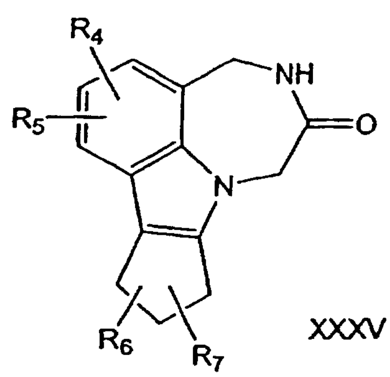

XXXV

dans laquelle $R_4$, $R_5$, $R_3$, $R_6$ et $R_7$ sont comme définis à la revendication 1 ou aux revendications 5-8.

**28.** Composé suivant la revendication 27, qui est l'un des suivants :

la 4,5,9,10-tétrahydro-8*H*-5,7a-diaza-benzo[*cd*]cyclopenta[*a*]azulén-6-one;
la 8-méthyl-4,5,9,10-tétrahydro-8*H*-5,7a-diaza-benzo[*cd*]cyclopenta[*a*]azulén-6-one;
la 9-méthyl-4,5,9,10-tétrahydro-8*H*-5,7a-diaza-benzo[*cd*]cyclopenta[*a*]azulén-6-one; ou

la 10-méthyl-4,5,9,10-tétrahydro-8H-5,7a-diaza-benzo[*cd*]cyclopenta[*a*]azulén-6-one.

**29.** Composé présentant l'une des formules :

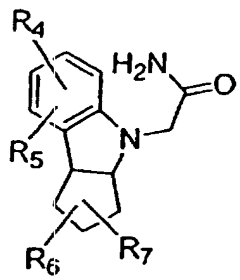

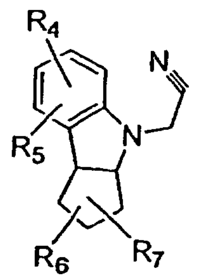

ou

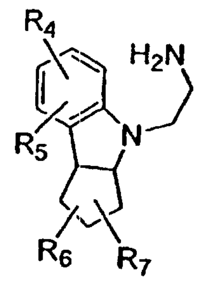

dans laquelle $R_4$, $R_5$, $R_6$ et $R_7$ sont chacun, indépendamment, un hydrogène, un hydroxy, un alkyle de 1-6 atomes de carbone, un cycloalkyle, de 3 à 7 atomes de carbone, un alcoxy de 1-6 atomes de carbone, un halogène, un alkyle fluoré de 1 à 6 atomes de carbone, - CN, -NH-$SO_2$-alkyle de 1-6 atomes de carbone, -$SO_2$-NH-alkyle de 1-6 atomes de carbone, un alkylamide de 1-6 atomes de carbone, un amino, un alkylamino de 1-6 atomes de carbone, un dialkylamino de 1-6 atomes de carbone par partie alkyle, un alcoxy fluoré de 1-6 atomes de carbone, un acyle de 2-7 atomes de carbone, un aryle, un hétéroaryle, un aroyle ou un hétéroaroyle.

**30.** Composé suivant la revendication 29, dans laquelle $R_4$ et $R_5$ sont un hydrogène, et $R_6$ et $R_7$ sont comme définis à la revendication 1.

**31.** Composé suivant la revendication 29, dans laquelle $R_4$, $R_5$ et $R_6$ sont un hydrogène, et $R_7$ est comme défini à la revendication 1.

**32.** Composé suivant la revendication 29, qui est le 2-(2,3,3a,8b-tétrahydro-1H-cyclopenta[*b*]indol-4-yl)acétamide.

**33.** Composé suivant la revendication 29, qui est l'un des suivants :

le 2- (2, 3, 3a, 8b-tétrahydro-1*H*-cyclopenta [*b*] indol-4-yl)-acétamide;
le 2- (2, 3, 3a, 8b-tétrahydro-1*H*-cyclopenta [*b*] indol-4-yl)-acétonitrile, ou
la 2- (2,3,3a,8b-tétrahydro-1*H*-cyclopenta[*b*]indol-4-yl)-éthylamine.